# EUROPÄISCHE PATENTSCHRIFT

(11) **EP 4 199 983 B1**
(45) Veröffentlichungstag und Bekanntmachung des Hinweises auf die Patenterteilung: **04.11.2026**
(21) Anmeldenummer: 21806293.3
(22) Anmeldetag: 16.11.2021
(51) Int. Cl.: A61L 29/16, A61L 31/16, A61L 29/08, A61L 31/08

(54) **BESCHICHTETES MEDIZINPRODUKT**
COATED MEDICAL PRODUCT
PRODUIT MÉDICAL REVÊTU

(30) Priorität: 16.11.2020 EP 20207915; 16.09.2021 EP 21197295
(43) Veröffentlichungstag der Anmeldung: 28.06.2023
(73) Patentinhaber: Freudenberg Medical Hemoteq GmbH, 52146 Würselen (DE)
(72) Erfinder: HOFFMANN, Michael, 52159 Roetgen (DE); HOFFMANN, Erika, 52159 Roetgen (DE); MATHAR, Günter, 52224 Stolberg (DE)
(74) Vertreter: Arth, Hans-Lothar
(86) Internationale Anmeldenummer: PCT/EP2021/081898
(87) Internationale Veröffentlichungsnummer: WO 2022/101514

(56) Entgegenhaltungen:
- WO-A1-2015/039969
- US-A1- 2015 250 772
- US-A1- 2020 197 673

## Beschreibung

### Gebiet der Erfindung

Die vorliegende Erfindung betrifft eine Suspension zur Beschichtung von Medizinprodukten enthaltend mindestens ein Tri-O-Acylglycerol, mindestens einen mikrokristallinen Limus-Wirkstoff, und mindestens ein Lösungsmittel oder Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-O-Acylglycerols nicht lösen. Die vorliegende Erfindung betrifft weiterhin Verfahren zur Herstellung dieser Suspension, Verfahren zur Beschichtung von Medizinprodukten und Medizinprodukte, die mit mindestens einem Tri-O-Acylglycerol und mindestens einem mikrokristallinen Limus-Wirkstoff beschichtet sind.

### Hintergrund der Erfindung

Medizinprodukte dienen dazu im Körper fehlende Funktionen zu übernehmen, körpereigene Funktionen zu unterstützen oder dazu mit ihrer Hilfe Wirkstoffe lokal übertragen zu können. Je nach Einsatzgebiet haben Medizinprodukte entweder kurz- oder langfristig Kontakt mit einem Organismus. Die Kontaktzeit kann sich dabei von wenigen Sekunden bis zu Jahrzehnten erstrecken. Wird der Einsatz eines Medizinproduktes notwendig, ist es erforderlich, die bei der Wundheilung auftretenden unvermeidlichen Entzündungsprozesse zu kontrollieren, um Überreaktionen des Immunsystems im Heilungsprozess zu verhindern.

Bei der Behandlung von Gefäßverengungen (Stenosen) mit mechanischen oder thermischen Verfahren, wie z.B. die Implantation von Gefäßstützen (Stents) oder Ballonangioplastie, tritt als häufige Komplikation wenige Wochen nach der Behandlung die Restenose auf. Um die Restenose zu verhindern, wurden Stents und Katheterballons mit Wirkstoffen, insbesondere mit antirestenotischen Wirkstoffen beschichtet. Als erfolgreiche Wirkstoffe haben sich in der Vergangenheit Limus-Wirkstoffe wie z.B. Rapamycin (Sirolimus) oder Taxane wie z.B. Paclitaxel bewährt. Limus-Wirkstoffe binden reversibel an FKBP12 und unterdrücken die Zellteilung, wohingegen Taxane wie Paclitaxel irreversibel an Microtubuli binden und ebenfalls die Zellteilung unterdrücken.

Aus dem Stand der Technik sind "Drug-Eluting Stents" (DES) (wirkstoffabgebende Stents) bekannt, bei denen neben der Gefäßweitung und der damit verbundenen Verletzung der Gefäßwand mit Hilfe von passenden Wirkstoffen die Heilung an der betroffenen Stelle kontrolliert werden soll.

Aus dem Stand der Technik sind weiterhin Medizinprodukte bekannt, die nicht permanent im Körper verbleiben, wie z.B. biodegradierbare Stents. Die biodegradierbaren Stents können zusätzlich noch eine Wirkstoffbeschichtung aufweisen, um die Vorteile eines langfristig wirkenden Medizinprodukts zu gewährleisten. Dieser Ansatz steht allerdings immer noch in der Entwicklung.

Neben den Stents sind im dem Stand der Technik auch wirkstoffabgebende Katheter, insbesondere Ballonkatheter bekannt, welche insbesondere den Vorteil aufweisen nur kurzzeitig mit dem Organismus in Kontakt treten.

Doch nicht nur Stents und Katheter lassen sich mit Wirkstoffen beschichten, wobei sich die Anforderungen an die Beschichtung anderer Medizinprodukte natürlich je nach Einsatzgebiet unterscheiden.

Insbesondere die Anforderungen an wirkstoffabgebende Beschichtungen von Kathetern sind sehr hoch, da besonders bei diesen sehr kurzfristig eingesetzten Medizinprodukten, speziell im vaskulären Bereich, eine über die sehr kurze Verweildauer des Medizinproduktes hinaus langfristige, gut dosierte und doch möglichst quantitative Applikation von Wirkstoffen eine besondere Herausforderung darstellt, wobei gewährleistet sein muss, dass zum einen der Wirkstoff nicht bereits auf dem Weg zum Zielort vorzeitig weggespült wird oder z.B. beim Expandieren abbröckelt und lediglich eine undefinierte bzw. ungenügende Menge an Wirkstoff die Gefäßwand erreicht. Zum anderen muss im Falle eines koronaren "Drug-Coated Balloon" (DCB) (wirkstoffbeschichteter Ballon) als kurzzeitig eingesetztes Medizinprodukt auch die stark limitierte Kontaktzeit von maximal 90 Sekunden ausreichen, damit der Wirkstoff in vorgesehener Dosierung vom Ballonkatheter auf bzw. in die Gefäßwand übertragen werden kann. Das periphere vaskuläre System z.B. in der Beinarterie erlaubt längere Kontaktzeiten von um die 120 Sekunden und mehr, je nachdem welches Gefäß behandelt wird, wobei die Obergrenze der Kontaktzeit in peripheren Gefäßen mit max. 5 Minuten in der oberflächigen Oberschenkelarterie *Arteria femoralis superficialis* (AFS) liegt.

Aus dem Stand der Technik sind einige Wirkstoffbeschichtungen von Katheterballons bekannt, die diese Probleme umgehen, indem die Wirkstofffreisetzung beschleunigt oder die Stabilität der Beschichtung durch zusätzliche Hilfsstoffe in der Beschichtung erhöht wird (siehe WO2010/121840A2, WO2013/007653A1, WO2012/146681 A1).

Insbesondere bei Katheterballons, die mit Limus-Verbindungen beschichtet sind, liegt, neben der geringen Wirkstoffübertragung, eine weitere Ursache für die geringe Wirksamkeit in der kurzen Verweildauer der Limus-Verbindung in der Gefäßwand.

Aus dem Stand der Technik ist bekannt, dass Limus-Wirkstoffkristalle ein langsameres Auflösungsverhalten zeigen als amorphe Limus-Partikel. So konnten höhere Wirkstoffkonzentrationen in der Gefäßwand einen Monat nach der Behandlung mit Katheterballons, die mit einem kristallinen Limus-Wirkstoff beschichtet waren, im Vergleich zu einer Behandlung mit Katheterballons mit einer amorphen Wirkstoffbeschichtung nachgewiesen werden (Clever et al., Circ. Cardiovasc. Interv. 2016, 9, 1-11; e003543).

Beschichtungen mit kristallinen Limus-Wirkstoffen sind daher erstrebenswert, um eine verlängerte Verweilzeit des Limus-Wirkstoffs in der Gefäßwand zu gewährleisten. Allerdings birgt die Verwendung von kristallinen Ballonbeschichtungen das Risiko der Embolie (WO2011/147408A2), so dass im Stand der Technik nach wie vor amorphe Beschichtungen für Katheterballons bevorzugt werden.

Die Applikation von Limus-Kristallen mittels Ballondilation an das zu behandelnde Gewebe hat den Vorteil, dass die Kristalle als Wirkstoffdepots wirken und den Wirkstoff verzögert freisetzen, wohingegen bei amorphen Wirkstoffen eine unmittelbare Freisetzung nach Dilatation erfolgt. Es hat sich aber gezeigt, dass eine direkte Beschichtung mit Limus-Kristallen oder eine Beschichtung mit einer kristallhaltigen reinen Limus-Wirkstoff-Suspension insbesondere den Nachteil aufweist, dass die Limus-Kristalle nicht ausreichend an der Medizinprodukteoberfläche haften. Ein weiteres Problem besteht darin, dass Suspensionen von Partikeln größer als 1-5 µm rasch zur Sedimentation neigen, was in Folge eine gleichmäßige Beschichtung mit mikrokristallinen Limus-Wirkstoffen aus Suspensionen deutlich erschwert.

Aus dem Stand der Technik sind Wirkstoffbeschichtungen mit kristallinen Limus-Wirkstoffen für Katheterballons bekannt, die versuchen diese Probleme zu umgehen. Die aus dem Stand der Technik bekannten Verfahren zur Beschichtung von Katheterballons mit kristallinen Limus-Verbindungen sind jedoch aufwändig und kompliziert.

Die internationale Patentanmeldung WO2013/022458A1 offenbart ein Verfahren zur Überführung von amorphem Everolimus in seine kristalline Form durch Altern einer übersättigten Lösung (Suspension) über mehrere Tage. Dabei wird die niedrigere Löslichkeit der kristallinen gegenüber der amorphen Form ausgenutzt.

Im Stand der Technik werden hauptsächlich Limus-Kristallsuspensionen vorgeschlagen, bei denen die Größe der Limus-Kristalle im Nanobereich von weniger als 1 µm liegt. Allerdings weisen Kristalle dieser Größe den Nachteil auf, dass mit diesen nicht ausreichend die gewünschte verlängerte Verweilzeit des Limus-Wirkstoffs in der Gefäßwand gewährleistet werden kann.

Die internationale Patentanmeldung WO2015/039969A1 offenbart ein Beschichtungsverfahren von Ballonkathetern mit kristallinen Limus-Wirkstoffen. Die kristallinen Limus-Wirkstoffe wurden entweder vorher hergestellt und als Suspension auf den Ballonkatheter aufgetragen, oder die Kristallisation wurde auf dem Ballon durch Impfkristalle ausgelöst. Eine Kristallisation an der Oberfläche hat jedoch den Nachteil, dass zusätzlich zur Kristallisation Fällungsprozesse eintreten, die zu amorphen Partikeln bzw. Agglomeraten mit einer Größe von 100 - 300 µm führen, die mittels Ultraschallbehandlung nicht dispergierbar sind. Solche Agglomerate mit einer Größe von über 100 µm bergen das Risiko während der Dilatation Gefäßverschlüsse distal von der Dilatationsstelle zu verursachen und können damit eine erhebliche Gefährdung des Patienten darstellen. Es ist daher besonders wünschenswert die Anzahl solcher amorphen Partikel bzw. Agglomerate so gering wie möglich zu halten.

Ausreichend feste, reproduzierbare Beschichtungen von Katheterballons mit Limus-Kristallen, die eine ausreichende Wirkstoffübertragung bei Dilatation aufweisen, konnten durch die Beschichtung eines Katheterballons mit einer Lösung bestehend aus einem Lösungsmittel und einem Limus-Wirkstoff als auch durch Aufstäuben von Limus-Kristallen auf die Ballonoberfläche mit nachfolgender optionaler Anhaftungsverbesserung durch "Solvent Bonding" sowie durch die Kristallisation eines gelösten Limus-Wirkstoffs aus einer Lösung des Limus-Wirkstoff in einem Lösungsmittel und einem Nicht-Lösungsmittel nicht erhalten werden.

Die US-Patentanmeldung US 2015/250772 A1 offenbart eine medizinische Vorrichtung mit einer polymerfreien äußeren Oberflächenbeschichtung, die ein kristallines Arzneimittel aus der Gruppe Everolimus, Tacrolimus, Sirolimus, Zotarolimus, Biolimus und Rapamycin umfasst. Die Beschichtung kann einen nichtpolymeren Hilfsstoff wie Glycerolester von kurzkettigen (d.h. C₂-C₈) Monocarbonsäuren wie Triacetin enthalten.

Aufgabe der vorliegenden Erfindung ist es Beschichtungsformulierungen und beschichtete Medizinprodukte bereitzustellen, wobei die Beschichtung flexibel ist, sehr gut an der Medizinprodukteoberfläche haftet, eine optimale Größenverteilung der Wirkstoffpartikel aufweist und auch bei sehr kurzer Verweilzeit im Körper möglichst quantitativ den Wirkstoff abgibt, der dann auch über einen viel längeren Zeitraum von der Gefäßwand in die Zellen diffundieren kann.

Anders formuliert besteht die Aufgabe der vorliegenden Erfindung also darin, Zusammensetzungen für Beschichtungen von kurz- als auch langfristig genutzten Medizinprodukten bereitzustellen, welche als Beschichtung stabil und doch flexibel auf der Medizinprodukteoberfläche haften und zum anderen eine möglichst vollständige als auch kontrollierte Wirkstoffübertragung auf die Gefäßwand bzw. Gewebe sicherstellen, um den Heilungsprozess optimal unterstützen zu können.

Insbesondere besteht die Aufgabe der vorliegenden Erfindung, eine Beschichtung von Katheterballons mit kristallinen Limus-Verbindungen bereitzustellen, wobei die Beschichtung stabil und doch flexibel auf der Oberfläche der Katheterballons haftet und zum anderen eine möglichst vollständige als auch kontrollierte Wirkstoffübertragung auf die Gefäßwand bzw. Gewebe während der Dilatation sicherstellt, um den Heilungsprozess optimal unterstützen zu können

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung, den Figuren sowie den Beispielen.

### Kurzbeschreibung

Die Erfindung wird in den beigefügten Ansprüchen definiert. Überraschenderweise wurde gefunden, dass eine Suspension zur Beschichtung von Medizinprodukten, insbesondere von Katheterballons, Ballonkatheter, Stents und Kanülen, enthaltend a) mindestens ein Tri-O-Acylglycerol ausgewählt aus der Gruppe bestehend aus Trioctanoylglycerol, Trinonanoylglycerol, Tridecanoylglycerol und Triundecanoylglycerol, b) mindestens einen Limus-Wirkstoff in Form von Mikrokristallen und c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen oder sich die Mikrokristalle des mindestens einen Limus-Wirkstoffs bei Anwesenheit des mindestens einen Tri-O-Acylglycerols nicht lösen, die oben genannte Aufgabe löst.

Es wurde nun gefunden, dass eine besonders vorteilhafte Kristallsuspension eines mikrokristallinen Limus-Wirkstoffs zur Beschichtung von Medizinprodukten, in der sich die Mikrokristalle des Limus-Wirkstoffs nicht auflösen, bereitgestellt werden kann, wenn in dieser Suspension mindestens ein Tri-O-Acylglycerol ausgewählt aus der Gruppe bestehend aus Trioctanoylglycerol, Trinonanoylglycerol, Tridecanoylglycerol und Triundecanoylglycerol in gelöster Form vorliegt.

Mit diesen erfindungsgemäßen Tri-O-Acylglycerolen konnten überraschenderweise solch stabile Suspensionen von mikrokristallinen Limus-Wirkstoffen hergestellt werden, dass die Mikrokristalle der Limus-Wirkstoffe "in der Schwebe" gehalten werden und in Folge nicht sedimentieren. Dies war insofern überraschend, da gerade Suspensionen von Kristallen im Mikrometerbereich, also Kristalle die größer als 1-5 µm sind, zur Sedimentation neigen. Die erfindungsgemäße Kristallsuspension ist somit insbesondere vorteilhaft, um eine gleichmäßige Beschichtung von Mikrokristallen eines Limus-Wirkstoffs auf Medizinprodukten herzustellen.

Ein weiterer besonderer Vorteil der Verwendung von mindestens einem Tri-O-Acylglycerol ausgewählt aus der Gruppe bestehend aus Trioctanoylglycerol, Trinonanoylglycerol, Tridecanoylglycerol und Triundecanoylglycerol, besteht darin, dass diese erfindungsgemäßen Tri-O-Acylglycerole in der Lage sind, die Mikrokristalle des Limus-Wirkstoffs wie "Flexkleber" auf einer Medizinprodukteoberfläche festzuhalten. Die erfindungsgemäße Kristallsuspension ist somit insbesondere vorteilhaft, um eine gleichmäßige Beschichtung von Mikrokristallen eines Limus-Wirkstoffs auf Medizinprodukten herzustellen, bei der auch die Mikrokristalle des Limus-Wirkstoffs ausreichend an der Medizinprodukteoberfläche haften.

Mit weiteren aus dem Stand der Technik bekannten nicht erfindungsgemäßen Tri-O-Acylglycerolen war die Herstellung von Kristallsuspensionen gemäß der vorliegenden Erfindung nicht möglich. Es hat sich gezeigt, dass nicht erfindungsgemäße Tri-O-Acylglycerole ein An- oder Auflösen der Mikrokristalle des Limus-Wirkstoffs in der Suspension bewirken oder fördern. Zudem trat bei nicht erfindungsgemäßen Tri-O-Acylglycerolen eine Sedimentation der Mikrokristalle des Limus-Wirkstoffs auf.

Zudem konnten mit weiteren aus dem Stand der Technik bekannten nicht erfindungsgemäßen Tri-O-Acylglycerolen keine gleichmäßigen Beschichtungen von Mikrokristallen eines Limus-Wirkstoffs auf Medizinprodukten hergestellt werden, wobei insbesondere eine mangelnde Haftfähigkeit der Mikrokristalle des Limus-Wirkstoffs auf der Medizinprodukteoberfläche beobachtet wurde. Es hat sich gezeigt, dass nicht erfindungsgemäße Tri-O-Acylglycerole die Mikrokristalle des Limus-Wirkstoffs auf einer Medizinprodukteoberfläche nicht ausreichend "kleben" und festhalten können.

Somit konnte nur mit den Tri-O-Acylglycerolen ausgewählt aus der Gruppe bestehend aus Trioctanoylglycerol, Trinonanoylglycerol, Tridecanoylglycerol und Triundecanoylglycerol oder auch mit Gemischen dieser Tri-O-Acylglycerole eine erfindungsgemäße Kristallsuspension eines mikrokristallinen Limus-Wirkstoffs hergestellt werden, in der die Mikrokristalle des Limus-Wirkstoffs intakt bleiben. Ein weiterer besonderer Vorteil ist, dass die Mikrokristalle des mindestens einen Limus-Wirkstoffs in dieser Suspension schweben und daher in der Suspension gleichmäßig verteilt vorliegen, womit der Limus-Wirkstoff nicht nur in Form von Mikrokristallen, sondern zudem auch gleichmäßig auf die Medizinprodukteoberfläche aufgebracht werden kann.

Medizinprodukte, die mit einer erfindungsgemäßen Suspension beschichtet worden sind, weisen auf der Medizinprodukteoberfläche eine Beschichtung von mindestens einem Tri-O-Acylglycerol ausgewählt aus der Gruppe bestehend aus Trioctanoylglycerol, Trinonanoylglycerol, Tridecanoylglycerol und Triundecanoylglycerol, und Mikrokristallen des mindestens einen Limus-Wirkstoffes auf. Diese Beschichtung zeichnet sich vor allem durch eine sehr gute Flexibilität und exzellente Haftfähigkeit an der Medizinprodukteoberfläche aus. Weiterhin bietet diese Beschichtung die vorteilhafte Eigenschaft, dass auch bei sehr kurzer Verweilzeit im Körper die Mikrokristalle des mindestens einen Limus-Wirkstoffs quantitativ abgegeben werden können, die dann im Gegensatz zu amorphen Limus-Wirkstoffpartikeln über einen viel längeren Zeitraum von der Gefäßwand in die Zellen diffundieren können.

Eine erfindungsgemäße Beschichtung lässt sich auf jedem Medizinprodukt bereitstellen, bevorzugt sind hierin Katheterballons, Ballonkatheter, Stents und Kanülen, insbesondere bevorzugt sind Katheterballons. Die Limus-Wirkstoffmenge und die Limus-Wirkstoffabgabegeschwindigkeit bzw. Elutionsrate kann entsprechend der notwendigen Vorgaben am Einsatzort variieren, während das mindestens eine Tri-O-Acylglycerol ausgewählt aus der Gruppe bestehend aus Trioctanoylglycerol, Trinonanoylglycerol, Tridecanoylglycerol und Triundecanoylglycerol zum einen die optimale Übertragung des mikrokristallinen Limus-Wirkstoffs in das Gewebe unterstützt und gleichzeitig für eine hohe Flexibilität und Stabilität der Beschichtung sorgt, so dass garantiert wird, dass der mikrokristalline Limus-Wirkstoff tatsächlich ohne Verluste in optimaler Konzentration in das umliegende Gewebe gelangt.

Dabei ist die sehr gute Flexibilität und Haftfähigkeit der erfindungsgemäßen Beschichtung im besonderen Maße für die Medizinprodukte wichtig, die

Formveränderung durchlaufen müssen z.B. bei Stents und Katheterballons. Beispielsweise erfordert das Inflatieren, Deflatieren, Falten und Crimpen besondere Anforderungen an die Stabilität einer Beschichtung, die zudem auch während der Implantation Reibungen und Körperflüssigkeit als auch Strömungen ausgesetzt wird. Auch die Einstellung der gewünschten Elutionsrate des mikrokristallinen Limus-Wirkstoffes und die möglichst optimale Übertragungsmenge an mikrokristallinem Limus-Wirkstoff in das Gewebe werden mit einer erfindungsgemäßen Beschichtung von Katheterballons (DCB) gelöst. Weitere zu beachtende Anforderungen, die die erfindungsgemäße Beschichtung unbeschadet erfüllt, beziehen sich auf die Sterilisierung der Medizinprodukte, die Lagerfähigkeit, Mindesthaltbarkeit, Temperaturbeständigkeit, und dergleichen.

Somit löst die erfindungsgemäße Beschichtung auf Medizinprodukten die wichtigen Aufgaben, die an ein langfristig und auch an ein kurzfristig im Körper eingesetztes Medizinprodukt gestellt werden.

### Detaillierte Beschreibung

Die vorliegende Erfindung betrifft eine Suspension zur Beschichtung von Medizinprodukten, ausgewählt aus Katheterballons, Ballonkathetern, Stents und Kanülen, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen.

Essentiell für die Erfindung ist die Verwendung von mikrokristallinem Limus-Wirkstoff und das Vorliegen einer Suspension des mikrokristallinen Limus-Wirkstoffs, wobei die Suspension mindestens ein Tri-O-Acylglycerol ausgewählt aus der Gruppe bestehend aus Trioctanoylglycerol, Trinonanoylglycerol, Tridecanoylglycerol und Triundecanoylglycerol enthalten muss.

Der Begriff **"Beschichtungsformulierung"** oder "wirkstoffenthaltende Zusammensetzung", wie hierin verwendet, bezieht sich auf eine Mischung von mindestens einem Limus-Wirkstoff und einem Lösungsmittel oder einem Lösungsmittelgemisch und mindestens einem Tri-O-Acylglycerol ausgewählt aus der Gruppe bestehend aus Trioctanoylglycerol, Trinonanoylglycerol, Tridecanoylglycerol und Triundecanoylglycerol, also einer Lösung, Dispersion, Suspension oder Emulsion. Der Begriff "Formulierung" soll verdeutlichen, dass es sich um ein flüssiges Gemisch (Suspension, Emulsion, Dispersion, Lösung) handelt. Der Begriff "Beschichtungsformulierung", wie hierin verwendet, stellt somit den Oberbegriff für die Begriffe "Lösung" bzw. "Beschichtungslösung", "Dispersion" bzw. "Beschichtungsdispersion", "Suspension" bzw. "Beschichtungssuspension" und "Emulsion" bzw. "Beschichtungsemulsion" dar.

Der Begriff **"Lösung"** oder "Beschichtungslösung", wie hierin verwendet, bezieht sich im Allgemeinen auf ein homogenes Gemisch, das aus zwei oder mehr chemisch reinen Stoffen besteht. Lösungen sind rein äußerlich nicht als solche erkennbar, da sie definitionsgemäß nur eine Phase bilden und die gelösten Stoffe gleichmäßig im Lösungsmittel verteilt sind.

Der Begriff **"Dispersion"** oder "Beschichtungsdispersion", wie hierin verwendet, bezieht sich im Allgemeinen auf ein heterogenes Gemisch aus mindestens zwei Stoffen, die sich nicht oder kaum ineinander lösen und sich chemisch nicht miteinander verbinden. Dabei sind ein oder mehrere Stoffe als disperse Phase fein verteilt in einem anderen kontinuierlichen Stoff, dem sogenannten Dispersionsmedium.

Der Begriff **"Emulsion"** oder "Beschichtungsemulsion", wie hierin verwendet, bezieht sich im Allgemeinen auf fein verteiltes Gemisch zweier normalerweise nicht mischbarer Flüssigkeiten ohne sichtbare Entmischung. Eine Flüssigkeit bildet kleine Tröpfchen, verteilt in der anderen Flüssigkeit. Die Emulsion ist eine bestimmte Form einer Dispersion.

Der Begriff **"Suspension"** bzw. "Beschichtungssuspension", wie hierin verwendet, bezieht sich im Allgemeinen auf ein heterogenes Stoffgemisch aus einem fein verteilten Feststoff in einer Flüssigkeit. Damit handelt es sich bei einer "Suspension" definitionsgemäß nicht um ein homogenes Gemisch und damit auch nicht um eine Lösung. Die Suspension ist eine bestimmte Form einer Dispersion.

Eine "Suspension" enthaltend mindestens einen Limus-Wirkstoff in Form von Mikrokristallen wird hierin auch als "Kristallsuspension" bezeichnet. Gemäß der vorliegenden Erfindung handelt es sich bei dem fein verteilten Feststoff der Suspension hierin um mindestens einen mikrokristallinen Limus-Wirkstoff bzw. um Mikrokristalle mindestens eines Limus-Wirkstoffes. Bei der Flüssigkeit der Suspension handelt es sich hierin um ein Lösungsmittel oder ein Lösungsmittelgemisch, wobei mindestens ein Tri-O-Acylglycerol ausgewählt aus der Gruppe bestehend aus Trioctanoylglycerol, Trinonanoylglycerol, Tridecanoylglycerol und Triundecanoylglycerol in dem Lösungsmittel oder dem Lösungsmittelgemisch gelöst vorliegt.

Eine "Suspension" gemäß der vorliegenden Erfindung betrifft somit ein heterogenes Stoffgemisch einer Flüssigkeit enthaltend mindestens ein Tri-O-Acylglycerol ausgewählt aus der Gruppe bestehend aus Trioctanoylglycerol, Trinonanoylglycerol, Tridecanoylglycerol und Triundecanoylglycerol, und in dieser Flüssigkeit fein verteilten Festkörpern, nämlich den Mikrokristallen des mindestens einen Limus-Wirkstoffs. Der mikrokristalline Limus-Wirkstoff ist somit erfindungsgemäß in einer Flüssigkeit enthaltend mindestens ein gelöstes Tri-O-Acylglycerol ausgewählt aus der Gruppe bestehend aus Trioctanoylglycerol, Trinonanoylglycerol, Tridecanoylglycerol und Triundecanoylglycerol suspendiert.

Die Suspension gemäß der vorliegenden Erfindung ist dadurch gekennzeichnet, dass weder eine Sedimentation noch ein Auflösen der Mikrokristalle des mindestens einen Limus-Wirkstoffs in der Suspension erfolgt. Die erfindungsgemäße Suspension wird hierin auch als "stabile Suspension" bezeichnet.

Die erfindungsgemäße Suspension kann aus dem Lösungsmittel oder dem Lösungsmittelgemisch und dem mikrokristallinen Limus-Wirkstoff und dem mindestens einen gelösten Tri-O-Acylglycerol ausgewählt aus der Gruppe bestehend aus Trioctanoylglycerol, Trinonanoylglycerol, Tridecanoylglycerol und Triundecanoylglycerol bestehen. Die Suspension darf aber bis zu 5,0 Gew.% bezogen auf den Limus-Wirkstoff andere Zusatzstoffe enthalten, d.h. bei 95g Limus-Wirkstoff können bis zu 5g an Zusatzstoffen in der Suspension enthalten sein. Nur im Falle von Antioxidantien als Zusatzstoffe darf die Menge an Antioxidantien bis zu 15 Gew.% betragen, wobei die Menge an Antioxidantien und allen anderen Zusatzstoffen trotzdem 15 Gew.% nicht überschreiten darf, d.h. bei 85g Limus-Wirkstoff können bis zu 15g an Antioxidantien in der Suspension enthalten sein. Sind somit 15 Gew.% Antioxidantien in der Suspension enthalten, dann können keine weiteren Zusatzstoffe anwesend sein. Sind hingegen 10 Gew.% an Antioxidantien in der Suspension enthalten, so können bis maximal 5,0 Gew.% auch noch andere Zusatzstoffe enthalten sein.

Somit ist die vorliegende Erfindung auch auf eine Suspension zur Beschichtung eines Medizinprodukts ausgewählt aus einem Katheterballon, einem Ballonkatheter, einem Stent oder einer Kanüle gerichtet, die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-O-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet.

Als Zusatzstoffe kommen die weiter unten genannten Substanzen in Frage, bevorzugt Antioxidantien, Polyvinylpyrrolidon (PVP) und Flokkulationsinhibitoren.

Die Antioxidantien und bevorzugt BHT sind vorzugsweise in einer Menge bis zu 12,0 Gew.% bezogen auf den Limus-Wirkstoff, weiter bevorzugt bis zu 10,0 Gew.% bezogen auf den Limus-Wirkstoff, weiter bevorzugt bis zu 9,0 Gew.% bezogen auf den Limus-Wirkstoff, weiter bevorzugt bis zu 8,0 Gew.% bezogen auf den Limus-Wirkstoff und weiter bevorzugt bis zu 7,0 Gew.% bezogen auf den Limus-Wirkstoff in der Suspension enthalten. Andere Zusatzstoffe wie z.B. PVP oder Flokkulationsinhibitoren, die nicht zu den Antioxidantien gehören, können vorzugsweise in einer Menge bis zu 4,0 Gew.% bezogen auf den Limus-Wirkstoff, bevorzugt bis zu 3,0 Gew.% bezogen auf den Limus-Wirkstoff, weiter bevorzugt bis zu 2,5 Gew.% bezogen auf den Limus-Wirkstoff, weiter bevorzugt bis zu 2,0 Gew.% bezogen auf den Limus-Wirkstoff, weiter bevorzugt bis zu 1,5 Gew.% bezogen auf den Limus-Wirkstoff und weiter bevorzugt bis zu 1,0 Gew.% bezogen auf den Limus-Wirkstoff in der Suspension enthalten sein.

Der Begriff **"Tri-*O*-Acylglycerol",** bzw. kurz "Triacylglycerol," wie hierin verwendet, bezieht sich auf eine chemische Verbindung von Glycerol (Glycerin), das mit drei Fettsäuren verestert ist, d.h. dreifach veresterte Glycerole (Glycerine). Triglycerid oder Glycerol-Triester sind synonyme Bezeichnungen von Tri-O-Acylglycerol, wobei die Bezeichnung Tri-O-Acylglycerol der IUPAC-Empfehlung entspricht.

Tri-O-Acylglycerole weisen die folgende allgemeine Formel (I) auf: 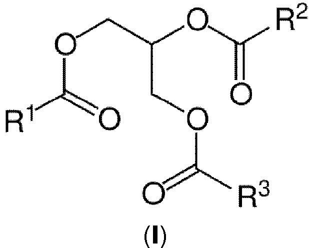 wobei R¹, R² und R³ Alkyl- oder Alkenylreste darstellen. Der Aufbau der Tri-O-Acylglycerole ist vielfältig, da mit R¹, R² und R³ viele verschiedene Fettsäuren und damit eine hohe Anzahl an Kombinationsmöglichkeiten möglich sind. Alle sind unpolar, d.h. lipophil. Bei Tri-O-Acylglycerolen kann weiterhin zwischen mittel- und langkettigen Tri-O-Acylglycerolen unterschieden werden. Mittelkettige Tri-O-Acylglycerole weisen Fettsäuren mit einer mittleren Länge von 6 bis 12 Kohlenstoffatomen und langkettige Tri-O-Acylglycerole weisen Fettsäuren mit einer Länge von 14 bis 24 Kohlenstoffatomen auf. Dabei können sich noch zwei Arten von Tri-O-Acylglycerolen ergeben: einfache und gemischte Tri-O-Acylglycerole. Bei einfachen Tri-O-Acylglycerolen sind die Fettsäurereste R¹, R² und R³ identisch, bei gemischten ist zumindest einer der Fettsäurereste R¹, R² und R³ verschieden von den anderen beiden. Beispiele von mittellangen Fettsäuren sind Capronsäure (Hexansäure), Önanthsäure (Heptansäure), Caprylsäure (Octansäure), Pelargonsäure (Nonansäure), Caprinsäure (Decansäure), Undecansäure und Laurinsäure (Dodecansäure).

Es hat sich nun unerwartet gezeigt, dass die chemischen, physikalischen und biologischen Eigenschaften der Tri-O-Acylglycerole, die mit den mittellangen Fettsäuren Caprylsäure (Octansäure), Caprinsäure (Decansäure), Pelargonsäure (Nonansäure) oder Undecansäure vollständig verestert sind, überhaupt erst eine gleichmäßige und ausreichend haftende Beschichtung von Medizinprodukten mit mikrokristallinen Limus-Wirkstoffen ermöglichen. Zudem war nur bei diesen mit Caprylsäure (Octansäure), Caprinsäure (Decansäure), Pelargonsäure (Nonansäure) oder Undecansäure vollständig veresterten Glycerolen die Herstellung einer erfindungsgemäßen Kristallsuspension möglich.

Die hierin bevorzugten Tri-O-Acylglycerole weisen daher die folgende allgemeine Formel (**I**) auf: 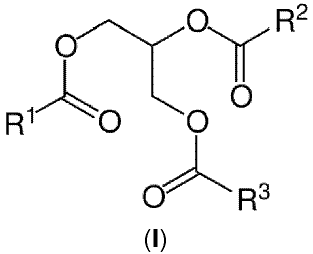 wobei R¹, R² und R³ unabhängig voneinander ausgewählt werden aus -CH₂(CH₂)₅CH₃, -CH₂(CH₂)₆CH₃, -CH₂(CH₂)₇CH₃ und -CH₂(CH₂)₈CH₃.

Es hat sich gezeigt, dass mit gemischten Tri-O-Acylglycerolen, bei denen R¹, R² und R³ unabhängig voneinander ausgewählt werden aus -CH₂(CH₂)₅CH₃, -CH₂(CH₂)₆CH₃, -CH₂(CH₂)₇CH₃ und -CH₂(CH₂)₈CH₃ und wobei nicht alle drei R¹, R² und R³ identisch sind, ebenfalls stabile Kristallsuspension von mikrokristallinen Limus-Wirkstoffen hergestellt werden können. Diese gemischten Tri-O-Acylglycerole sind allerdings aufwendiger in der Herstellung und nicht kosteneffizient, so dass diese hierin nicht bevorzugt sind.

Daher sind gemäß der vorliegenden Erfindung alle drei Reste R¹, R² und R³ identisch, d.h. R¹, R² und R³ sind -CH₂(CH₂)₅CH₃ oder R¹, R² und R³ sind -CH₂(CH₂)₆CH₃ oder R¹, R² und R³ sind -CH₂(CH₂)₇CH₃ oder R¹, R² und R³ sind -CH₂(CH₂)₈CH₃.

In Versuchen zur Herstellung von Kristallsuspensionen enthaltend Tri-O-Acylglycerole, die mit den anderen oben genannten nicht erfindungsgemäßen mittellangen Fettsäuren vollständig verestert sind, insbesondere den kürzeren mittellangen Fettsäuren wie Capronsäure (Tricaproin) aber auch den kürzeren Monocarbonsäuren wie Essigsäure (Triacetin) konnten keine erfindungsgemäßen Kristallsuspensionen hergestellt werden, da in Anwesenheit dieser nicht erfindungsgemäßen Tri-O-Acylglycerole ein An- oder Auflösen der Mikrokristalle des Limus-Wirkstoffes in der Suspension auftrat oder gefördert wurde. Ein Auflösen der Mikrokristalle des Limus-Wirkstoffes in der Suspension trat dabei auch bei mit mittellangen Fettsäuren nur teilweise veresterten Glycerolen, wie den Mono-O-Acylglycerolen oder Di-O-Acylglycerolen auf. Zudem trat bei diesen nicht erfindungsgemäßen Tri-O-Acylglycerolen eine Sedimentation der Mikrokristalle des Limus-Wirkstoffes auf.

Bei jeglicher Abwesenheit von Tri-O-Acylglycerolen in der Suspension erfolgte die Sedimentation der Mikrokristalle des mindestens einen Limus-Wirkstoffs rasch. Die Herstellung einer stabilen Suspension war nicht möglich.

Das Vorliegen von mindestens einem gelösten Tri-O-Acylglycerol ausgewählt aus der Gruppe bestehend aus Trioctanoylglycerol, Trinonanoylglycerol, Tridecanoylglycerol und Triundecanoylglycerol in der Suspension ist somit wesentlich für die Kristallsuspension gemäß der vorliegenden Erfindung.

Es hat sich somit gezeigt, dass das mit drei Octansäure-Molekülen vollständig veresterte Glycerol, das mit drei Decansäure-Molekülen vollständig veresterte Glycerol, das mit drei Nonansäure-Molekülen vollständig veresterte Glycerol oder das mit drei Undecansäure vollständig veresterte Glycerol, also mindestens ein Tri-O-Acylglycerol ausgewählt aus der Gruppe bestehend aus Trioctanoylglycerol, Trinonanoylglycerol, Tridecanoylglycerol und Triundecanoylglycerol, mikrokristalline Limus-Wirkstoffe in einer Suspension nicht an- oder auflösen.

Somit lässt sich mit diesen erfindungsgemäßen Tri-O-Acylglycerolen eine Kristallsuspension als Beschichtungsformulierung bereitstellen, in der die Mikrokristalle des Limus-Wirkstoffs intakt bleiben, in der Suspension schweben und sich in der Suspension gleichmäßig verteilen und keine Sedimentation der Mikrokristalle des Limus-Wirkstoffs oder eine Agglomeration von Partikeln stattfindet, so dass der Limus-Wirkstoff in mikrokristalliner Form gleichmäßig auf eine Medizinprodukteoberfläche aufgebracht werden kann.

Ein weiterer besonderer Vorteil der Verwendung von mindestens einem Tri-O-Acylglycerol ausgewählt aus der Gruppe bestehend aus Trioctanoylglycerol, Trinonanoylglycerol, Tridecanoylglycerol und Triundecanoylglycerol besteht darin, dass die erfindungsgemäßen Tri-O-Acylglycerole in der Lage sind, die Mikrokristalle des Limus-Wirkstoffs wie "Flexkleber" auf einer Medizinprodukteoberfläche festzuhalten, so dass eine ausreichende Haftfähigkeit der Mikrokristalle des Limus-Wirkstoffs auf einer Medizinprodukteoberfläche bereitgestellt werden kann.

Die erfindungsgemäßen Tri-O-Acylglycerole ausgewählt aus der Gruppe bestehend aus Trioctanoylglycerol, Trinonanoylglycerol, Tridecanoylglycerol und Triundecanoylglycerol oder ein Gemisch dieser Tri-O-Acylglycerole haben den Vorteil, dass diese aufgrund ihrer Schmelzpunkte für den Einsatz im Körper gefahrlos einsetzbar sind. Es hat sich zudem herausgestellt, dass ein Schmelzpunkt von unter 37°C wesentlich ist, um eine ausreichende Haftfähigkeit der Mikrokristalle des Limus-Wirkstoffs auf einer Medizinprodukteoberfläche zu gewährleisten. Nur die erfindungsgemäßen Tri-O-Acylglycerole ausgewählt aus der Gruppe bestehend aus Trioctanoylglycerol, Trinonanoylglycerol, Tridecanoylglycerol und Triundecanoylglycerol oder Gemische dieser Tri-O-Acylglycerole können mikrokristalline Limus-Wirkstoffe wie ein _{"}Kleber" festhalten, womit eine optimale Flexibilität und ein verlustfreier Transport an den Zielbereich garantiert wird. Bereits das nächsthöhere Homologe, das Tridodecanoylglycerol hat den Nachteil, dass dieses erst bei 45-46°C schmilzt.

Mit den nicht erfindungsgemäßen Tri-O-Acylglycerolen, die mit den anderen oben genannten nicht erfindungsgemäßen mittellangen Fettsäuren oder langkettigen Fettsäuren vollständig verestert sind, wie Laurinsäure, Myristinsäure oder Palmitinsäure, konnten keine erfindungsgemäßen Kristallsuspension bereitgestellt werden, bei denen die Mikrokristalle des Limus-Wirkstoffs in der Suspension schweben und sich in der Suspension gleichmäßig verteilen. Bei Verwendung von nicht erfindungsgemäßen Tri-O-Acylglycerolen, wie Tridodecanoylglycerol, war eine gleichmäßige Beschichtung mit Limus-Wirkstoff in mikrokristalliner Form auf Medizinprodukteoberflächen nicht möglich. Beschichtete Katheterballons mit einer Beschichtung von Limus-Wirkstoff in mikrokristalliner Form und Tridodecanoylglycerol zeigten deutlich, dass eine gleichmäßige Beschichtung fehlt, die Oberfläche ist uneben und die Beschichtung bröselt bei der Inflation leicht ab.

In Versuchen zur Beschichtung von Medizinprodukten mit Suspensionen enthaltend Mikrokristalle eines Limus-Wirkstoffes und in der Suspension gelösten nicht erfindungsgemäßen Tri-O-Acylglycerolen, die mit den anderen oben genannten nicht erfindungsgemäßen mittellangen Fettsäuren oder langkettigen Fettsäuren vollständig verestert sind, wie Laurinsäure, Myristinsäure oder Palmitinsäure konnten keine Beschichtungen hergestellt werden, bei denen die Mikrokristalle des Limus-Wirkstoffs auf der Medizinprodukteoberfläche gleichmäßig verteilt sind und ausreichend auf der Medizinprodukteoberfläche haften. Für Beschichtungen mit Mikrokristallen eines Limus-Wirkstoffs und einem nicht erfindungsgemäßen Tri-O-Acylglycerol, wie Tridodecanoylglycerol, konnte im "Bröseltest", eine höhere Partikel-Freisetzung beobachtet werden, im Vergleich zu Beschichtungen von Mikrokristallen eines Limus-Wirkstoffs und mindestens einem Tri-O-Acylglycerol ausgewählt aus der Gruppe bestehend aus Trioctanoylglycerol, Trinonanoylglycerol, Tridecanoylglycerol und Triundecanoylglycerol. Der Vergleich zeigte deutlich, dass die Partikel-Freisetzung in allen gemessenen Partikelgrößen für die erfindungsgemäße Beschichtung mit Mikrokristallen eines Limus-Wirkstoffs und mindestens einem Tri-O-Acylglycerol ausgewählt aus der Gruppe bestehend aus Trioctanoylglycerol, Trinonanoylglycerol, Tridecanoylglycerol und Triundecanoylglycerol sehr weit unter der Partikel-Freisetzung für Beschichtungen mit Mikrokristallen eines Limus-Wirkstoffs und einem nicht erfindungsgemäßen Tri-O-Acylglycerol, wie Tridodecanoylglycerol, liegt.

Es hat sich zudem überraschenderweise gezeigt, dass eine herausragende Haftfähigkeit der Mikrokristalle des Limus-Wirkstoff auf der Medizinprodukteoberfläche insbesondere dann erzielt wird, wenn das erfindungsgemäße Tri-O-Acylglycerol in der erfindungsgemäßen Kristallsuspension gelöst vorliegt und mit den in der Suspension suspendierten Mikrokristallen des mindestens einen Limus-Wirkstoffs zusammen bzw. zeitgleich auf die Medizinprodukteoberfläche aufgetragen wird.

Diese herausragende Haftfähigkeit der Mikrokristalle des Limus-Wirkstoffs an der Medizinprodukteoberfläche konnte nicht reproduziert werden, wenn die Medizinprodukteoberfläche zunächst mit einer Lösung enthaltend mindestens ein erfindungsgemäßes Tri-O-Acylglycerol beschichtet worden ist und die Mikrokristalle des Limus-Wirkstoffs im Anschluss auf die Tri-O-Acylglycerol-Schicht aufgetragen worden sind. Auch ergab sich keine ausreichende Haftfähigkeit der Mikrokristalle des Limus-Wirkstoffs, wenn die Medizinprodukteoberfläche zunächst mit Kristallen des Limus-Wirkstoffs gemäß Stand der Technik-Verfahren und anschließend mit einer Lösung enthaltend ein Tri-O-Acylglycerol, in der sich der mikrokristalline Limus-Wirkstoff nicht löst, beschichtet worden ist.

Die mangelnde Haftfähigkeit der Mikrokristalle des Limus-Wirkstoffs auf der Medizinprodukteoberfläche stellte im Stand der Technik bisher das größte Hindernis für die Verwendung von mikrokristallinen Limus-Wirkstoffen zur Beschichtung von Medizinprodukten dar. Die vorliegende Erfindung überwindet dieses Hindernis und bietet eine Lösung, um Beschichtungen mit mikrokristallinen Limus-Wirkstoffen auf Medizinprodukteoberflächen bereitstellen zu können.

**In** der erfindungsgemäßen Suspension liegt das mindestens eine Tri-O-Acylglycerol ausgewählt aus der Gruppe bestehend aus Trioctanoylglycerol, Trinonanoylglycerol, Tridecanoylglycerol und Triundecanoylglycerol vorteilhaft in gelöster Form vor, so dass beim Beschichten eines Medizinprodukts mit einer erfindungsmäßen Suspension eine Beschichtung erhalten wird, bei der nicht nur die Mikrokristalle des Limus-Wirkstoffs gleichmäßig verteilt vorliegen, sondern auch das mindestens eine Tri-O-Acylglycerol in der gesamten Beschichtung gleichmäßig verteilt vorliegt. Dies ist insbesondere dann vorteilhaft, wenn eine Beschichtung von Mikrokristallen eines Limus-Wirkstoffs bereitgestellt werden soll, die eine größere Schichtdicke aufweisen soll und bei der die erfindungsgemäße Suspension mehrfach hintereinander aufgetragen wird. Die erfindungsgemäßen Tri-O-Acylglycerole liegen in so einer Beschichtung dann gleichmäßig verteilt vor und halten die Mikrokristalle des Limus-Wirkstoffs wie "Flexkleber" auf der Medizinprodukteoberfläche, aber auch untereinander wie "Flexkleber" fest.

Somit bietet die Suspension der vorliegenden Erfindung zusätzlich den Vorteil, dass die Haftfähigkeit der Mikrokristalle des mindestens einen Limus-Wirkstoffs auch untereinander erhöht ist. Werden die Mikrokristalle des mindestens einen Limus-Wirkstoffs zunächst ohne ein in der Suspension gelöstes Tri-O-Acylglycerol aufgetragen und in einem anschließenden Schritt mit einer Tri-O-Acylglycerol-Lösung beschichtet, kann die Tri-O-Acylglycerol-Lösung nicht ausreichend unter und zwischen die Mikrokristalle des Limus-Wirkstoffs vordringen und somit die technische Wirkung der erhöhten Haftfähigkeit auf der Medizinprodukteoberfläche und zwischen den Mikrokristallen des mindestens einen Limus-Wirkstoffs nicht ausreichend entfalten.

Mit der Bereitstellung einer Suspension gemäß der vorliegenden Erfindung enthaltend mindestens ein gelöstes Tri-O-Acylglycerol ausgewählt aus der Gruppe bestehend aus Trioctanoylglycerol, Trinonanoylglycerol, Tridecanoylglycerol und Triundecanoylglycerol, und mindestens einen Limus-Wirkstoff in Form von Mikrokristallen konnte das schwerwiegende Problem der mangelnden Haftfähigkeit der Mikrokristalle des Limus-Wirkstoffs auf der Medizinprodukteoberfläche nun überraschenderweise gelöst werden.

Die Beschichtung von Medizinprodukten mit mikrokristallinen Limus-Wirkstoffen in Anwesenheit von mindestens einem Tri-O-Acylglycerol ausgewählt aus der Gruppe bestehend aus Trioctanoylglycerol, Trinonanoylglycerol, Tridecanoylglycerol und Triundecanoylglycerol zeigen zudem ein im Vergleich zu den auf dem Markt erhältlichen Medizinprodukten ein wesentlich vermindertes "Bröselverhalten", da sie eine deutlich reduzierte Partikel-Freisetzung im Vergleich zu den zur Zeit auf dem Markt erhältlichen Medizinprodukten, speziell im Bereich von wirkstoffabgebenden Ballonkathetern zeigen, womit erwiesen ist, dass die Stabilität einer Beschichtung gemäß der vorliegenden Erfindung gleichermaßen erhöht ist. Dies ergibt sich insbesondere aus dem Vorteil, dass das mindestens eine Tri-O-Acylglycerol ausgewählt aus der Gruppe bestehend aus Trioctanoylglycerol, Trinonanoylglycerol, Tridecanoylglycerol und Triundecanoylglycerol, die Mikrokristalle des Limus-Wirkstoffs wie "Flexkleber" fest auf der Medizinprodukteoberfläche halten, so dass sich eine stabile, nicht bröselige und flexible Beschichtung ergibt.

Zudem hat sich gezeigt, dass die Tri-O-Acylglycerole ausgewählt aus der Gruppe bestehend aus Trioctanoylglycerol, Trinonanoylglycerol, Tridecanoylglycerol und Triundecanoylglycerol oder Gemische dieser Tri-O-Acylglycerole noch weitere Vorteile aufweisen. Sie ermöglichen unter anderem eine optimale Übertragung des mikrokristallinen Limus-Wirkstoffes in ein Gewebe. Die mit der erfindungsgemäßen Suspension hergestellten Beschichtungen weisen eine gleichmäßigere, einheitlichere Oberfläche und uniforme Verteilung der Mikrokristalle des Limus-Wirkstoffs auf der Medizinoberfläche auf, als dies bei den bekannten auf dem Markt erhältlichen beschichteten Medizinprodukten ist. Diese vorteilhaften Eigenschaften des mindestens einen Tri-O-Acylglycerol ausgewählt aus der Gruppe bestehend aus Trioctanoylglycerol, Trinonanoylglycerol, Tridecanoylglycerol und Triundecanoylglycerol führen somit zu einer optimalen Wirkstoffübertragung und - elution ins Gewebe, wie auch zur einer optimalen Wirkstoffverteilung am Implantationsort.

Die für die vorliegende Erfindung wesentlichen Tri-O-Acylglycerole ausgewählt aus der Gruppe bestehend aus Trioctanoylglycerol, Trinonanoylglycerol, Tridecanoylglycerol und Triundecanoylglycerol oder Gemische dieser Tri-O-Acylglycerole erfüllen somit unter anderem folgende wichtige Aufgaben: Sie sind die mikrokristallinen Limus-Wirkstoffträger und beeinflussen damit die mechanischen Eigenschaften der Beschichtung ("drug carrier") wie z.B. die Haftung auf der Medizinprodukteoberfläche, sorgen für möglichst geringen Verlust an mikrokristallinem Limus-Wirkstoffs während der Implantation ("drug transit loss"), aber ebenso beeinflussen sie die Partikelgrößenverteilung der Beschichtung und damit das "Bröselverhalten" ("particle release"), womit die Brüchigkeit bzw. Geschmeidigkeit und Anpassungsfähigkeit der Beschichtung vor und bei der Implantation und der damit einhergehenden Formveränderung gemeint ist. Die Gleichmäßigkeit der Beschichtung ("uniformity") gilt als weiterer wichtiger Parameter, da mit einer gleichmäßigen Beschichtung auch eine gleichmäßige Wirkstoffverteilung der Mikrokristalle des Limus-Wirkstoffs auf der Medizinprodukteoberfläche und damit auch eine gleichmäßige Verteilung des mikrokristallinen Limus-Wirkstoffes in das umliegende Gewebe erzielt werden kann. Zudem beschleunigen bzw. erleichtern sie als "Katalysator" die Wirkstoffübertragung der Mikrokristalle des Limus-Wirkstoffs in das umliegende Gewebe, ohne den mikrokristallinen Limus-Wirkstoff zu verändern und seine Wirksamkeit zu beeinflussen ("drug transfer promoter").

Die erfindungsgemäßen Tri-O-Acylglycerole ausgewählt aus der Gruppe bestehend aus Trioctanoylglycerol, Trinonanoylglycerol, Tridecanoylglycerol und Triundecanoylglycerol oder Gemische dieser Tri-O-Acylglycerole sind somit für Beschichtungen von Medizinprodukten mit Mikrokristallen eines Limus-Wirkstoffs besonders vorteilhaft, da die resultierenden Beschichtungen verlustfrei an der Oberfläche des Medizinproduktes haften, Formveränderungen des Untergrundes z.B. Dehnungen problemlos und ohne vorzeitige Ablösung oder gar Auflösung mitmachen, den eingebetteten oder aufliegenden mindestens einen mikrokristallinen Limus-Wirkstoff nicht vorzeitig "verlieren", aber die Mikrokristalle des mindestens einen Limus-Wirkstoffs vor Ort problemlos an der vorbestimmten Stelle abgeben. Dabei nimmt die Beschichtung auch bei starken Formveränderungen, z.B. durch Falten, Expansion und Deflation eines derart beschichteten Ballonkatheters, keinen Schaden.

Somit wird die Aufgabe der vorliegenden Erfindung hinsichtlich der ausreichenden Wirkstoffhaftung und Wirkstofffreigabe des mikrokristallinen Limus-Wirkstoffs über die erfindungsgemäße Suspension, welche mindestens ein Tri-O-Acylglycerol ausgewählt aus der Gruppe bestehend aus Trioctanoylglycerol, Trinonanoylglycerol, Tridecanoylglycerol und Triundecanoylglycerol in gelöster Form enthält, gelöst.

Der Begriff **"Trioctanoylglycerol"** bzw. "Tri-O-Octanoylglycerol", wie hierin verwendet, bezieht sich auf ein Tri-O-Acylglycerol, bei dem das Glycerol vollständig mit Caprylsäure bzw. Octansäure verestert ist. Aus dem Stand der Technik bekannte synonyme Bezeichnungen für Trioctanoylglycerol lauten Trioctanoylglycerid, Glycerin-Trioctanoin, Tricapryloylglycerin, Octansäure-1,1',1 "-(1,2,3-Propantriyl)ester, Glycerintricaprylat, Tricaprylylglycerin, TG(8:0/8:0/8:0), Glycerintricaprylat, Caprylsäure-1,2,3-Propantriyl-Ester, Caprylin, Octansäuretriglycerid, Tricapryl-Glycerid, Tricaprylin, Trioctanoylglycerin, Octansäure-1,2,3-Propantriyl-Ester, Glycerin-Trioctanoat, 1,2,3-Propantrioltrioctanoat, Caprylsäuretriglycerid, Glycerintricaprylat, Caprylisches Triglycerid, Tricaprylin, Tricaprylyl-Glycerin, 1,2,3-Trioctanoylglycerin, Glycerin-trioctanoat und 1,2,3-Tricapryloyl-glycerin. Trioctanoylglycerol hat die CAS-Nummer 538-23-8, ein Molekulargewicht von 470,68 g/mol und weist die folgende Strukturformel auf:

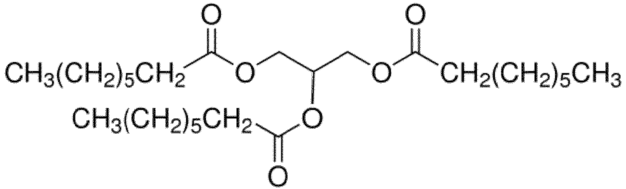

Octansäure ist eine Carbonsäure, die unter dem Trivialnamen Caprylsäure bekannt ist, und eine gesättigte Fettsäure der folgenden Strukturformel darstellt:

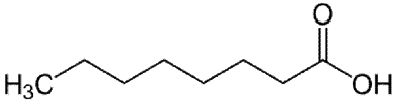

In bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens Trioctanoylglycerol. In bevorzugten Ausführungsformen der vorliegenden Erfindung wird das mindestens eine Tri-O-Acylglycerol somit bevorzugt ausgewählt aus Trioctanoylglycerol.

Der Schmelzpunkt von Trioctanoylglycerol liegt im Bereich von 9-10°C. Trioctanoylglycerol liegt unter Normalbedingungen (20°C, 101 hPa) als geruchlose klare farblose bis bernsteinfarbene Flüssigkeit vor. Trioctanoylglycerol ist praktisch unlöslich in Wasser.

Der Begriff "Trinonanoylglycerol" bzw. "Tri-O-Nonanoylglycerol", wie hierin verwendet, bezieht sich auf ein Tri-O-Acylglycerol, bei dem das Glycerol vollständig mit Pelargonsäure bzw. Nonansäure verestert ist. Aus dem Stand der Technik bekannte synonyme Bezeichnungen für Tridecanoylglycerol lauten Glycerintripelargonat, Trinonanoin, 1,2,3-Trinonanoylglycerin, Tripelargonin, 1,2,3-Tripelargonoyl-glycerin. Trinonanoylglycerol hat die CAS-Nummer 126-53-4, ein Molekulargewicht von 512,76 g/mol und weist die folgende Strukturformel auf:

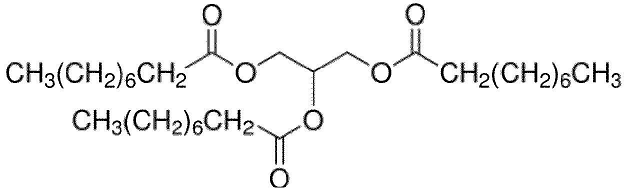

Nonansäure ist eine Carbonsäure, die unter dem Trivialnamen Perlagonsäure bekannt ist, und eine gesättigte Fettsäure der folgenden Strukturformel darstellt:

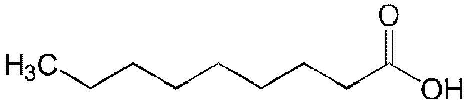

In bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens Trinonanoylglycerol. In bevorzugten Ausführungsformen der vorliegenden Erfindung wird das mindestens eine Tri-O-Acylglycerol somit bevorzugt ausgewählt aus Trinonanoylglycerol.

Der Schmelzpunkt von Trinonanoylglycerol liegt im Bereich von 8-9°C. Trinonanoylglycerol liegt unter Normalbedingungen (20°C, 101 hPa) als Flüssigkeit vor. Trinonanoylglycerol ist praktisch unlöslich in Wasser.

Der Begriff "Tridecanoylglycerol" bzw. "Tri-O-Decanoylglycerol", wie hierin verwendet, bezieht sich auf ein Tri-O-Acylglycerol, bei dem das Glycerol vollständig mit Caprinsäure bzw. Decansäure verestert ist. Aus dem Stand der Technik bekannte synonyme Bezeichnungen für Tridecanoylglycerol lauten Glycerin-tris-(decanoat), 1,2,3-Tricaprinoyl-glycerin, Tricaprin, 1,2,3-Tridecanoylglycerin, Glycerintridecanoat, Tridecanoin. Tridecanoylglycerol hat die CAS-Nummer 621-71-6, ein Molekulargewicht von 554,84 g/mol und weist die folgende Strukturformel auf:

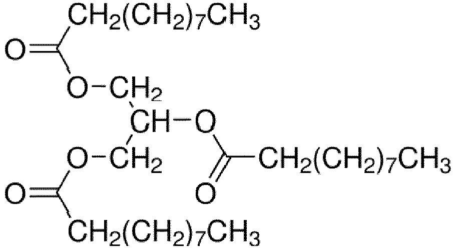

Decansäure ist eine Carbonsäure, die unter dem Trivialnamen Caprinsäure bekannt ist, und eine gesättigte Fettsäure der folgenden Strukturformel darstellt:

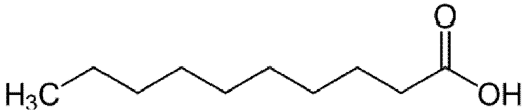

In bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens Tridecanoylglycerol. In bevorzugten Ausführungsformen der vorliegenden Erfindung wird das mindestens eine Tri-O-Acylglycerol somit bevorzugt ausgewählt aus Tridecanoylglycerol.

Der Schmelzpunkt von Tridecanoylglycerol liegt im Bereich von 31-33°C. Tridecanoylglycerol liegt unter Normalbedingungen (20°C, 101 hPa) als blassgelber Feststoff vor. Tridecanoylglycerol ist praktisch unlöslich in Wasser.

Der Begriff **"Triundecanoylglycerol"** bzw. "Tri-O-Undecanoylglycerol", wie hierin verwendet, bezieht sich auf ein Tri-O-Acylglycerol, bei dem das Glycerol vollständig mit Undecansäure verestert ist. Aus dem Stand der Technik bekannte synonyme Bezeichnungen für Triundecanoylglycerol lauten Glycerin-triundecanoat, Triundecanoin, 1,2,3-Triundecanoylglycerin, Triundecanin. Der IUPAC-Name ist 1,3-Bis(undecanoyloxy)propan-2-yl-undecanoat. Triundecanoylglycerol hat die CAS-Nummer 13552-80-2, ein Molekulargewicht von 596,9 g/mol und weist die folgende Strukturformel auf:

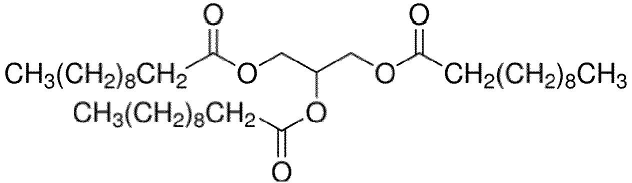

Undecansäure ist eine gesättigte Fettsäure der folgenden Strukturformel:

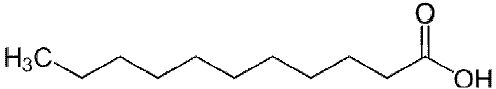

In bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens Triundecanoylglycerol. In bevorzugten Ausführungsformen der vorliegenden Erfindung wird das mindestens eine Tri-O-Acylglycerol somit bevorzugt ausgewählt aus Triundecanoylglycerol.

Der Schmelzpunkt von Triundecanoylglycerol liegt im Bereich von 30-32°C. Triundecanoylglycerol liegt unter Normalbedingungen (20°C, 101 hPa) als Feststoff vor. Triundecanoylglycerol ist praktisch unlöslich in Wasser.

Somit enthält die Suspension der vorliegenden Erfindung zur Beschichtung eines Medizinprodukts ausgewählt aus einem Katheterballon, einem Ballonkatheter, einem Stent oder einer Kanüle erfindungsgemäß mindestens ein Tri-O-Acylglycerol ausgewählt aus der Gruppe bestehend aus Trioctanoylglycerol, Trinonanoylglycerol, Tridecanoylglycerol und Triundecanoylglycerol.

Mit anderen Worten enthält die Suspension der vorliegenden Erfindung zur Beschichtung eines Medizinprodukts ausgewählt aus einem Katheterballon, einem Ballonkatheter, einem Stent oder einer Kanüle erfindungsgemäß mindestens ein Tri-O-Acylglycerol ausgewählt aus der Gruppe bestehend aus Tri-O-Octanoylglycerol, Tri-O-Nonanoylglycerol, Tri-O-Decanoylglycerol und Tri-O-Undecanoylglycerol.

Anders formuliert enthält die Suspension der vorliegenden Erfindung zur Beschichtung eines Medizinprodukts ausgewählt aus einem Katheterballon, einem Ballonkatheter, einem Stent oder einer Kanüle erfindungsgemäß entweder ein Tri-O-Acylglycerol ausgewählt aus der Gruppe bestehend aus Trioctanoylglycerol, Trinonanoylglycerol, Tridecanoylglycerol und Triundecanoylglycerol oder ein Gemisch aus mindestens zwei Tri-O-Acylglycerolen ausgewählt aus der Gruppe bestehend aus Trioctanoylglycerol, Trinonanoylglycerol, Tridecanoylglycerol und Triundecanoylglycerol.

Noch anders formuliert enthält die Suspension der vorliegenden Erfindung zur Beschichtung eines Medizinprodukts ausgewählt aus einem Katheterballon, einem Ballonkatheter, einem Stent oder einer Kanüle erfindungsgemäß entweder ein Tri-O-Acylglycerol ausgewählt aus der Gruppe bestehend aus Trioctanoylglycerol, Trinonanoylglycerol, Tridecanoylglycerol und Triundecanoylglycerol oder ein Gemisch aus zwei, drei oder vier Tri-O-Acylglycerolen ausgewählt aus der Gruppe bestehend aus Trioctanoylglycerol, Trinonanoylglycerol, Tridecanoylglycerol und Triundecanoylglycerol.

Die Formulierung "mindestens ein Tri-O-Acylglycerol ausgewählt aus der Gruppe bestehend aus Trioctanoylglycerol, Trinonanoylglycerol, Tridecanoylglycerol und Triundecanoylglycerol" bezieht sich hierin somit ebenfalls auf Gemische der Tri-O-Acylglycerole ausgewählt aus der Gruppe bestehend aus Trioctanoylglycerol, Trinonanoylglycerol, Tridecanoylglycerol und Triundecanoylglycerol. Der Ausdruck "mindestens ein Tri-O-Acylglycerol" schließt daher Formulierung wie "mindestens zwei Tri-O-Acylglycerole", "mindestens drei Tri-O-Acylglycerole", "zwei Tri-O-Acylglycerole", "drei Tri-O-Acylglycerole" und "vier Tri-O-Acylglycerole" ein.

In einigen Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens zwei Tri-O-Acylglycerole ausgewählt aus der Gruppe bestehend aus Trioctanoylglycerol, Trinonanoylglycerol, Tridecanoylglycerol und Triundecanoylglycerol.

In einigen Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens drei Tri-O-Acylglycerole ausgewählt aus der Gruppe bestehend aus Trioctanoylglycerol, Trinonanoylglycerol, Tridecanoylglycerol und Triundecanoylglycerol.

In einigen Ausführungsformen der vorliegenden Erfindung enthält die Suspension zur Beschichtung eines Medizinprodukts ausgewählt aus einem Katheterballon, einem Ballonkatheter, einem Stent oder einer Kanüle, Trioctanoylglycerol, Trinonanoylglycerol, Tridecanoylglycerol und Triundecanoylglycerol.

Die erfindungsgemäßen Tri-O-Acylglycerole ausgewählt aus der Gruppe bestehend aus Trioctanoylglycerol, Trinonanoylglycerol, Tridecanoylglycerol und Triundecanoylglycerol weisen einen Schmelzpunkt von unter 37° C auf, so dass diese bei Körpertemperatur geschmolzen vorliegen oder bei Körpertemperatur schmelzen oder erweichen.

In einigen Ausführungsformen der vorliegenden Erfindung ist bevorzugt, dass Tri-O-Acylglycerole, die unter Normalbedingungen (20°C, 101 hPa) als Flüssigkeit vorliegen, wie Trioctanoylglycerol oder Trinonanoylglycerol, insbesondere bevorzugt Trioctanoylglycerol zur Herstellung der erfindungsgemäßen Suspensionen eingesetzt werden. Insbesondere mit Trioctanoylglycerol konnten exzellente stabile, nicht bröselige und flexible Beschichtungen erhalten werden. In einigen Ausführungsformen sind zudem Gemische von Trioctanoylglycerol, Trinonanoylglycerol, Tridecanoylglycerol und Triundecanoylglycerol bevorzugt, die mindestens Trioctanoylglycerol und/oder Trinonanoylglycerol, insbesondere bevorzugt Trioctanoylglycerol enthalten, da die resultierenden Gemische unter Normalbedingungen (20°C, 101 hPa) als Flüssigkeit vorliegen. Ein hierin besonders bevorzugtes Gemisch von Tri-O-Acylglycerolen ist ein Gemisch von Trioctanoylglycerol und Tridecanoylglycerol.

In einigen weiteren Ausführungsformen der vorliegenden Erfindung ist bevorzugt, dass Tri-O-Acylglycerole, die unter Normalbedingungen (20°C, 101 hPa) als Feststoff vorliegen, wie Tridecanoylglycerol oder Triundecanoylglycerol, zur Herstellung der erfindungsgemäßen Suspensionen eingesetzt werden. Insbesondere mit Tridecanoylglycerol konnten hervorragende stabile, nicht bröselige und flexible Beschichtungen erhalten werden. Da die Körpertemperatur immer noch höher liegt als die Schmelzpunkte dieser Tri-O-Acylglycerole, liegen Tridecanoylglycerol oder Triundecanoylglycerol während der Implantation bei Körpertemperatur geschmolzen vor, so dass insbesondere während der Inflation von Medizinprodukten, wie Katheterballons oder Stents, keine Nachteile bezüglich der Partikel-Freisetzung und damit der Bröseligkeit im Vergleich zu den Tri-O-Acylglycerolen, die unter Normalbedingungen (20°C, 101hPa) als Flüssigkeit vorliegen, wie Trioctanoylglycerol oder Trinonanoylglycerol, bestehen. Zudem kann, falls erforderlich oder gewünscht, das beschichtete Medizinprodukt auch noch vor der Implantation erwärmt werden, so dass das Tridecanoylglycerol oder Triundecanoylglycerol bereits vor der Implantation schmilzt oder erweicht und somit zu Beginn der Implantation bereits geschmolzen vorliegen.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle somit mindestens ein Tri-O-Acylglycerol ausgewählt aus der Gruppe bestehend aus Trioctanoylglycerol, Trinonanoylglycerol, Tridecanoylglycerol und Triundecanoylglycerol, weiter bevorzugt mindestens ein Tri-O-Acylglycerol ausgewählt aus Trioctanoylglycerol, Trinonanoylglycerol und Tridecanoylglycerol, noch weiter bevorzugt mindestens ein Tri-O-Acylglycerol ausgewählt aus der Gruppe bestehend aus Trioctanoylglycerol und Tridecanoylglycerol, weiter bevorzugt mindestens ein Tri-O-Acylglycerol ausgewählt aus der Gruppe bestehend aus Trioctanoylglycerol oder mindestens ein Tri-O-Acylglycerol ausgewählt aus Tridecanoylglycerol.

In einigen weiteren bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle ein Gemisch von Tri-O-Acylglycerolen ausgewählt aus der Gruppe bestehend aus Trioctanoylglycerol, Trinonanoylglycerol, Tridecanoylglycerol und Triundecanoylglycerol. In einigen dieser bevorzugten Ausführungsformen enthält die Suspension zur Beschichtung eines Medizinprodukts, vorzugsweise ausgewählt aus einem Katheterballon, einem Ballonkatheter, einem Stent oder einer Kanüle, ein Gemisch aus Trioctanoylglycerol, Trinonanoylglycerol und Tridecanoylglycerol oder ein Gemisch aus Trioctanoylglycerol, Trinonanoylglycerol und Triundecanoylglycerol oder ein Gemisch aus Trioctanoylglycerol, Tridecanoylglycerol und Triundecanoylglycerol oder ein Gemisch aus Trinonanoylglycerol, Tridecanoylglycerol und Triundecanoylglycerol, weiter bevorzugt ein Gemisch aus Trioctanoylglycerol, Trinonanoylglycerol und Tridecanoylglycerol oder ein Gemisch aus Trioctanoylglycerol, Tridecanoylglycerol und Triundecanoylglycerol, noch weiter bevorzugt ein Gemisch aus Trioctanoylglycerol, Trinonanoylglycerol und Tridecanoylglycerol.

In einigen bevorzugten Ausführungsformen enthält die Suspension zur Beschichtung eines Medizinprodukts ausgewählt aus einem Katheterballon, einem Ballonkatheter, einem Stent oder einer Kanüle ein Gemisch aus Trioctanoylglycerol und Tridecanoylglycerol oder ein Gemisch aus Trioctanoylglycerol und Trinonanoylglycerol oder ein Gemisch aus Trioctanoylglycerol und Triundecanoylglycerol oder ein Gemisch aus Trinonanoylglycerol und Tridecanoylglycerol oder ein Gemisch aus Trinonanoylglycerol und Triundecanoylglycerol oder ein Gemisch aus Tridecanoylglycerol und Triundecanoylglycerol.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, ein Gemisch aus Tridecanoylglycerol und Trinonanoylglycerol oder ein Gemisch aus Tridecanoylglycerol und Triundecanoylglycerol oder ein Gemisch aus Tridecanoylglycerol und Trioctanoylglycerol, weiter bevorzugt ein Gemisch aus Tridecanoylglycerol und Trinonanoylglycerol oder ein Gemisch aus Tridecanoylglycerol und Trioctanoylglycerol, und am meisten bevorzugt ein Gemisch aus Tridecanoylglycerol und Trioctanoylglycerol.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, ein Gemisch aus Trioctanoylglycerol und Trinonanoylglycerol oder ein Gemisch aus Trioctanoylglycerol und Triundecanoylglycerol oder ein Gemisch aus Trioctanoylglycerol und Tridecanoylglycerol, weiter bevorzugt ein Gemisch aus Trioctanoylglycerol und Trinonanoylglycerol oder ein Gemisch aus Trioctanoylglycerol und Tridecanoylglycerol, und am meisten bevorzugt ein Gemisch aus Trioctanoylglycerol und Tridecanoylglycerol.

In am meisten bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens ein Tri-O-Acylglycerol ausgewählt aus der Gruppe bestehend aus Trioctanoylglycerol und Tridecanoylglycerol oder ein Gemisch aus Trioctanoylglycerol und Tridecanoylglycerol.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens ein Tri-O-Acylglycerol ausgewählt aus Trioctanoylglycerol oder ein Gemisch aus Trioctanoylglycerol und mindestens einem Tri-O-Acylglycerol ausgewählt aus der Gruppe bestehend aus Trinonanoylglycerol, Tridecanoylglycerol und Triundecanoylglycerol, bevorzugt Trinonanoylglycerol und Tridecanoylglycerol, noch weiter bevorzugt Tridecanoylglycerol.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, Trioctanoylglycerol oder ein Gemisch aus Trioctanoylglycerol und mindestens einem weiteren Tri-O-Acylglycerol ausgewählt aus der Gruppe bestehend aus Trinonanoylglycerol, Tridecanoylglycerol und Triundecanoylglycerol, bevorzugt Trinonanoylglycerol und Tridecanoylglycerol, noch weiter bevorzugt Tridecanoylglycerol.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, mindestens ein Tri-O-Acylglycerol ausgewählt aus Tridecanoylglycerol, oder ein Gemisch aus Tridecanoylglycerol und mindestens einem weiteren Tri-O-Acylglycerol ausgewählt aus der Gruppe bestehend aus Trinonanoylglycerol, Trioctanoylglycerol und Triundecanoylglycerol, bevorzugt Trinonanoylglycerol und Trioctanoylglycerol, noch weiter bevorzugt Trioctanoylglycerol.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, Tridecanoylglycerol, oder ein Gemisch aus Tridecanoylglycerol und mindestens einem weiteren Tri-O-Acylglycerol ausgewählt aus der Gruppe bestehend aus Trinonanoylglycerol, Trioctanoylglycerol und Triundecanoylglycerol, bevorzugt Trinonanoylglycerol und Trioctanoylglycerol, noch weiter bevorzugt Trioctanoylglycerol.

Das mindestens eine Tri-O-Acylglycerol ausgewählt aus der Gruppe bestehend aus Trioctanoylglycerol, Trinonanoylglycerol, Tridecanoylglycerol und Triundecanoylglycerol weist bevorzugt mindestens eine Reinheit von ≥90%, bevorzugt ≥95% und insbesondere bevorzugt von ≥99% auf. Ein Gemisch der Tri-O-Acylglycerole ausgewählt aus der Gruppe bestehend aus Trioctanoylglycerol, Trinonanoylglycerol, Tridecanoylglycerol und Triundecanoylglycerol besteht vorzugsweise mindestens zu ≥90%, bevorzugt ≥95% und insbesondere bevorzugt zu ≥99% aus den Tri-O-Acylglycerolen ausgewählt aus der Gruppe bestehend aus Trioctanoylglycerol, Trinonanoylglycerol, Tridecanoylglycerol und Triundecanoylglycerol.

Besonders bevorzugt sind in der erfindungsgemäßen Suspension neben dem mindestens einen Tri-O-Acylglycerol ausgewählt aus der Gruppe bestehend aus Trioctanoylglycerol, Trinonanoylglycerol, Tridecanoylglycerol und Triundecanoylglycerol keine weiteren Tri-O-Acylglycerole enthalten.

Beispielsweise sind Trioctanoylglycerol und Tridecanoylglycerol auch als natürliche Bestandteile in verschiedenen Pflanzenölen, wie Sojaöl, Olivenöl oder Kokosnussöl oder tierischen Ölen enthalten. Diese natürlichen Pflanzenöle oder tierischen Öle enthalten jedoch auch weitere nicht erfindungsgemäße gesättigte und auch ungesättigte Tri-O-Acylglycerole in verschiedenen Anteilen oder auch weitere Substanzen wie z.B. Mono-O-Acylglycerole, Di-O-Acylglycerole, Fettsäuren und Lipide, so dass natürliche Pflanzenöle oder tierischen Öle für die Herstellung von erfindungsgemäßen Kristallsuspensionen nicht geeignet sind. Pflanzenöle oder tierische Öle können für die Herstellung von erfindungsgemäßen Kristallsuspensionen eingesetzt werden, sofern diese mindestens zu ≥90%, bevorzugt ≥95% und insbesondere bevorzugt von ≥99% aus mindestens einem Tri-O-Acylglycerol ausgewählt aus der Gruppe bestehend aus Trioctanoylglycerol, Trinonanoylglycerol, Tridecanoylglycerol und Triundecanoylglycerol bestehen.

Nicht erfindungsgemäße Gemische von Tri-O-Acylglycerolen sind hierin daher Öle wie z.B. Leinöl, Hanföl, Maiskeimöl, Walnussöl, Rapsöl, Sojaöl, Sonnenblumenöl, Mohnöl, Safloröl (Färberdistelöl), Weizenkeimöl, Distelöl, Traubenkernöl, Nachtkerzenöl, Borretschöl, Schwarzkümmelöl, Algenöl, Fischöl, Lebertran, Kokosöl Leinsamenöl, Baumwollsamenöl und/oder Mischungen der vorgenannten Öle.

Für die Herstellung der erfindungsgemäßen Suspension enthaltend mindestens ein Tri-O-Acylglycerol ausgewählt aus der Gruppe bestehend aus Trioctanoylglycerol, Trinonanoylglycerol, Tridecanoylglycerol und Triundecanoylglycerol, wird das mindestens eine Tri-O-Acylglycerol entweder als chemisch reiner Stoff für die Herstellung der erfindungsgemäßen Kristallsuspension verwendet oder ein Gemisch eingesetzt, dass mindestens zu ≥90%, bevorzugt ≥95% und insbesondere bevorzugt von ≥99% aus mindestens einem Tri-O-Acylglycerol ausgewählt aus der Gruppe bestehend aus Trioctanoylglycerol, Trinonanoylglycerol, Tridecanoylglycerol und Triundecanoylglycerol besteht. Das mindestens eine Tri-O-Acylglycerol ausgewählt aus der Gruppe bestehend aus Trioctanoylglycerol, Trinonanoylglycerol, Tridecanoylglycerol und Triundecanoylglycerol kann selbstverständlich auch aus einem natürlichen Pflanzenöl oder tierischen Öl gewonnen werden, z.B. sind die Tri-O-Acylglycerole Trioctanoylglycerol und Tridecanoylglycerol und Gemische von Trioctanoylglycerol und Tridecanoylglycerol unter den Handelsnamen Captex^{®} 8000 (Trioctanoylglycerol), Captex^{®} 1000 (Tridecanoylglycerol), Captex^{®} 300 (Trioctanoylglycerol/Tridecanoylglycerol), Captex^{®} 355 (Trioctanoylglycerol/ Tridecanoylglycerol, Miglyol^{®} 810 (Trioctanoylglycerol : Tridecanoylglycerol, ca. 70:30), und Miglyol^{®} 812 (Trioctanoylglycerol : Tridecanoylglycerol, ca. 50:50) kommerziell erhältlich. Solche kommerziell erhältlichen Gemische können zur Herstellung einer Suspension der vorliegenden Erfindung verwendet werden.

In einigen bevorzugten Ausführungsformen der vorliegenden Erfindung enthält die Suspension zur Beschichtung eines Medizinprodukts ausgewählt aus einem Katheterballon, einem Ballonkatheter, einem Stent oder einer Kanüle, somit ein Gemisch aus mindestens zwei Tri-O-Acylglycerolen ausgewählt aus der Gruppe bestehend aus Trioctanoylglycerol, Trinonanoylglycerol, Tridecanoylglycerol und Triundecanoylglycerol, wobei das Gemisch mindestens zu ≥90%, bevorzugt ≥95% und insbesondere bevorzugt zu ≥99% aus mindestens zwei Tri-O-Acylglycerolen ausgewählt aus der Gruppe bestehend aus Trioctanoylglycerol, Trinonanoylglycerol, Tridecanoylglycerol und Triundecanoylglycerol besteht.

Der Begriff **"Gewichtsprozent"** (Abk.: Gew.-%), wie hierin verwendet, bezieht sich auf den Anteil eines Stoffes in einem Gemisch oder einer Lösung, der in Gramm je 100 g Mischung gemessen wird. Der Begriff Gewichtsprozent ist hierin eine Bezeichnung für den Massenanteil eines Gemisches.

Der Begriff **"Massenanteil",** wie hierin verwendet, bezieht sich auf eine physikalisch-chemische Größe zur quantitativen Beschreibung der Zusammensetzung von Stoffgemischen. Die Masse einer betrachteten Mischungskomponente wird auf die Summe der Massen aller Mischungskomponenten bezogen, der Massenanteil gibt den relativen Anteil der Masse einer betrachteten Mischungskomponente an der Gesamtmasse des Gemisches an.

In bevorzugten Ausführungsformen beträgt der Massenanteil (m/m; [g]/[g]; m=Masse) von Tri-O-Acylglycerol an der Gesamtmasse von Tri-O-Acylglycerol und mikrokristallinem Limus-Wirkstoff in der Suspension vorzugsweise 5-40%, weiter bevorzugt 10-30% und am meisten bevorzugt 20%. Die Masse von Tri-O-Acylglycerol bezieht sich hierbei auf die Gesamtmasse von Tri-O-Acylglycerol ausgewählt aus der Gruppe bestehend aus Trioctanoylglycerol, Trinonanoylglycerol, Tridecanoylglycerol und Triundecanoylglycerol, d.h. bei Gemischen von Tri-O-Acylglycerole ausgewählt aus der Gruppe bestehend aus Trioctanoylglycerol, Trinonanoylglycerol, Tridecanoylglycerol und Triundecanoylglycerol wird der Massenanteil der Masse des Gemisches an der Gesamtmasse von Tri-O-Acylglycerol-Gemisch und mikrokristallinem Limus-Wirkstoff berechnet.

Zur Berechnung des Massenanteils von Tri-O-Acylglycerol an der Gesamtmasse von Tri-O-Acylglycerol und mikrokristallinen Limus-Wirkstoff in der Suspension wird somit der Quotient aus der Masse von Tri-O-Acylglycerol und der Gesamtmasse von Tri-O-Acylglycerol und mikrokristallinem Limus-Wirkstoff gebildet. Beispielsweise beträgt der Massenanteil von Tri-O-Acylglycerol in einer Mischung aus 4 g Limus-Wirkstoff und 1 g Tri-O-Acylglycerol 20%.

In bevorzugten Ausführungsformen beträgt der Massenanteil (m/m; [g]/[g]; m=Masse) von Tri-O-Acylglycerol an der Gesamtmasse von Tri-O-Acylglycerol und mikrokristallinem Limus-Wirkstoff in der Suspension vorzugsweise 0,1-50%, weiterhin bevorzugt 1-40%, besonders bevorzugt 10-30% und am meisten bevorzugt 20%.

In bevorzugten Ausführungsformen beträgt der Massenanteil (m/m; [g]/[g]; m=Masse) von Limus-Wirkstoff an der Gesamtmasse von Tri-O-Acylglycerol und mikrokristallinem Limus-Wirkstoff in der Suspension vorzugsweise 99,9-50%, weiterhin bevorzugt 99-60%, besonders bevorzugt 90-70% und am meisten bevorzugt 80%.

Somit betragen die Massenanteile von Tri-O-Acylglycerol und mikrokristallinem Limus-Wirkstoff an der Gesamtmasse von Tri-O-Acylglycerol und mikrokristallinen Limus-Wirkstoff vorzugsweise 0,1-50% Tri-O-Acylglycerol und 99,9-50% Limus-Wirkstoff, weiterhin bevorzugt 1-40% Tri-O-Acylglycerol und 99-60% Limus-Wirkstoff, besonders bevorzugt 10-30% Tri-O-Acylglycerol und 90-70% Limus-Wirkstoff und am meisten bevorzugt 20% Tri-O-Acylglycerol und 80% Limus-Wirkstoff.

Werden Gemische von Tri-O-Acylglycerolen eingesetzt, wird der Massenanteil der Masse des Tri-O-Acylglycerol-Gemisches an der Gesamtmasse an Tri-O-Acylglycerol-Gemisch und mikrokristallinen Limus-Wirkstoff bestimmt. Beispielsweise beträgt der Massenanteil von Tri-O-Acylglycerol-Gemisch in einer Mischung aus 4 g Limus-Wirkstoff und 1 g Tri-O-Acylglycerol-Gemisch 20%.

**In** bevorzugten Ausführungsformen liegt der Anteil an Tri-O-Acylglycerol bezogen auf den Limus-Wirkstoff vorzugsweise bei 0,1-50 Gew.%, weiterhin bevorzugt mit 1-40 Gew.%, besonders bevorzugt mit 10-30 Gew.% und am meisten bevorzugt mit 20 Gew. %.

In bevorzugten Ausführungsformen liegt der Anteil an Limus-Wirkstoff bezogen auf das Tri-O-Acylglycerol vorzugsweise bei 99,9-50 Gew.%, weiterhin bevorzugt mit 99-60 Gew.%, besonders bevorzugt 90-70 Gew.% und am meisten bevorzugt mit 80 Gew.% in der Suspension vor.

Somit liegen das Tri-O-Acylglycerol und der mikrokristallinem Limus-Wirkstoff in der Suspension vorzugsweise mit 0,1-50 Gew.% Tri-O-Acylglycerol zu 99,9-50 Gew.% Limus-Wirkstoff, weiterhin bevorzugt mit 1-40 Gew.% Tri-O-Acylglycerol zu 99-60 Gew.% Limus-Wirkstoff, besonders bevorzugt mit 10-30 Gew.% Tri-O-Acylglycerol zu 90-70 Gew.% Limus-Wirkstoff und am meisten bevorzugt mit 20 Gew.% Tri-O-Acylglycerol und 80Gew,% Limus-Wirkstoff vor.

**In** bevorzugten Ausführungsformen beträgt der Massenanteil (m/m; [g]/[g]; m=Masse) von Antioxidantien an der Gesamtmasse von Antioxidantien und mikrokristallinem Limus-Wirkstoff in der Suspension vorzugsweise 0,001 - 15,0%, weiterhin bevorzugt 0,01 - 10,0%, besonders bevorzugt 0,05 - 5,0%.

**In** bevorzugten Ausführungsformen beträgt der Massenanteil (m/m; [g]/[g]; m=Masse) von Zusatzstoffen, die nicht Antioxidantien sind, an der Gesamtmasse von Zusatzstoffen und mikrokristallinem Limus-Wirkstoff in der Suspension vorzugsweise 0,001 - 1,0%, weiterhin bevorzugt 0,01 - 2,5%, weiter bevorzugt besonders bevorzugt 0,05 - 5,0%.

**In** bevorzugten Ausführungsformen beträgt der Massenanteil (m/m; [g]/[g]; m=Masse) von Antioxidantien zusammen mit weiteren Zusatzstoffen, die nicht Antioxidantien sind, an der Gesamtmasse von Antioxidantien und den Zusatzstoffen und mikrokristallinem Limus-Wirkstoff in der Suspension vorzugsweise 0,001 - 15,0%, weiterhin bevorzugt 0,01 - 10,0%, besonders bevorzugt 0,05 - 5,0%.

In bevorzugten Ausführungsformen beträgt der Massenanteil (m/m; [g]/[g]; m=Masse) von Antioxidantien zusammen mit weiteren Zusatzstoffen, die nicht Antioxidantien sind, an der Gesamtmasse von Antioxidantien und den Zusatzstoffen und mikrokristallinem Limus-Wirkstoff in der Suspension vorzugsweise 0,001 - 15,0%, weiterhin bevorzugt 0,01 - 10,0%, besonders bevorzugt 0,05 - 5,0%, wobei der Massenanteil (m/m; [g]/[g]; m=Masse) von Zusatzstoffen, die keine Antioxidantien sind, an der Gesamtmasse von Zusatzstoffen und mikrokristallinem Limus-Wirkstoff in der Suspension vorzugsweise 0,001 - 1,0%, weiterhin bevorzugt 0,01 - 2,5%, weiter bevorzugt besonders bevorzugt 0,05 - 5,0%.

Der Begriff **"Massenverhältnis",** wie hierin verwendet, bezieht sich auf eine physikalisch-chemische Größe zur quantitativen Beschreibung der Zusammensetzung von Stoffgemischen. Das Massenverhältnis gibt das Verhältnis der Massen zweier betrachteter Mischungskomponenten zueinander an.

Das Massenverhältnis (m/m; [g]/[g]; m=Masse) von Tri-O-Acylglycerol zu mikrokristallinem Limus-Wirkstoff liegt vorzugsweise bei 1:1000 - 1:1, weiter bevorzugt 1:100 - 1:1,5, weiter bevorzugt 1:10 - 1:3 und bevorzugt bei 1:5 - 1:4 und am meisten bevorzugt bei 1:4. Die Masse von Tri-O-Acylglycerol bezieht sich hierbei auf die Gesamtmasse von Tri-O-Acylglycerol ausgewählt aus der Gruppe bestehend aus Trioctanoylglycerol, Trinonanoylglycerol, Tridecanoylglycerol und Triundecanoylglycerol, d.h. bei Gemischen von Tri-O-Acylglycerolen ausgewählt aus der Gruppe bestehend aus Trioctanoylglycerol, Trinonanoylglycerol, Tridecanoylglycerol und Triundecanoylglycerol wird die Masse des Tri-O-Acylglycerol-Gemisches zur Berechnung des Massenverhältnisses herangezogen.

Zur Berechnung des Massenverhältnisses von Tri-O-Acylglycerol zu mikrokristallinem Limus-Wirkstoff wird somit der Quotient aus der Masse von Tri-O-Acylglycerol und der Masse von Limus-Wirkstoff gebildet. Beispielsweise beträgt das Massenverhältnis von Tri-O-Acylglycerol zu mikrokristallinem Limus-Wirkstoff in einer Mischung aus 1 g Tri-O-Acylglycerol und 4 g Limus-Wirkstoff 1:4.

Das Massenverhältnis von Tri-O-Acylglycerol zu mikrokristallinem Limus-Wirkstoff liegt weiterhin vorzugsweise bei 0,1-50%, weiter bevorzugt 1-40%, weiter bevorzugt 10-30% und weiter bevorzugt bei 20-25% und am meisten bevorzugt bei 25%. In einigen bevorzugten Ausführungsformen beträgt das Massenverhältnis von Tri-O-Acylglycerol zu mikrokristallinem Limus-Wirkstoff vorzugsweise 5-40 %, weiter bevorzugt 10-30%, und weiter bevorzugt 20-25%.

Zur Berechnung des Massenverhältnisses von Tri-O-Acylglycerol zu mikrokristallinem Limus-Wirkstoff wird somit der Quotient aus der Masse von Tri-O-Acylglycerol und der Masse von Limus-Wirkstoff gebildet. Beispielsweise beträgt das Massenverhältnis von Tri-O-Acylglycerol zu mikrokristallinem Limus-Wirkstoff in einer Mischung aus 1 g Tri-O-Acylglycerol und 4 g Limus-Wirkstoff 25%.

In bevorzugten Ausführungsformen beträgt das Massenverhältnis (m/V; [g]/[100mL]; m=Masse; V=Volumen) von mikrokristallinem Limus-Wirkstoff zu 100 mL Suspensionsvolumen 0,5-6%, weiter bevorzugt 1-5%, noch weiter bevorzugt 2-4%, und am meisten bevorzugt 3%.

Zur Berechnung des Massenverhältnisses von mikrokristallinem Limus-Wirkstoff zu 100 mL Suspensionsvolumen wird somit der Quotient aus der Masse von mikrokristallinem Limus-Wirkstoff und 100mL Suspensionsvolumen gebildet. Beispielsweise beträgt das Massenverhältnis von mikrokristallinem Limus-Wirkstoff zu 100mL Suspensionsvolumen in einer Suspension aus 3 g Limus-Wirkstoff und 100mL Suspensionsvolumen 3%.

Bezogen auf ein Volumen von 1 L Suspensionsvolumen beträgt das Massenverhältnis (m/V; [g]/[L]; m=Masse; V=Volumen) von mikrokristallinem Limus-Wirkstoff zu 1L Suspensionsvolumen 0,5-6%, weiter bevorzugt 1-5%, noch weiter bevorzugt 2-4%, und am meisten bevorzugt 3%.

Zur Berechnung des Massenverhältnisses von mikrokristallinem Limus-Wirkstoff zu 1 L Suspensionsvolumen wird somit der Quotient aus der Masse von mikrokristallinem Limus-Wirkstoff zu 1 L Suspensionsvolumen gebildet. Beispielsweise beträgt das Massenverhältnis von mikrokristallinem Limus-Wirkstoff zu 1 L Suspensionsvolumen in einer Suspension aus 30g Limus-Wirkstoff und 1 L Suspensionsvolumen 3%.

In bevorzugten Ausführungsformen beträgt das Massenverhältnis (m/V; [g]/[100mL]; m=Masse; V=Volumen) von Tri-O-Acylglycerol zu 100 mL Suspensionsvolumen 0,13-1,5%, weiter bevorzugt 0,25-1,25%, noch weiter bevorzugt 0,5-1% und am meisten bevorzugt 0,75%.

Zur Berechnung des Massenverhältnisses von Tri-O-Acylglycerol zu 100 mL Suspensionsvolumen wird somit der Quotient aus der Masse von Tri-O-Acylglycerol und 100mL Suspensionsvolumen gebildet. Beispielsweise beträgt das Massenverhältnis von Tri-O-Acylglycerol zu 100 mL Suspensionsvolumen in einer Suspension aus 0,75 g Tri-O-Acylglycerol und 100 mL Suspensionsvolumen 0,75%.

Anders formuliert beträgt das Massenverhältnis (m/V; [g]/[L]; m=Masse; V=Volumen) von Tri-O-Acylglycerol zu 1 L Suspensionsvolumen 0,13-1,5%, weiter bevorzugt 0,25-1,25%, noch weiter bevorzugt 0,5-1% und am meisten bevorzugt 0,75%. Zur Berechnung des Massenverhältnisses von Tri-O-Acylglycerol zu 1 L Suspensionsvolumen wird somit der Quotient aus der Masse von Tri-O-Acylglycerol und 1 L Suspensionsvolumen gebildet. Beispielsweise beträgt das Massenverhältnis von Tri-O-Acylglycerol zu 1 L Suspensionsvolumen in einer Suspension aus 7,5 g Limus-Wirkstoff und 1 L Suspensionsvolumen 0,75%.

Der Begriff **"Massenkonzentration",** wie hierin verwendet, bezieht sich auf eine physikalisch-chemische Größe zur quantitativen Beschreibung der Zusammensetzung von Stoffgemischen/Mischphasen. Hierbei wird die Masse einer betrachteten Mischungskomponente auf das Gesamtvolumen der Mischphase bezogen.

In bevorzugten Ausführungsformen beträgt die Massenkonzentration (m/V; [mg]/[mL]; m=Masse; V=Volumen) von mikrokristallinem Limus-Wirkstoff in der Suspension vorzugsweise 5 - 60 mg/mL, weiter bevorzugt 20 - 40 mg/mL, weiter bevorzugt 20 - 30 mg/mL. In einigen bevorzugten Ausführungsformen beträgt die Massenkonzentration von mikrokristallinem Limus-Wirkstoff in der Suspension 20-25 mg/mL. In einigen bevorzugten Ausführungsformen beträgt die Massenkonzentration von mikrokristallinem Limus-Wirkstoff in der Suspension 25-30 mg/mL. In einigen bevorzugten Ausführungsformen beträgt die Massenkonzentration von mikrokristallinem Limus-Wirkstoff in der Suspension 20 mg/mL. In einigen bevorzugten Ausführungsformen beträgt die Massenkonzentration von mikrokristallinem Limus-Wirkstoff in der Suspension 25 mg/mL. In einigen bevorzugten Ausführungsformen beträgt die Massenkonzentration von mikrokristallinem Limus-Wirkstoff in der Suspension 30 mg/mL.

Mit anderen Worten beträgt die Massenkonzentration (m/V; [kg]/[m³]; m=Masse; V=Volumen) von mikrokristallinem Limus-Wirkstoff in der Suspension vorzugsweise 5 - 60 kg/m³, weiter bevorzugt 20 - 40 kg/m³, weiter bevorzugt 20 - 30 kg/m³. In einigen bevorzugten Ausführungsformen beträgt die Massenkonzentration von mikrokristallinem Limus-Wirkstoff in der Suspension 20-25 kg/m³. In einigen bevorzugten Ausführungsformen beträgt die Massenkonzentration von mikrokristallinem Limus-Wirkstoff in der Suspension 25-30 kg/m³. In einigen bevorzugten Ausführungsformen beträgt die Massenkonzentration von mikrokristallinem Limus-Wirkstoff in der Suspension 20 kg/m³. In einigen bevorzugten Ausführungsformen beträgt die Massenkonzentration von mikrokristallinem Limus-Wirkstoff in der Suspension 25 kg/m³. In einigen bevorzugten Ausführungsformen beträgt die Massenkonzentration von mikrokristallinem Limus-Wirkstoff in der Suspension 30 kg/m³.

In bevorzugten Ausführungsformen beträgt die Massenkonzentration (m/V; [mg]/[mL]; m=Masse; V=Volumen) von Tri-O-Acylglycerol in der Suspension vorzugsweise 1,3 - 15 mg/mL, weiter bevorzugt 2,5-12,5 mg/mL, weiter bevorzugt 5-10 mg/mL.

In einigen bevorzugten Ausführungsformen beträgt die Massenkonzentration von Tri-O-Acylglycerol in der Suspension 6 - 9 mg/mL. In einigen bevorzugten Ausführungsformen beträgt die Massenkonzentration von Tri-O-Acylglycerol in der Suspension 5,5-9,5 mg/mL. In einigen bevorzugten Ausführungsformen beträgt die Massenkonzentration von Tri-O-Acylglycerol in der Suspension 7,5 mg/mL. In einigen bevorzugten Ausführungsformen beträgt die Massenkonzentration von Tri-O-Acylglycerol in der Suspension 7 mg/mL. In einigen bevorzugten Ausführungsformen beträgt die Massenkonzentration von Tri-O-Acylglycerol in der Suspension 6,5 mg/mL.

In einigen bevorzugten Ausführungsformen beträgt die Massenkonzentration von Tri-O-Acylglycerol in der Suspension 4 - 6 mg/mL. In einigen bevorzugten Ausführungsformen beträgt die Massenkonzentration von Tri-O-Acylglycerol in der Suspension 4,5 - 5,5 mg/mL. In einigen bevorzugten Ausführungsformen beträgt die Massenkonzentration von Tri-O-Acylglycerol in der Suspension 4,5 mg/mL. In einigen bevorzugten Ausführungsformen beträgt die Massenkonzentration von Tri-O-Acylglycerol in der Suspension 5 mg/mL. In einigen bevorzugten Ausführungsformen beträgt die Massenkonzentration von Tri-O-Acylglycerol in der Suspension 5,5 mg/mL.

In bevorzugten Ausführungsformen beträgt die Massenkonzentration (m/V; [mg]/[mL]; m=Masse; V=Volumen) von mikrokristallinem Limus-Wirkstoff in der Suspension 5 - 60 mg/mL und von Tri-O-Acylglycerol 1,3 - 15 mg/mL, weiter bevorzugt ist eine Massenkonzentration von mikrokristallinem Limus-Wirkstoff in der Suspension von 20 - 40 mg/mL und von Tri-O-Acylglycerol 2,5 - 12,5 mg/mL, weiter bevorzugt ist eine Massenkonzentration von mikrokristallinem Limus-Wirkstoff in der Suspension von 20 - 30 mg/mL und von Tri-O-Acylglycerol 5 - 10 mg/mL.

Der Begriff **"Stoffmengenkonzentration",** wie hierin verwendet, bezieht sich auf eine physikalisch-chemische Größe zur quantitativen Beschreibung der Zusammensetzung von Stoffgemischen/Mischphasen. Hierbei wird die Stoffmenge einer betrachteten Mischungskomponente auf das Gesamtvolumen der Mischphase bezogen.

In bevorzugten Ausführungsformen beträgt die Stoffmengenkonzentration (n/V; [mmol]/[L]; n=Stoffmenge; V=Volumen) von mikrokristallinem Limus-Wirkstoff in der Suspension vorzugsweise 5 - 60 mmol/L, weiter bevorzugt 20 - 40 mmol/L, weiter bevorzugt 25 - 35 mmol/L.

Mit anderen Worten beträgt die Stoffmengenkonzentration (n/V; [mmol]/[L]; n=Stoffmenge; V=Volumen) von mikrokristallinem Limus-Wirkstoff in der Suspension vorzugsweise 5 - 60 mol/m³, weiter bevorzugt 20 - 40 mol/m³, weiter bevorzugt 25 - 35 mol/m³.

In einigen bevorzugten Ausführungsformen beträgt die die Stoffmengenkonzentration (n/V; [mmol]/[L]; n=Stoffmenge; V=Volumen) von Tri-O-Acylglycerol in der Suspension 2 - 35 mmol/L, weiter bevorzugt 4 - 25 mmol/L, weiter bevorzugt 10 - 20 mmol/L, weiter bevorzugt 12 - 18 mmol/L.

In einigen bevorzugten Ausführungsformen beträgt die die Stoffmengenkonzentration (n/V; [mmol]/[L]; n=Stoffmenge; V=Volumen) von Tri-O-Acylglycerol in der Suspension 2 - 35 mol/m³, weiter bevorzugt 4 - 25 mol/m³, weiter bevorzugt 10 - 20 mol/m³, weiter bevorzugt 12 - 18 mol/m³.

Der Begriff **"Limus-Wirkstoff"** bezeichnet die Gruppe der Macrolidlactone umfassend die Wirkstoffe Rapamycin (Sirolimus) und Rapamycin-Derivate wie Everolimus, Umirolimus (Biolimus^{®}), Deforolimus, Myolimus, Novolimus, Pimecrolimus, Ridaforolimus, Tacrolimus, Temsirolimus und Zotarolimus.

Als Limus-Wirkstoff können erfindungsgemäß folgende Substanzen verwendet werden: Rapamycin, Deforolimus, Myolimus, Novolimus, 28-O-Methylrapamycin, C-22-Methylrapamycin, C-49-Methylrapamycin, 42-O-(2-ethoxyethyl)-rapamycin (Umirolimus, Biolimus^{®} oder Biolimus A9^{®}), 40-O-(2-Hydroxyethyl)rapamycin (Everolimus), 40-O-Benzylrapamycin, 40-O-(4'-Hydroxymethyl)benzylrapamycin, 40-O-[4'-(1,2-Dihydroxyethyl)]-benzylrapamycin, 40-O-Allylrapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycarbonylmethylrapamycin, 40-O-(3-Hydroxy)propylrapamycin 40-O-(6-Hydroxy)hexylrapamycin 40-O-[2-(2-Hydroxy)ethoxy]ethylrapamycin 40-O-[(3S)-2,2-Dimethyldioxolan-3-yl]methylrapamycin, 40-O-[(2S)-2,3-Dihydroxyprop-1-yl]-rapamycin, 40-O-(2-Acetoxy)ethylrapamycin 40-O-(2-Nicotinoyloxy)ethylrapamycin, 40-O-[2-(N-Morpholino)acetoxy]ethylrapamycin 40-O-(2-N-Imidazolylacetoxy)ethyl-rapamycin, 40-O-[2-(N-Methyl-N'-piperazinyl)acetoxy]ethylrapamycin, 39-O-Desmethyl-39,40-O, O-ethylenrapamycin, (26R)-26-Dihydro-40-O-(2-hydroxy)ethylrapamycin, 40-O-(2-Aminoethyl)-rapamycin, 40-O-(2-Acetaminoethyl)rapamycin 40-O-(2-Nicotinamidoethyl)rapamycin, 40-O-(2-(N-Methyl-imidazo-2'-ylcarbethoxamido)ethyl)rapamycin, 40-O-(2-Ethoxycarbonylaminoethyl)rapamycin, 40-O-(2-Tolylsulfonamidoethyl)rapamycin, 40-O-[2-(4',5'-Dicarboethoxy-1',2',3'-triazol-1'-yl)-ethyl]-rapamycin, 42-Epi-(tetrazolyl)rapamycin (Tacrolimus), 42-[3-Hydroxy-2-(hydroxymethyl)-2-methylpropanoat]rapamycin (Temsirolimus), (42S)-42-Deoxy-42-(1H-tetrazol-1-yl)rapamycin (Zotarolimus), (3S,4R,5S,8R,9E,12S, 14S,15R,16S,18R,19R,26aS)-3-{(E)-2-[(1R,3R,4S)-4-Chlor-3-methoxycyclohexyl]-1-methylvinyl}-8-ethyl-5,6,8,11,12,13,14,15,16,17,18,19,24,25,26,26a-hexadecahydro-5,19.dihhydroxy-14,16-dimethoxy-4,10,12,18-tetramethyl-15,19-epoxy-3H-pyrido[2,1-c][1,4]oxazacyclotricosin-1,7,20,21(4H,23H)-tetron (Pimecrolimus), (1R,2R,4S)-4-[(2R)-2-[(1R,9S,12S,15R,16E,18R,19R,21R,23S,24E,26E,28Z,30S,32S,35R)-1,18-dihydroxy-19,30-dimethoxy-15,17,21,23,29,35-hexamethyl-2,3,10,14,20-pentaoxo-11,36-dioxa-4-azatricyclo[30.3.1.04,9]hexatriaconta-16,24,26,28-tetraen-12-yl]propyl]-2-methoxycyclohexyl-dimethylphosphinat (Ridaforolimus).

Bevorzugt ist die Verwendung von Limus-Wirkstoffen, die in Form von Mikrokristallen vorliegen können, wie z.B. die Limus-Wirkstoffe Rapamycin (Sirolimus), Everolimus, Zotarolimus, Umirolimus, Deforolimus, Myolimus, Novolimus, Pimecrolimus Ridaforolimus, Tacrolimus und Temsirolimus. Besonders bevorzugt sind Everolimus und Rapamycin.

Der Limus-Wirkstoff wird bevorzugt ausgewählt aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, weiter bevorzugt sind Rapamycin (Sirolimus) und Everolimus. In einer noch bevorzugteren Ausführungsform ist der Limus-Wirkstoff Rapamycin (Sirolimus). In einer besonders bevorzugten Ausführungsform ist der Limus-Wirkstoff Everolimus.

Rapamycin ist auch unter Rapamun oder dem International Nonproprietary Name (INN) Sirolimus sowie dem IUPAC-Namen [3S-[3R*[E(1S*,3S*,4S*)],4S*,5R*,8S*, 9E,12R*,14R*,15S*,16R*,18S*,19S*,26aR*]]-5,6,8,11,12,13,14,15,16,17,18,19,24, 25,26,26a-hexadeca-hydro-5,19-dihydroxy-3-[2-(4-hydroxy-3-methoxy-cyclohexyl)-1-methylethenyl]-14,16-dimethoxy-4,10,12,18-tetramethyl-8-(2-propenyl)-15,19-epoxy-3H-pyrido[2,1-c][1,4]-oxaazacyclo-tricosin-1,7,20,21(4H,23H)-tetron-monohydrat bekannt.

Rapamycin weist die folgende Strukturformel auf:

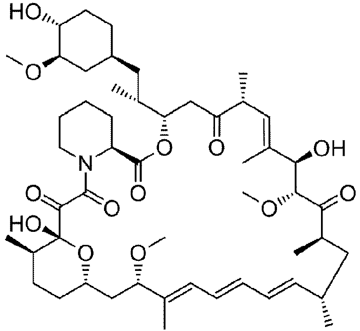

Everolimus ist ein Derivat von Rapamycin mit dem IUPAC-Namen Dihydroxy-12-[(2*R*)-1-[(1*S*,3*R*,4*R*)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.04,9]hexatriaconta-16,24,26,28-tetraen-2,3,10,14,20-penton.

Everolimus weist die folgende Strukturformel auf:

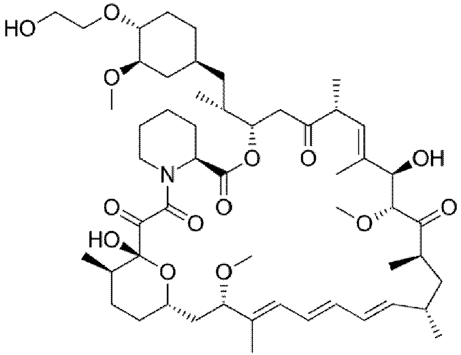

Der Begriff **"Mikrokristalle",** wie hierin verwendet, bezieht sich auf Festkörper, dessen Bausteine regelmäßig in einer Kristallstruktur angeordnet sind und eine Größe im Mikrometerbereich aufweisen. Der Begriff "Mikrometerbereich", wie hierin verwendet, entspricht dem Bereich von 1 µm bis 300 µm, dabei entspricht 1 µm bekanntermaßen 10⁻⁶ m, 10⁻³ mm oder 1000 nm. Der Begriff Mikrokristalle, wie hierin verwendet, bezeichnet somit Kristalle mit einer Kristallgröße im Bereich von 1 µm bis 300 µm.

Der Begriff **"Kristallgröße",** wie hierin verwendet, bezeichnet die Länge der Kristalle entlang ihrer größten Ausdehnung, also bei stäbchenförmigen oder nadelförmigen Kristallen entlang ihrer Längsachse. Die Mikrokristalle, wie hierin definiert, weisen somit eine Länge im Bereich 1 µm bis 300 µm entlang ihrer größten Ausdehnung auf.

Der Begriff **"Kristallinität",** wie hierin verwendet, ist der kristalline Anteil einer Verbindung, also der Anteil an Kristallen einer Verbindung an der Gesamtmenge dieser Verbindung in kristalliner Form und in anderen Formen.

Der Begriff **"mikrokristalliner Limus-Wirkstoff",** wie hierin verwendet, bezeichnet einen Limus-Wirkstoff, der in Form von Mikrokristallen vorliegt. Somit wird der Ausdruck "mikrokristalliner Limus-Wirkstoff" und "Limus-Wirkstoff in Form von Mikrokristallen" hierin synonym verwendet.

Aus dem Stand der Technik sind Kristallisationsverfahren zur Herstellung von Limus-Wirkstoffen bekannt. Im Allgemeinen kann zur Kristallisation von Limus-Wirkstoffen eine Lösung eines Limus-Wirkstoffs hergestellt und die Löslichkeit des Limus-Wirkstoffs in der Lösung verringert werden. Zu den gängigen Methoden zur Verringerung der Löslichkeit gehören beispielsweise Kühlung, Zugabe eines Antilösungsmittels und Verdampfung.

Kristallisation durch Kühlung: Der Limus-Wirkstoff kann bei Raumtemperatur oder höherer Temperatur bis zur Sättigung in einem Lösungsmittel gelöst und bei niedrigerer Temperatur z.B. bei 0°C zur Kristallisation gebracht. Die Kristallgrößenverteilung kann über eine kontrollierte Abkühlungsrate beeinflusst werden. Als Lösungsmittel zur Kristallisation eignen sich für Limus-Wirkstoffe sowohl polare als auch unpolare organische Lösungsmittel, wie z.B. Toluol, Acetonitril, Ethylformiat, Isopropylacetat, Isobutylacetat, Ethanol, Dimethylformamid, Anisol, Ethylacetat, Methylethylketon, Methylisopropylketon, Tetrahydrofuran, Nitromethan, Propionitril.

Kristallisation durch Zugabe von Impfkristallen: Der Limus-Wirkstoff wird bis zur Sättigung in einem Lösungsmittel gelöst und die Kristallisation durch Zugabe von Impfkristallen initiiert, um einen kontrollierten Abbau der Übersättigung zu erzielen.

Kristallisation durch Zugabe von Antilösungsmitteln: Der Wirkstoff wird in einem Lösungsmittel gelöst und anschließend ein Nichtlöser oder Wasser hinzugegeben. Hierbei sind auch Zwei-Phasen-Gemische möglich. Als Lösungsmittel zum Lösen des Limus-Wirkstoffs können polare organische Lösungsmittel wie beispielsweise Aceton, Acetonitril, Ethylacetat, Methanol, Ethanol, Isopropanol, Butanol, Butylmethylether, Tetrahydrofuran, Dimethylformamid oder Dimethylsulfoxid eingesetzt werden. Als Nichtlöser eignen sich beispielsweise Pentan, Hexan, Cyclohexan oder Heptan. Das Lösungsmittelgemisch kann zur Kristallisation stehengelassen, gerührt oder langsam i. Vak. eingeengt bzw. verdampft werden. Die Kristallgröße und Kristallinität des Wirkstoffs kann durch kontrollierte Zugabe des unpolaren Lösungsmittels beeinflusst werden. Für die Erzeugung großer Kristalle sollte die Übersättigung langsamer und zur Erzeugung kleiner Kristalle schneller erfolgen. Die Steuerung der Zugaberate des Antilösungsmittels zur Kontrolle der Kristallgröße ist allgemein bekannt.

Zur Herstellung von Mikrokristallen kann die Kristallisation auch durch Ultraschall unterstützt werden. Es ist allgemein bekannt, dass die Kristallgröße mittels Ultraschall beeinflusst werden kann. Ultraschall kann dabei zu Beginn der Kristallisation zur Initiierung der Kristallisation und Keimbildung eingesetzt werden, wobei das weitere Kristallwachstum dann ungehindert verläuft, so dass größere Kristalle heranwachsen können. Die Anwendung einer kontinuierlichen Beschallung einer übersättigten Lösung mit Ultraschall führt hingegen zu kleineren Kristallen, da hierbei viele Keime entstehen, wodurch zahlreiche kleine Kristalle heranwachsen. Eine weitere Option besteht in der Beschallung mit Ultraschall im Pulsmodus, um das Kristallwachstum so zu beeinflussen, dass maßgeschneiderte Kristallgrößen erzielt werden.

Hierin bevorzugte Kristallisationsverfahren zur Herstellung von mikrokristallinen Limus-Wirkstoffen sind kontrolliert ablaufende Kristallisationen, um so Mikrokristalle in nativem und intaktem Zustand zu erhalten und um mögliche Schädigungen, z.B. durch ein Vermahlen oder Mikronisierung zu vermeiden.

Zur Bereitstellung der gewünschten Kristallgrößen können auch noch weitere aus dem Stand der Technik bekannte Verfahren wie Mikronisierung, Vermahlung oder Siebung eingesetzt werden. Eine Möglichkeit ist die Vermahlung der Kristalle, die auch während der Kristallisation durch ein Nassvermahlen erfolgen kann. Das Vermahlen kann vorteilhaft sein, um verschiedene Kristallgrößen, d.h. eine breitere Kristallgrößenverteilung erhalten. Das Vermahlen lässt alle Wunschgrößen im Kristallgrößenbereich zu. Einheitlichere Kristallgrößen können bereitgestellt werden, indem nach der Isolierung und Trocknung beispielsweise ein spezielles Siebverfahren durchgeführt wird. Hierzu kann auf aus dem Stand der Technik bekannte spezielle Siebvorrichtungen zurückgegriffen werden. Im Siebverfahren können die Limus-Wirkstoffkristalle z.B. durch einen Stapel von Sieben gesiebt und in verschiedene Größenbereiche aufgeteilt werden.

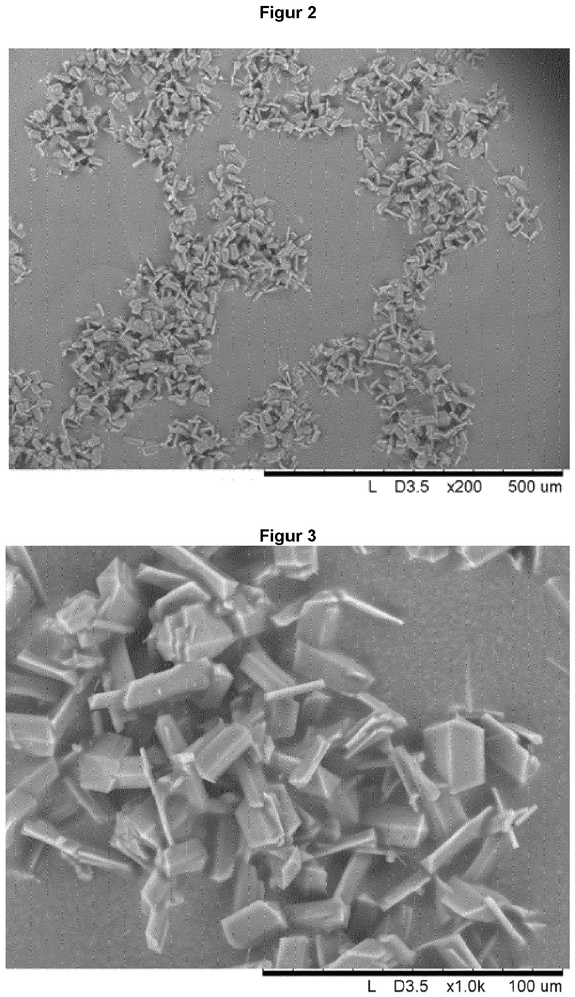

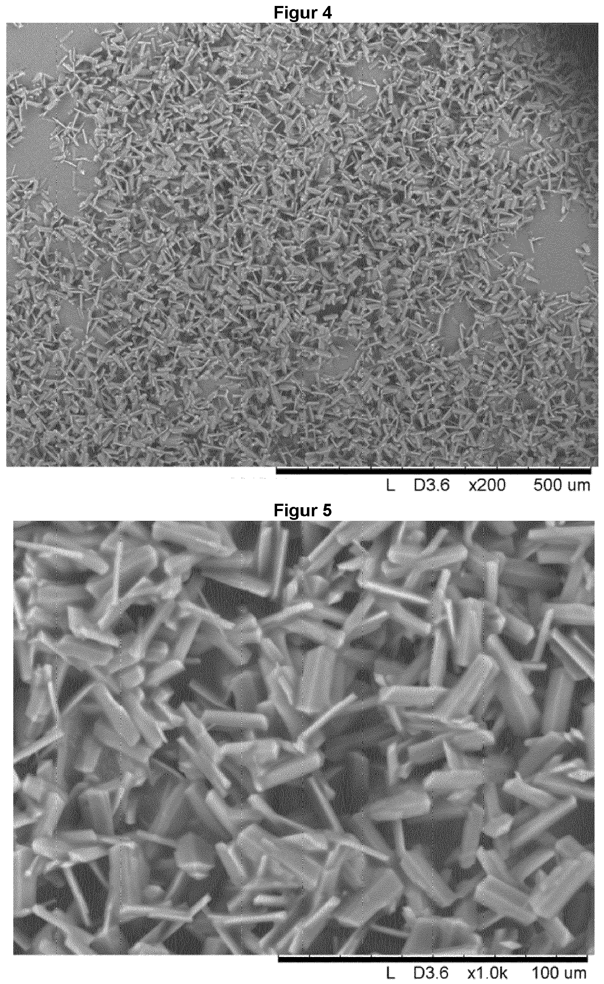

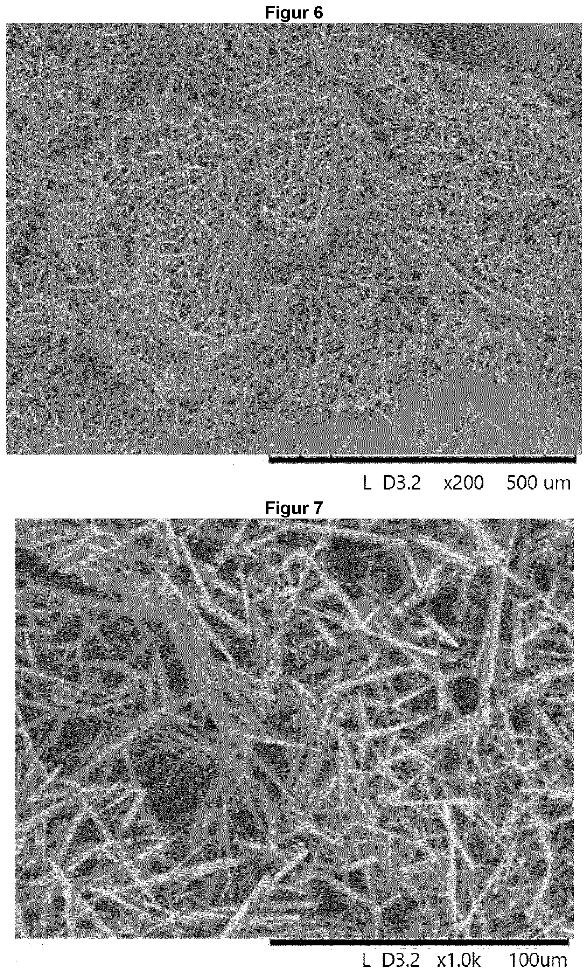

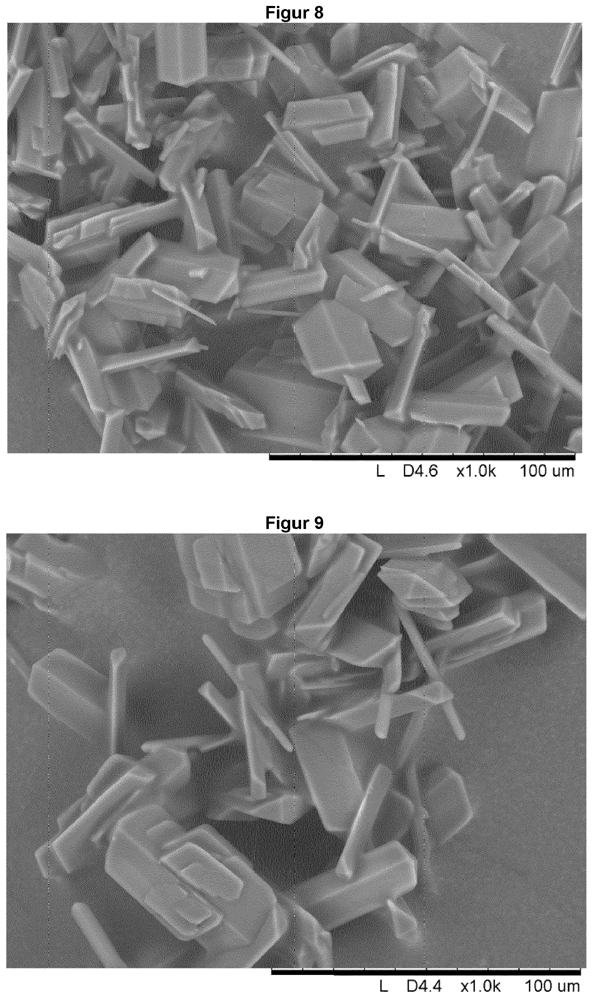

Beispielaufnahmen der Limus-Wirkstoffe Rapamycin und Everolimus in Form von Mikrokristallen sind in Fig. 2 bis Fig. 9 gezeigt. Fig. 2 und 3 zeigen Rapamycin in Stäbchenform mit sehr enger Partikelgrößenverteilung im Bereich von 10 µm bis 30 µm. Fig. 4 und 5 zeigen Rapamycin in Form von Mikrokristallen in Stäbchenform mit extrem enger Partikelgrößenverteilung im Bereich von 15 µm bis 30 µm. Fig. 7 und 8 zeigen Rapamycin mit einer Partikelgrößenverteilung im Bereich von 20 µm bis 40 µm. Fig. 6 und 7 zeigen Everolimus in Nadelform mit einer Partikelgrößenverteilung im Bereich von 20 µm bis 40 µm. In Fig. 2 bis 9 ist deutlich zu erkennen, dass keinerlei größere Kristalle oder Agglomerate vorliegen. Es ist zudem deutlich zu erkennen, dass Everolimus in Form von Nadeln vorliegt, während Rapamycin in Form von rhomboedrischen Prismen vorliegt.

Es hat sich gezeigt, dass sich mit mikrokristallinen Limus-Wirkstoffen besonders stabile Suspensionen herstellen lassen, wenn die Suspensionen mindestens ein Tri-O-Acylglycerol ausgewählt aus der Gruppe bestehend aus Trioctanoylglycerol, Trinonanoylglycerol, Tridecanoylglycerol und Triundecanoylglycerol enthalten. Der mindestens eine Limus-Wirkstoff liegt erfindungsgemäß in Form von Mikrokristallen vor. Damit liegt der Limus-Wirkstoff in Form von Mikrokristallen mit einer Kristallgröße im Bereich von 1 µm bis 300 µm vor. Die Mikrokristalle des mindestens einen Limus-Wirkstoffs weisen daher eine Kristallgröße zwischen 1 µm bis 300 µm auf.

Die Mikrokristalle des Limus-Wirkstoffes sind nicht verkapselt und sind nicht beschichtet wie z.B. mit einem Polymer und sind an der Oberfläche nicht modifiziert. Zudem enthalten die Mikrokristalle des Limus-Wirkstoffes kein Polymer, keine Polymerpartikel, kein Metall, keine Metallpartikel, keine Keramik und keine Keramikpartikel. Auch sind keine weiteren pharmazeutischen Wirkstoffe oder Peptide, Proteine, Aminosäure, Fettsäuren, Fettsäureester oder Nucleotide oder andere Biopolymere enthalten.

Die vorliegende Erfindung betrifft daher eine Suspension enthaltend mindestens einen mikrokristallinen Limus-Wirkstoff, wie hierin definiert. **In** der Suspension liegt der mindestens eine Limus-Wirkstoff in Form von Mikrokristallen vor. Der Gehalt an im Lösungsmittel oder Lösungsmittelgemisch in der Suspension gelösten Limus-Wirkstoff liegt unter 10%, bevorzugt unter 5% und weiter bevorzugt unter 2%, am meisten bevorzugt unter 1% bezogen auf die Masse des bei der Herstellung der Suspension eingesetzten Limus-Wirkstoffes in Form von Mikrokristallen. Bevorzugt ist daher, dass sich maximal 10%, bevorzugt maximal 5% und weiter bevorzugt maximal 2%, am meisten bevorzugt maximal 1% der Mikrokristalle des mindestens einen Limus-Wirkstoffes in der Suspension lösen.

Erfindungsgemäß enthält die Suspension zur Beschichtung eines Medizinprodukts, vorzugsweise ausgewählt aus einem Katheterballon, einem Ballonkatheter, einem Stent oder einer Kanüle, ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen.

Der Ausdruck "worin die Mikrokristalle des mindestens einen Limus-Wirkstoffs nicht lösen", wie hierin verwendet, bedeutet, dass sich bevorzugt maximal 10%, bevorzugt maximal 9%, weiter bevorzugt maximal 8%, weiter bevorzugt maximal 7%, weiter bevorzugt maximal 6%, weiter bevorzugt maximal 5%, weiter bevorzugt maximal 4%, noch weiter bevorzugt maximal 3%, weiter bevorzugt maximal 2%, und am meistens bevorzugt maximal 1% der Mikrokristalle des mindestens einen Limus-Wirkstoffs in der Suspension lösen. Bevorzugt ist natürlich, dass sich die Mikrokristalle des mindestens einen Limus-Wirkstoffs zu 100% nicht in der Suspension lösen.

Weiter bevorzugt ist zudem, dass die Löslichkeit der Mikrokristalle des mindestens einen Limus-Wirkstoff in dem Lösungsmittel oder dem Lösungsmittelgemisch der Suspension <20 mg/mL, weiter bevorzugt <15 mg/mL, weiter bevorzugt <10 mg/mL, weiter bevorzugt <9 mg/mL, weiter bevorzugt <8 mg/mL, weiter bevorzugt <7 mg/mL, weiter bevorzugt <6 mg/mL, weiter bevorzugt <5 mg/mL, weiter bevorzugt <4 mg/mL, weiter bevorzugt <3 mg/mL, weiter bevorzugt <2 mg/mL, weiter bevorzugt <1 mg/mL ist.

Überraschenderweise wurde gefunden, dass sich Mikrokristalle von Limus-Wirkstoffen in einer Lösung enthaltend mindestens ein Tri-O-Acylglycerol ausgewählt aus der Gruppe bestehend aus Trioctanoylglycerol, Trinonanoylglycerol, Tridecanoylglycerol und Triundecanoylglycerol nicht auflösen. Somit lässt sich eine Kristallsuspension als Beschichtungsformulierung herstellen, in der die Mikrokristalle des Limus-Wirkstoffes intakt bleiben.

Die Kristalle des Limus-Wirkstoffes sollten mindestens eine Größe von 1 µm aufweisen. Kristalle mit einer Größe von unter 1 µm sind zu klein, so dass sie sich relativ schnell auflösen. Bei der Herstellung der Suspension der vorliegenden Erfindung hat sich gezeigt, dass stabile Suspensionen nur erhalten werden können, wenn der mindestens eine Limus-Wirkstoff im Wesentlichen keine Kristalle mit einer Kristallgröße von unter 1 µm aufweist. Mit anderen Worten liegt der Limus-Wirkstoff besonders bevorzugt nicht in Form von Nanokristallen vor. Der Begriff Nanokristalle, wie hierin verwendet, betrifft Kristalle mit einer Kristallgröße im Bereich 1 nm bis weniger als 1000 nm.

Bevorzugt ist daher, dass mindestens 95% - 97% des mindestens einen Limus-Wirkstoffs, weiter bevorzugt mindestens 95% - 99%, weiter bevorzugt mindestens 97% - 99% und besonders bevorzugt mindestens 98% - 99,9% des mindestens einen Limus-Wirkstoffs in Form von Mikrokristallen mit einer Kristallgröße von mindestens 1 µm vorliegt. In weiter bevorzugten Ausführungsformen liegt 100% des mindestens einen Limus-Wirkstoffs in Form von Mikrokristallen mit einer Kristallgröße von mindestens 1 µm vor.

Es hat sich zudem gezeigt, dass vorteilhaft ist, wenn der Limus-Wirkstoff in Form von Mikrokristallen mindestens eine Kristallgröße von 10 µm aufweist. Daher ist bevorzugt, dass der mindestens eine Limus-Wirkstoff einen geringen Anteil an Mikrokristallen mit einer Kristallgröße von 1 µm - 10 µm aufweist. Besonders bevorzugt ist, dass nur wenige Kristalle, d.h. deutlich weniger als 10% sämtlicher Kristalle kleiner als 10 µm sind. In bevorzugten Ausführungsformen liegen daher weniger als 10% sämtlicher Mikrokristalle des Limus-Wirkstoffs mit einer Kristallgröße im Bereich von kleiner als 10 µm vor.

Bevorzugt ist daher, dass mindestens 90% des mindestens einen Limus-Wirkstoffs, bevorzugt mindestens 90% - 95% des mindestens einen Limus-Wirkstoffs, weiter bevorzugt mindestens 93% - 98% des mindestens einen Limus-Wirkstoffs, weiter bevorzugt mindestens 95% - 99% des mindestens einen Limus-Wirkstoffs und besonders bevorzugt mindestens 98% - 99,9% des mindestens einen Limus-Wirkstoffs in Form von Mikrokristallen mit einer Kristallgröße von mindestens 10 µm vorliegt.

Weiter bevorzugt ist, dass die Mikrokristalle des mindestens einen Limus-Wirkstoffes mindestens eine Kristallgröße von 5 µm aufweisen. Bevorzugt ist daher, dass mindestens 90% des mindestens einen Limus-Wirkstoffs, bevorzugt mindestens 90% - 95% des mindestens einen Limus-Wirkstoffs, weiter bevorzugt mindestens 93% - 98% des mindestens einen Limus-Wirkstoffs, weiter bevorzugt mindestens 95% - 99% des mindestens einen Limus-Wirkstoffs, und besonders bevorzugt mindestens 98% - 99,9% des mindestens einen Limus-Wirkstoffs in Form von Mikrokristallen mit einer Kristallgröße von mindestens 5 µm vorliegt. Mikrokristalle mit einer Kristallgröße im Bereich von kleiner als 5 µm können sich schneller auflösen und sind daher weniger bevorzugt.

Noch weiter bevorzugt ist, dass die Mikrokristalle des mindestens einen Limus-Wirkstoffes mindestens eine Kristallgröße von 20 µm aufweisen. Bevorzugt ist daher, dass mindestens 90% des mindestens einen Limus-Wirkstoffs, bevorzugt mindestens 90% - 95% des mindestens einen Limus-Wirkstoffs, weiter bevorzugt mindestens 93% - 98% des mindestens einen Limus-Wirkstoffs, weiter bevorzugt mindestens 95% - 99% des mindestens einen Limus-Wirkstoffs und besonders bevorzugt mindestens 98% - 99,9% des mindestens einen Limus-Wirkstoffs in Form von Mikrokristallen mit einer Kristallgröße von mindestens 20 µm vorliegt.

Zudem liegen bevorzugt nur wenige Mikrokristalle des Limus-Wirkstoffs, d.h. weniger als 40% und bevorzugter weniger als 30% oder sogar weniger als 25%, mit einer Kristallgröße im Bereich von 50 µm - 300 µm vor. Bevorzugt ist daher, dass maximal 40% des mindestens einen Limus-Wirkstoffes, bevorzugt maximal 30% des mindestens einen Limus-Wirkstoffes, weiter bevorzugt maximal 25% des mindestens einen Limus-Wirkstoffes in Form von Mikrokristallen mit einer Partikelgröße von 50 µm - 300 µm vorliegt. **In** weiteren Ausführungsformen ist bevorzugt, dass maximal 20% des mindestens einen Limus-Wirkstoffes, bevorzugt maximal 15% des mindestens einen Limus-Wirkstoffes, weiter bevorzugt maximal 10% des mindestens einen Limus-Wirkstoffes in Form von Mikrokristallen mit einer Partikelgröße von 50 µm - 300 µm vorliegt. **In** besonders bevorzugten Ausführungsformen liegen die Mikrokristalle des mindestens einen Limus-Wirkstoffs im Wesentlichen mit einer Kristallgröße von maximal 50 µm vor.

Weiter bevorzugt liegen sehr wenige Mikrokristalle des Limus-Wirkstoffs, d.h. weniger als 10% und bevorzugter weniger als 5% oder sogar weniger als 2%, und am meisten bevorzugt weniger als 1% mit einer Kristallgröße im Bereich von 100 µm -300 µm vor. Mikrokristalle mit einer Kristallgröße im Bereich von 100 µm - 300 µm könnten Agglomerate bilden und sich zu größeren Partikeln zusammenschließen, welche die Gefahr von Gefäßverschlüssen mit sich bringen können. Es ist daher besonders bevorzugt, wenn der Anteil an Mikrokristallen mit einer Partikelgröße im Bereich von 100 µm - 300 µm so gering wie möglich ist.

Bevorzugt ist daher, dass maximal 10% des mindestens einen Limus-Wirkstoffes, bevorzugt maximal 5% des mindestens einen Limus-Wirkstoffes, weiter bevorzugt maximal 2% des mindestens einen Limus-Wirkstoffes, noch weiter bevorzugt maximal 1% des mindestens einen Limus-Wirkstoffes in Form von Mikrokristallen mit einer Partikelgröße von 100 µm - 300 µm vorliegt. In weiteren Ausführungsformen ist bevorzugt, dass mindestens 99%, bevorzugt 99,5%, weiter bevorzugt mindestens 99,7%, noch weiter bevorzugt mindestens 99,9% und am meisten bevorzugt 100% des mindestens einen Limus-Wirkstoffes mit einer Partikelgröße von ≤ 100 µm vorliegt. In bevorzugten Ausführungsformen liegen die Mikrokristalle des mindestens einen Limus-Wirkstoffs im Wesentlichen mit einer Kristallgröße von maximal 100 µm vor. In insbesondere bevorzugten Ausführungsformen liegen die Mikrokristalle des mindestens einen Limus-Wirkstoffs im Wesentlichen mit einer Kristallgröße von maximal 100 µm vor.

Bevorzugt ist daher, dass der Limus-Wirkstoff in Form von Mikrokristalle mit einer Kristallgröße von 1 µm bis 100 µm vorliegt.

Es hat sich gezeigt, dass Mikrokristalle des Limus-Wirkstoffs mit einer Kristallgröße im Bereich von 10 µm bis 50 µm gut geeignet sind, um eine erfindungsgemäße Suspension zur Beschichtung von Medizinprodukten bereitzustellen. Bevorzugt ist daher, dass mindestens 70% des mindestens einen Limus-Wirkstoffes, bevorzugt mindestens 70% - 80% des mindestens einen Limus-Wirkstoffes, weiter bevorzugt mindestens 80% - 90% des mindestens einen Limus-Wirkstoffes, weiter bevorzugt mindestens 90% - 95% des mindestens einen Limus-Wirkstoffes und besonders bevorzugt mindestens 95% - 99% des mindestens einen Limus-Wirkstoffes in Form von Mikrokristallen mit einer Kristallgröße von 10 µm bis 50 µm vorliegen.

Es hat sich gezeigt, dass Mikrokristalle des Limus-Wirkstoffs mit einer Partikelgröße im Bereich von 5 µm bis 35 µm geeignet sind, um eine stabile Suspension zur Beschichtung von Medizinprodukten bereitzustellen. Es daher bevorzugt, wenn mindestens 70% der Mikrokristalle des Limus-Wirkstoffs mit einer Kristallgröße im Bereich von 5 µm bis 35 µm vorliegen. Bevorzugt ist daher, dass mindestens 70% des Limus-Wirkstoffs, bevorzugt mindestens 70% - 80% des Limus-Wirkstoffs, weiter bevorzugt mindestens 80% - 90% des Limus-Wirkstoffs in Form von Mikrokristallen mit einer Partikelgröße von 5 µm bis 35 µm vorliegt. Weiter ist bevorzugt, dass mindestens 70% des mindestens einen Limus-Wirkstoffes, bevorzugt mindestens 70% - 80% des mindestens einen Limus-Wirkstoffes, weiter bevorzugt mindestens 80% - 90% des mindestens einen Limus-Wirkstoffes, weiter bevorzugt mindestens 90% - 95% des mindestens einen Limus-Wirkstoffes und besonders bevorzugt mindestens 95% - 99% des mindestens einen Limus-Wirkstoffes in Form von Mikrokristallen mit einer Partikelgröße von 5 µm bis 35 µm vorliegen.

In besonders bevorzugten Ausführungsformen liegen die Mikrokristalle des Limus-Wirkstoffs mit einer Kristallgröße im Bereich von 20 µm bis 40 µm vor. Bevorzugt ist daher, dass mindestens 70% des mindestens einen Limus-Wirkstoffes, bevorzugt mindestens 70% - 80% des mindestens einen Limus-Wirkstoffes, weiter bevorzugt mindestens 80% - 90% des mindestens einen Limus-Wirkstoffes, weiter bevorzugt mindestens 90% - 95% des mindestens einen Limus-Wirkstoffes und besonders bevorzugt mindestens 95% - 99% des mindestens einen Limus-Wirkstoffes in Form von Mikrokristallen mit einer Kristallgröße von 20 µm bis 40 µm vorliegen.

Vorzugsweise weist der Limus-Wirkstoff eine Kristallinität von mindestens 90 Gew.-%, mehr bevorzugt mindestens 92,5 Gew.-%, mehr bevorzugt mindestens 95 Gew.-%, mehr bevorzugt mindestens 97,5 Gew.-% und am meisten bevorzugt mindestens 99 Gew.-% auf.

Bei den Mikrokristallen des mindestens einen Limus-Wirkstoffs handelt es sich bevorzugt um Mikrokristalle von mindestens einem Limus-Wirkstoff ausgewählt aus der Gruppe umfassend oder bestehend aus aus Rapamycin, Everolimus, Zotarolimus, Umirolimus, Deforolimus, Myolimus, Novolimus, Pimecrolimus Ridaforolimus, Tacrolimus und Temsirolimus.

Die Mikrokristalle des mindestens einen Limus-Wirkstoffs sind bevorzugt Mikrokristalle von Rapamycin oder Mikrokristalle von Everolimus. Hierin bevorzugt sind mikrokristallines Rapamycin und mikrokristallines Everolimus. Besonders bevorzugt ist hierin mikrokristallines Everolimus.

Kristalle mit prismatischen bis nadeligen Habitus sind eindimensional gestreckte Formen, bei denen die Länge des Kristalls deutlich größer als dessen Durchmesser ist.

In bevorzugten Ausführungsformen ist der mindestens eine Limus-Wirkstoff Rapamycin. Rapamycin kristallisiert in Form von rhomboedrischen Prismen. Es ist daher bevorzugt, dass mindestens 90%, mehr bevorzugt mindestens 92,5%, mehr bevorzugt mindestens 95%, mehr bevorzugt mindestens 97,5% und am meisten bevorzugt mindestens 99% der Mikrokristalle von Rapamycin in Form von rhomboedrischen Prismen vorliegen. Es ist daher bevorzugt, dass mindestens 90%, mehr bevorzugt mindestens 92,5%, mehr bevorzugt mindestens 95%, mehr bevorzugt mindestens 97,5% und am meisten bevorzugt mindestens 99% der Mikrokristalle von Rapamycin prismatisch sind. Es ist daher bevorzugt, dass Rapamycin zu mindestens 90 %, mehr bevorzugt mindestens 92,5%, mehr bevorzugt mindestens 95%, mehr bevorzugt mindestens 97,5% und am meisten bevorzugt mindestens 99% in Form prismatischer Mikrokristalle vorliegt.

In bevorzugten Ausführungsformen ist der mindestens eine Limus-Wirkstoff Everolimus. Everolimus kristallisiert in Form von Nadeln. Es ist daher bevorzugt, dass mindestens 90%, mehr bevorzugt mindestens 92,5%, mehr bevorzugt mindestens 95%, mehr bevorzugt mindestens 97,5% und am meisten bevorzugt mindestens 99% der Mikrokristalle von Everolimus in Form von Nadeln vorliegen. Es ist daher bevorzugt, dass mindestens 90 %, mehr bevorzugt mindestens 92,5%, mehr bevorzugt mindestens 95%, mehr bevorzugt mindestens 97,5% und am meisten bevorzugt mindestens 99% der Mikrokristalle von Everolimus nadelförmig sind. Es ist daher bevorzugt, dass Everolimus zu mindestens 90 %, mehr bevorzugt mindestens 92,5%, mehr bevorzugt mindestens 95%, mehr bevorzugt mindestens 97,5% und am meisten bevorzugt mindestens 99% in Form nadeliger Mikrokristalle vorliegt.

Der Begriff **"Lösungsmittel",** wie hierin verwendet, bezieht sich auf einen Stoff, der bei Normaltemperatur (20°C) und Normaldruck (101 hPa; 1 bar, 1 atm) im flüssigen Aggregatzustand vorliegt und der Gase, Flüssigkeiten oder Feststoffe lösen oder verdünnen kann, ohne dass zwischen gelöstem Stoff und lösendem Stoff chemische Reaktionen stattfinden. Flüssigkeiten wie Wasser und flüssige organische Stoffe werden als Lösungsmittel zum Lösen anderer Stoffe eingesetzt.

Der Begriff **"Nichtlöser",** wie hierin verwendet, bezieht sich auf ein Lösungsmittel, das mikrokristalline Limus-Wirkstoffe nicht an- oder auflösen kann, d.h. ein Lösungsmittel, in dem mikrokristalline Limus-Wirkstoffe praktisch unlöslich sind, aber in dem die Tri-O-Acylglycerole ausgewählt aus der Gruppe bestehend aus Trioctanoylglycerol, Trinonanoylglycerol, Tridecanoylglycerol und Triundecanoylglycerol oder Gemische dieser Tri-O-Acylglycerole löslich sind.

Die Löslichkeit eines mikrokristallinen Limus-Wirkstoffs in einem Nichtlöser sollte höchstens 1 mg/mL betragen. Beispiele von Lösungsmitteln in denen die Löslichkeit von mikrokristallinen Limus-Wirkstoffen höchstens 1 mg/mL beträgt sind Wasser und einige unpolare organische Lösungsmittel wie gesättigte aliphatische Kohlenwasserstoffe.

Beispiele von Lösungsmitteln, in denen die Tri-O-Acylglycerole Trioctanoylglycerol, Trinonanoylglycerol, Tridecanoylglycerol und Triundecanoylglycerol oder Gemische dieser Tri-O-Acylglycerole löslich sind, schließen ein, sind aber nicht beschränkt auf unpolare organische Lösungsmittel wie Hexan, Heptan, Cyclohexan, Toluol, aber auch polare organische Lösungsmittel wie Diethylether, Ethylacetat, Aceton, Isopropanol und Ethanol.

Tri-O-Acylglycerole sind unpolar, d.h. lipophil und sind in sehr polaren Lösungsmitteln wie Wasser oder Glycerol schwer oder praktisch unlöslich. Eine Suspension, die als Lösungsmittel ausschließlich ein sehr polares Lösungsmittel wie Wasser oder Glycerol enthält, ist somit nicht erfindungsgemäß, da die Tri-O-Acylglycerole ausgewählt aus der Gruppe bestehend aus Trioctanoylglycerol, Trinonanoylglycerol, Tridecanoylglycerol oder Triundecanoylglycerol oder ein Gemisch dieser Tri-O-Acylglycerole in sehr polaren Lösungsmitteln wie Wasser oder Glycerol nicht in gelöster Form vorliegen.

Der Begriff "Nichtlöser", wie hierin verwendet, bezieht sich somit auf unpolare organische Lösungsmittel, insbesondere auf gesättigte aliphatische Kohlenwasserstoffe. Ein "Nichtlöser" kann daher auch als "unpolarer organischer Nichtlöser" bezeichnet werden. Unpolare organische Lösungsmittel, die hierin als "Nichtlöser" bezeichnet werden, umfassen daher bei Normaltemperatur (20°C) und Normaldruck (101 hPa; 1 bar, 1 atm) flüssige gesättigte aliphatische Kohlenwasserstoffe, d.h. unverzweigte (lineare) gesättigte Kohlenwasserstoffe mit der allgemeinen Summenformel CₙH₂ₙ₊₂ mit n = 5 bis 16, verzweigte gesättigte Kohlenwasserstoffe mit der allgemeinen Summenformel CₙH₂ₙ₊₂ mit n = 4 bis 16 oder auch cyclische gesättigte Kohlenwasserstoffe mit der allgemeinen Summenformel CₙH₂ₙ mit n = 5 bis 16. Beispiele von Nichtlösern schließen ein, sind aber nicht beschränkt auf unverzweigte C₅₋₁₆-Alkane wie Pentan, Hexan, Heptan, Octan, Nonan, Decan, Petrolether, verzweigte C₅₋₁₆-Alkane (iso-Alkane) wie Isopentan, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, C₅₋₁₆-Cycloalkane wie Cyclopentan, Cyclohexan, Methylcyclopentan, tert-Butylcyclohexan, Methylcyclohexan, Cycloheptan, Cyclooctan, Cyclononan, Cyclodecan, 2,3-Dimethylcyclobutan, 1,2-Dimethylcyclobutan, Decalin, Pinan Hexylcyclohexan, Heptylcyclopentan, 1,4-Dimethylcyclohexan, 1,1-Dimethylcyclohexan, Spiropentan, Spirohexan, Spiroheptan. Natürlich können auch Gemische von Nichtlösern eingesetzt werden.

Für die vorliegende Erfindung geeignete Nichtlöser liegen bei Normaltemperatur (20°C) und Normaldruck (101 hPa; 1 bar, 1 atm) im flüssigen Aggregatzustand vor. Bevorzugte Nichtlöser weisen einen Schmelzpunkt von <20°C, weiter bevorzugt <15°C, noch weiter bevorzugt <10°C auf. Bevorzugte Nichtlöser weisen zudem einen Siedepunkt von <200°C, weiter bevorzugt <150°C, noch weiter bevorzugt von <100°C auf. Bevorzugte Nichtlöser weisen ebenfalls einen Siedepunkt von >25°C, weiter bevorzugt >30°C, noch weiter bevorzugt von >40°C auf. Bevorzugte Nichtlöser weisen daher einen Siedepunkt zwischen 25°C und 200°C, weiter bevorzugt zwischen 30°C und 150°C, noch weiter bevorzugt zwischen 40°C und 100°C auf. Die Angaben zu den Schmelzpunkten und Siedepunkten beziehen sich hierbei auf den Normaldruck (101 hPa; 1 bar, 1 atm).

Bevorzugte Nichtlöser weisen zudem bei Normaltemperatur (20°C) einen Dampfdruck von <600 hPa, weiter bevorzugt <300 hPa, noch weiter bevorzugt <200 hPa auf. Bevorzugte Nichtlöser weisen zudem einen Dampfdruck bei 20°C von >1 hPa, weiter bevorzugt >10 hPa, noch weiter bevorzugt >30 hPa auf. Bevorzugte Nichtlöser weisen daher einen Dampfdruck Normaltemperatur (20°C) zwischen 1hPa bis 600 hPa, weiter bevorzugt zwischen 10 hPa und 300 hPa, noch weiter bevorzugt zwischen 30 hPa und 200 hPa auf.

Bevorzugte Nichtlöser weisen kein permanentes Dipolmoment auf, d.h. weisen ein Dipolmoment von 0,0 bis maximal 0,1 D (0,0 - 0,3·10⁻³⁰Cm) auf.

Bevorzugte Nichtlöser weisen bei Normaltemperatur (20°C) eine Dielektrizitätskonstante εᵣ von ≤ 2,5, weiter bevorzugt ≤ 2,2, noch weiter bevorzugt von ≤ 2,0 auf. Bevorzugte Nichtlöser weisen einen n-Octanol-Wasser-Verteilungskoeffizienten logK_{OW} von > 2,0, weiter bevorzugt von ≥ 2,5, noch weiter bevorzugt von ≥ 3,0 auf. Bevorzugt sind daher Nichtlöser die bei Normaltemperatur (20°C) eine Dielektrizitätskonstante εᵣ von ≤ 2,5 und einen logK_{OW} von > 2,0, weiter bevorzugt eine Dielektrizitätskonstante εᵣ von ≤ 2,2 und einen logK_{OW} von ≥ 2,5, noch weiter bevorzugt eine Dielektrizitätskonstante εᵣ von ≤ 2,0 und einen einen logK_{OW} von ≥ 3,0 aufweisen.

Bevorzugte Nichtlöser weisen zudem bei Normaltemperatur (20°C) eine Dichte von <0,95 g/mL, weiter bevorzugt <0,9 g/mL, noch weiter bevorzugt von <0,8g/mL auf. Bevorzugte Nichtlöser weisen zudem bei Normaltemperatur (20°C) eine Viskosität von <2,0 mPa s, weiter bevorzugt <1,5 mPa s, noch weiter bevorzugt <1,0 mPa s auf.

Somit sind hierin Nichtlöser bevorzugt, die bei Normaltemperatur (20°C) und Normaldruck (101 hPa; 1 bar, 1 atm) im flüssigen Aggregatzustand vorliegen und einen Schmelzpunkt von <20°C, weiter bevorzugt <15°C, noch weiter bevorzugt <10°C, einen Siedepunkt von <200°C, weiter bevorzugt <150°C, noch weiter bevorzugt von <100°C, bzw. einen Siedepunkt von >25°C, weiter bevorzugt >30°C, noch weiter bevorzugt von >40°C, einen Dampfdruck bei 20°C von <600 hPa, weiter bevorzugt <300 hPa, noch weiter bevorzugt <200 hPa, bzw. einen Dampfdruck bei 20°C von >1 hPa, weiter bevorzugt >10 hPa, noch weiter bevorzugt >30hPa, eine Dichte von <0,95 g/mL, weiter bevorzugt <0,9 g/mL, noch weiter bevorzugt von <0,8 g/mL, ein Dipolmoment von 0,0 - 0,1 D (0,0 - 0,3·10⁻³⁰Cm), eine Viskosität bei 20°C von <2,0 mPa s, weiter bevorzugt <1,5 mPa s, noch weiter bevorzugt <1,0 mPa s, und insbesondere eine Dielektrizitätskonstante εᵣ bei 20°C von ≤ 2,5, weiter bevorzugt ≤ 2,2, noch weiter bevorzugt von ≤ 2,0, einen n-Octanol-Wasser-Verteilungskoeffizienten logK_{OW} von > 2,0, weiter bevorzugt von ≥ 2,5, noch weiter bevorzugt von ≥ 3,0 aufweisen.

Eine Übersicht von Orientierungswerten (die angegebenen Werte sind gerundete Werte) der Dielektrizitätskonstante und logK_{OW} von hierin geeigneten Nichtlösern ist in Tabelle 1 gezeigt. Eine Übersicht von weiteren Parametern von einigen konkreten Nichtlösern ist in Tabelle 2 gezeigt (die angegebenen Werte sind gerundete Werte).

**Tabelle 1: Übersicht Dielektrizitätskonstanten und logK_{OW}-Werte von Nichtlösern (gerundete Werte)**

| Lösungsmittel | Dielektrizitätskonstante εᵣ (20°C) | logK_{OW} |
|---|---|---|
| n-Alkane | ca. 1,80-2,00 | ca. 3-8 |
| iso-Alkane | ca. 1,80-1,95 | ca. 3-8 |
| Cycloalkane | ca. 1,90-2,20 | ca. 3-8 |

**Tabelle 2: Übersicht einiger physikalische Parameter von einigen Nichtlösern (gerundete Werte)**

| Lösungsmittel | Dielektrizitätskonstante εᵣ (20°C) | Siedepunkt (°C) | Schmelzpunkt (°C) | Dampfdruck (20°C, hPa) | logK_{OW} |
|---|---|---|---|---|---|
| Pentan | 1,84 | 36,1 | -129,7 | 573 | 3,2 |
| Hexan | 1,89 | 69 | -95 | 160 | 3,8 |
| Heptan | 1,92 | 98 | -90,6 | 48 | 4,5 |
| Octan | 1,95 | 126 | -57 | 14 | 5,2 |
| Nonan | 1,97 | 151 | -54 | 4,8 | 5,7 |
| Decan | 2,00 | 174 | -30 | 1,66 | 6,3 |
| Undecan | 2,01 | 196 | -26 | 0,55 | 6,5 |
| Isopentan | 1,85 | 28 | -160 | 761 | 3,2 |
| 2-Methylpentan | 1,89 | 60 | -154 | 227 | 3,6 |
| 2,2-Dimethylbutan | 1,87 | 50 | -100 | 348 | 3,8 |
| 2,2-Dimethylpentan | 1,91 | 79 | -123 | 111 | 3,8 |
| 2-Methylhexan | 1,92 | 90 | -118 | 53 | 3,7 |
| 2,3-Dimethylpentan | 1,94 | 90 | -135 | 72 | 3,8 |
| 2,2,4-Trimethylpentan | 1,94 | 98 | -107 | 53 | 4,1 |
| Cyclopentan | 1,97 | 49,3 | -93,9 | 346 | 3,0 |
| Cyclohexan | 2,02 | 80,7 | 6,5 | 104 | 3,4 |
| Methylcyclopentan | 1,99 | 71 | -143 | 18 | 3,4 |
| tert-Butylcyclohexan | 2,08 | 167 | -41 | <20 | 4,0 |
| Methylcyclohexan | 2,02 | 101 | -127 | <20 | 3,9 |
| Cycloheptan | 2,08 | 119 | -8 | 22,3 | 4 |
| Cyclooctan | 2,12 | 150 | 13 | 5,5 | 4,5 |
| Cyclononan | 2,14 | 170 | 11 | <5 | 5,1 |
| Cyclodecan | 2,15 | 201 | 10 | <5 | 5,5 |

Bevorzugte Nichtlöser mit einem Schmelzpunkt von <15°C, einer Dielektrizitätskonstante εᵣ bei 20°C von ≤ 2,5, einem logK_{OW} von ≥ 3,0, einem Siedepunkt von <200°C, einem Siedepunkt von >30°C, einem Dampfdruck bei 20°C zwischen 1 hPa und 600 hPa schließen ein, sind aber nicht beschränkt auf Pentan, Hexan, Heptan, Octan, Nonan, Decan, Petrolether, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, Cyclopentan, Cyclohexan, Methylcyclopentan, tert-Butylcyclohexan, Methylcyclohexan, Cycloheptan, Cyclooctan, Cyclononan, Cyclodecan, 2,3-Dimethylcyclobutan, 1,2-Dimethylcyclobutan, Decalin, Pinan. Die Angaben zu den Schmelzpunkten und Siedepunkten beziehen sich hierbei auf den Normaldruck (101 hPa; 1 bar, 1atm).

Hierin bevorzugte Nichtlöser sind Pentan, Cyclopentan, Hexan, Cyclohexan, Heptan, Octan, Nonan und Decan.

Weiter bevorzugte Nichtlöser mit einem Schmelzpunkt von <10°C, einer Dielektrizitätskonstante εᵣ bei 20°C von ≤ 2,0, einem logK_{OW} von ≥ 3,0, einem Siedepunkt von <150°C, einem Siedepunkt von >30°C, einem Dampfdruck bei 20°C zwischen 10 hPa und 600hPa schließen ein, sind aber nicht beschränkt auf Pentan, Hexan, Heptan, Octan, Petrolether, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, Cyclopentan, Cyclohexan, Methylcyclopentan, Methylcyclohexan, Cycloheptan, 2,3-Dimethylcyclobutan, 1,2-Dimethylcyclobutan. Die Angaben zu den Schmelzpunkten und Siedepunkten beziehen sich hierbei auf den Normaldruck (101 hPa; 1 bar, 1 atm).

Noch weiter bevorzugte Nichtlöser mit einem Schmelzpunkt von <10°C, einer Dielektrizitätskonstante εᵣ bei 20°C von ≤ 2,0, einem logK_{OW} von ≥ 3,0, einem Siedepunkt von <100°C, einem Siedepunkt von >40°C, einem Dampfdruck bei 20°C zwischen 30 hPa und 300hPa schließen ein, sind aber nicht beschränkt auf Hexan, Heptan, 2-Methylpentan, 3-Methylpentan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, Cyclohexan, Cycloheptan, 2,3-Dimethylcyclobutan, 1,2-Dimethylcyclobutan. Die Angaben zu den Schmelzpunkten und Siedepunkten beziehen sich hierbei auf den Normaldruck (101 hPa; 1 bar, 1 atm).

Hierin besonders bevorzugte Nichtlöser sind Hexan, Cyclohexan und Heptan.

Der Begriff **"unpolares organisches Lösungsmittel",** wie hierin verwendet, bezieht sich auf ein Lösungsmittel auf Kohlenstoffbasis, das bei Normaltemperatur (20°C) und Normaldruck (101 hPa; 1 bar, 1 atm) flüssig ist, d.h. mindestens einen Schmelzpunkt von <20°C aufweist. Beispiele von unpolaren organischen Lösungsmitteln schließen ein, sind aber nicht beschränkt auf Tetrachlorkohlenstoff, reine Kohlenwasserstofflösungsmittel wie beispielsweise Pentan, Cyclopentan, Hexan, Cyclohexan, Heptan, Octan, Nonan oder Decan, aromatische Lösungsmittel wie Toluol, Benzol und Xylol.

Unpolare organische Lösungsmittel, wie hierin definiert, weisen eine Dielektrizitätskonstante εᵣ bei 20°C von ≤ 10, weiter bevorzugt von ≤ 5,0, weiter bevorzugt ≤ 3,0, noch weiter bevorzugt von ≤ 2,0 und gleichzeitig einen n-Octanol-Wasser-Verteilungskoeffizienten logK_{OW} > 2,0, weiter bevorzugt von ≥ 2,5, noch weiter bevorzugt von ≥ 3,0. Ein Lösungsmittel mit einer Dielektrizitätskonstante εᵣ bei 20°C von ≤ 10 und einem logK_{OW} ≤ 2,0, insbesondere ≤ 1,5 stellt hierin somit kein unpolares organisches Lösungsmittel dar. Beispielsweise hat 1,4-Dioxan eine Dielektrizitätskonstante εᵣ bei 20°C von ca. 2,3, aber einen logK_{OW} von ca. -0,4 und stellt hierin somit kein unpolares organisches Lösungsmittel dar.

Bevorzugt sind daher unpolare organische Lösungsmittel, die bei Normaltemperatur (20°C) eine Dielektrizitätskonstante εᵣ von ≤ 10 und einen logK_{OW} von > 2,0, weiter bevorzugt eine Dielektrizitätskonstante εᵣ von ≤ 5 und einen logK_{OW} von ≥ 2,5, noch weiter bevorzugt eine Dielektrizitätskonstante εᵣ von ≤ 3,0 und einen logK_{OW} von ≥ 3,0 aufweisen. Insbesondere bevorzugt sind unpolare organische Lösungsmittel die bei Normaltemperatur (20°C) eine Dielektrizitätskonstante εᵣ von ≤ 2,0 und einen logK_{OW} von > 3,0 aufweisen.

Für die vorliegende Erfindung geeignete unpolare organische Lösungsmittel liegen bei Normaltemperatur (20°C) und Normaldruck (101 hPa; 1 bar, 1 atm) im flüssigen Aggregatzustand vor. Bevorzugte unpolare organische Lösungsmittel weisen einen Schmelzpunkt von <20°C, weiter bevorzugt <15°C, noch weiter bevorzugt <10°C auf. Bevorzugte unpolare organische Lösungsmittel weisen zudem einen Siedepunkt von <200°C, weiter bevorzugt <150°C, noch weiter bevorzugt von <100°C auf. Bevorzugte unpolare organische Lösungsmittel weisen ebenfalls einen Siedepunkt von >25°C, weiter bevorzugt >30°C, noch weiter bevorzugt von >40°C auf. Bevorzugte unpolare organische Lösungsmittel weisen daher einen Siedepunkt zwischen 25°C und 200°C, weiter bevorzugt zwischen 30°C und 150°C, noch weiter bevorzugt zwischen 40°C und 100°C auf. Die Angaben zu den Schmelzpunkten und Siedepunkten beziehen sich hierbei auf den Normaldruck (101 hPa; 1 bar, 1 atm).

Bevorzugte unpolare organische Lösungsmittel weisen zudem bei Normaltemperatur (20°C) einen Dampfdruck von <600 hPa, weiter bevorzugt <300 hPa, noch weiter bevorzugt <200 hPa auf. Bevorzugte unpolare organische Lösungsmittel weisen zudem einen Dampfdruck bei 20°C von >1 hPa, weiter bevorzugt >10 hPa, noch weiter bevorzugt >30 hPa auf. Bevorzugte unpolare organische Lösungsmittel weisen daher einen Dampfdruck Normaltemperatur (20°C) zwischen 1hPa bis 600hPa, weiter bevorzugt zwischen 10 hPa und 300 hPa, noch weiter bevorzugt zwischen 30 hPa und 200 hPa auf.

Beispiele von unpolaren organischen Lösungsmitteln die bei Normaltemperatur (20°C) eine Dielektrizitätskonstante εᵣ von ≤ 10 und einen logK_{OW} von > 2 aufweisen, schließen ein, sind aber nicht beschränkt auf unverzweigte C₅₋₁₆-Alkane wie Pentan, Hexan, Heptan, Octan, Nonan, Decan, Petrolether, verzweigte C₅₋₁₆-Alkane wie Isopentan, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, oder C₅₋₁₆-Cycloalkane wie Cyclopentan, Cyclohexan, Methylcyclopentan, tert-Butylcyclohexan, Methylcyclohexan, Cycloheptan, Cyclooctan, Cyclononan, Cyclodecan, 2,3-Dimethylcyclobutan, 1,2-Dimethylcyclobutan, Decalin, Pinan 1,4-Dimethylcyclohexan, 1,1-Dimethylcyclohexan, Spiropentan, Spirohexan, Spiroheptan, Ligroin, Halogenalkane wie Tetrachlorkohlenstoff, Tetradecafluorhexan aromatische Kohlenwasserstoffe wie Benzol, aromatische Kohlenwasserstoffe mit gesättigten aliphatischen Substituenten wie Toluol, o-Xylol, m-Xylol, p-Xylol, Mesitylen, 1-Phenylbutan, 2-Methyl-1-phenylpropan, 2-Phenbutan, Cumol, iso-Butylbenzol, Propylbenzol, Hexylbenzol, iso-Butylbenzol, Halogenaromaten wie Chlorbenzol, Fluorbenzol, p-Dichlorbenzol, o-Dichlorbenzol, Hexafluorbenzol, Brombenzol, Benzylchlorid, Benzylbromid oder auch weitere substituierte Aromaten wie Anisol und Ethoxybenzol.

Somit sind hierin unpolare organische Lösungsmittel bevorzugt, die bei Normaltemperatur (20°C) und Normaldruck (101 hPa; 1 bar, 1 atm) im flüssigen Aggregatzustand vorliegen und einen Schmelzpunkt von <20°C, weiter bevorzugt <15°C, noch weiter bevorzugt <10°C, einen Siedepunkt von <200°C, weiter bevorzugt <150°C, noch weiter bevorzugt von <100°C, bzw. einen Siedepunkt von >25°C, weiter bevorzugt >30°C, noch weiter bevorzugt von >40°C, einen Dampfdruck bei 20°C von <600 hPa, weiter bevorzugt <300 hPa, noch weiter bevorzugt <200 hPa, bzw. einen Dampfdruck bei 20°C von >1 hPa, weiter bevorzugt >10 hPa, noch weiter bevorzugt >30hPa, ein Dipolmoment von 0,0 - 0,5 D (0,0 - 1,7·10⁻³⁰Cm), bevorzugt 0,0 - 0,1 D (0,0 - 0,3·10⁻³⁰Cm), und insbesondere eine Dielektrizitätskonstante εᵣ bei 20°C von ≤ 10, weiter bevorzugt ≤ 5,0, noch weiter bevorzugt von ≤ 3,0, noch weiter bevorzugt von ≤ 2,0, und einen n-Octanol-Wasser-Verteilungskoeffizienten logK_{OW} von > 2,0, weiter bevorzugt von ≥ 2,5, noch weiter bevorzugt von ≥ 3,0 aufweisen.

Der Begriff "unpolares organisches Lösungsmittel", wie hierin verwendet, betrifft bevorzugt aprotisch-unpolare Lösungsmittel, die aufgrund der geringen Unterschiede in der Elektronegativität zwischen Kohlenstoff und Wasserstoff unpolar sind und kein permanentes Dipolmoment aufweisen, weniger bevorzugt sind daher Halogenaromaten wie Chlorbenzol, Fluorbenzol, p-Dichlorbenzol, o-Dichlorbenzol, Brombenzol, Benzylchlorid, Benzylbromid oder weitere substituierte Aromaten wie Anisol, Ethoxybenzol, die ein Dipolmoment von mindestens 1,0 D (3,3·10⁻³⁰Cm) oder eine Dielektrizitätskonstante εᵣ bei 20°C von ≥ 3 aufweisen.

Bevorzugte unpolare organische Lösungsmittel weisen daher ein Dipolmoment von 0,0 - 0,5 D (0,0 - 1,7·10⁻³⁰Cm), weiter bevorzugte unpolare organische Lösungsmittel weisen kein permanentes Dipolmoment auf, d.h. weisen ein Dipolmoment von 0,0 - 0,1 D (0,0 - 0,3·10⁻³⁰Cm) auf.

Aprotisch-unpolare Lösungsmittel sind sehr lipophil und sehr hydrophob und daher hierin als unpolare organische Lösungsmittel bevorzugt. Vertreter von hierin bevorzugten aprotisch-unpolaren Lösungsmitteln sind Alkane, Benzol und Aromaten mit aliphatischen und aromatischen Substituenten, perhalogenierte Kohlenwasserstoffe, z. B. Tetrachlorkohlenstoff oder Hexafluorbenzol.

Weitere Vertreter von aprotisch-unpolaren Lösungsmitteln sind Alkene, Alkine, Aromaten mit ungesättigten aliphatischen Substituenten und weitere, völlig symmetrisch gebaute Moleküle, wie Tetramethylsilan oder Kohlenstoffdisulfid. Die aprotisch-unpolaren Lösungsmittel wie die Alkene, Alkine, Aromaten mit ungesättigten aliphatischen Substituenten und weitere, völlig symmetrisch gebaute Moleküle, wie Tetramethylsilan oder Kohlenstoffdisulfid können hierin als unpolare organische Lösungsmittel verwendet werden, sind aber weniger bevorzugt. Werden solche aprotisch-unpolaren Lösungsmittel eingesetzt, ist zu gewährleisten, dass keine chemischen Reaktionen zwischen diesen und dem mikrokristallinen Limus-Wirkstoff und dem mindestens einen Tri-O-Acylglycerol stattfinden. Ein Fachmann auf dem technischen Gebiet ist leicht in der Lage, beurteilen zu können, ob chemische Reaktionen zwischen einem bestimmten Lösungsmittel und einem mikrokristallinen Limus-Wirkstoff bzw. Tri-O-Acylglycerol stattfinden können und ob ein bestimmtes Lösungsmittel für die Herstellung einer Kristallsuspension geeignet ist. Die Wahl eines geeigneten Lösungsmittels gehört daher zu den routinemäßigen Arbeiten eines Fachmanns auf dem Gebiet.

Bevorzugte unpolare organische Lösungsmittel sind hierin daher unverzweigte, verzweigte und cyclische gesättigte aliphatische Kohlenwasserstoffe, aromatische Kohlenwasserstoffe und aromatische Kohlenwasserstoffe mit gesättigten aliphatischen Substituenten und perhalogenierte Kohlenwasserstoffe.

Bevorzugte unpolare organische Lösungsmittel weisen somit bei Normaltemperatur (20°C) und Normaldruck (101 hPa; 1 bar, 1 atm) eine Dielektrizitätskonstante εᵣ von ≤ 3, weiter bevorzugt von ≤ 2,5, weiter bevorzugt ≤ 2,2, noch weiter bevorzugt von ≤ 2,0 und einen n-Octanol-Wasser-Verteilungskoeffizienten logK_{OW} > 2,0, weiter bevorzugt von ≥ 2,5, noch weiter bevorzugt von ≥ 3,0 auf.

Somit sind hierin unpolare organische Lösungsmittel besonders bevorzugt, die bei Normaltemperatur (20°C) und Normaldruck (101 hPa; 1 bar, 1 atm) im flüssigen Aggregatzustand vorliegen und einen Schmelzpunkt von <20°C, weiter bevorzugt <15°C, noch weiter bevorzugt <10°C, einen Siedepunkt von <200°C, weiter bevorzugt <150°C, noch weiter bevorzugt von <100°C, bzw. einen Siedepunkt von >25°C, weiter bevorzugt >30°C, noch weiter bevorzugt von >40°C, einen Dampfdruck bei 20°C von <600 hPa, weiter bevorzugt <300 hPa, noch weiter bevorzugt <200 hPa, bzw. einen Dampfdruck bei 20°C von >1 hPa, weiter bevorzugt >10 hPa, noch weiter bevorzugt >30 hPa, ein Dipolmoment von 0,0 - 0,5 D (0,0 - 1,7·10⁻³⁰Cm), bevorzugt 0,0-0,1 D (0,0 - 0,3·10⁻³⁰Cm), und eine Dielektrizitätskonstante εᵣ bei 20°C von ≤ 3, weiter bevorzugt ≤ 2,5, noch weiter bevorzugt von ≤ 2,2, noch weiter bevorzugt von ≤ 2,0, und einen n-Octanol-Wasser-Verteilungskoeffizienten logK_{OW} von > 2,0, weiter bevorzugt von ≥ 2,5, noch weiter bevorzugt von ≥ 3,0 aufweisen.

Eine Übersicht von Orientierungswerten (die angegebenen Werte sind gerundete Werte) der Dielektrizitätskonstanten und logK_{OW} von unpolaren organischen Lösungsmitteln ist in Tabelle 3 gezeigt.

**Tabelle 3: Übersicht der Dielektrizitätskonstanten und logKow-Werte für unpolare organische Lösungsmittel (gerundete Werte)**

| Lösungsmittel | Dielektrizitätskonstante εᵣ (20°C) | logK_{OW} |
|---|---|---|
| n-Alkane | ca. 1,8-2,0 | ca. 3-8 |
| iso-Alkane | ca. 1,8-1,9 | ca. 3-8 |
| Cycloalkane | ca. 1,9-2,2 | ca. 3-8 |
| Benzol | ca. 2,3 | ca. 2,1 |
| Aromaten mit aliphatischen Resten | ca. 2,2-2,5 | ca. 2-6 |
| Tetrachlorkohlenstoff | ca. 2,3 | ca. 2,8 |
| Halogenaromaten | ca. 5-10 | ca. 2-4 |
| Aromaten mit weiteren Resten | ca. 4-5 | ca. 2-3 |

Eine Übersicht einiger physikalischer Parameter von einigen konkreten Beispielen von unpolaren organischen Lösungsmitteln, zusätzlich zu den bereits in Tabelle 2 gezeigten, ist in der folgenden Tabelle 4 gezeigt (die angegebenen Werte sind gerundete Werte).

**Tabelle 4: Übersicht einiger physikalischer Parameter für einige Beispiele von unpolaren organischen Lösungsmitteln.**

| Lösungsmittel | Dielektrizitätskonstante εᵣ (20°C) | Siedepunkt (°C) | Schmelzpunkt (°C) | Dampfdruck (20°C, hPa) | logK_{OW} |
|---|---|---|---|---|---|
| Benzol | 2,3 | 80 | 6 | 100 | 2,1 |
| Toluol | 2,4 | 111 | -95 | 29,1 | 2,7 |
| o-Xylol | 2,6 | 144 | -25 | <20 | 3,1 |
| m-Xylol | 2,4 | 139 | -48 | <20 | 3,2 |
| p-Xylol | 2,3 | 138 | 13 | 15 | 3,2 |
| Mesitylen | 2,4 | 165 | -45 | 2,69 | 3,4 |
| 1-Phenylbutan | 2,4 | 183 | -88 | 1,3 | 3,4 |
| 2-Methyl-1-phenylpropan | 2,4 | 173 | -51 | 1,8 | 3,4 |
| 2-Phenylbutan | 2,4 | 173 | -75 | 1,3 | 3,4 |
| 2-Methyl-2-phenylpropan | 2,4 | 169 | -58 | 2,2 | 3,4 |
| Cumol | 2,4 | 152 | -96 | 5,3 | 3,4 |
| Ethylbenzol | 2,4 | 136 | -95 | 9,8 | 3,2 |
| iso-Butylbenzol | 2,2 | 77 | -23 | 1,8 | 4,1 |
| Tetrachlorkohlenstoff | 2,0 | 81 | 7 | 104 | 2,8 |
| Tetradecafluorhexan | 1,6 | 56 | -90 | 300 | 5,8 |
| Chlorbenzol | 5,6 | 132 | -46 | 18 | 2,9 |
| o-Dichlorbenzol | 9,9 | 180 | -17 | 1,3 | 3,4 |
| Fluorbenzol | 6,4 | 85 | -42 | 22,3 | 2,2 |
| Hexafluorbenzol | 2,1 | 81 | 4 | 77 | 2,6 |
| Anisol | 4,3 | 154 | -37 | 3,6 | 2,1 |
| Ethoxybenzol | 4,2 | 170 | -30 | 47 | 2,5 |

Bevorzugte unpolare organische Lösungsmittel mit einem Schmelzpunkt von <20°C, einer Dielektrizitätskonstante εᵣ bei 20°C von ≤ 3, einem logK_{OW} > 2,0, einem Siedepunkt von <200°C, einem Siedepunkt von >30°C, einem Dampfdruck bei 20°C zwischen 1 hPa und 600 hPa schließen ein, sind aber nicht beschränkt auf Pentan, Hexan, Heptan, Octan, Nonan, Decan, Petrolether, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, Cyclopentan, Cyclohexan, Methylcyclopentan, tert-Butylcyclohexan, Methylcyclohexan, 2,3-Dimethylcyclobutan, Cycloheptan, Cyclooctan, Cyclononan, Cyclodecan, 1,2-Dimethylcyclobutan, Decalin, Pinan, Tetrachlorkohlenstoff, Tetradecafluorhexan, Hexafluorbenzol, Benzol, Toluol, o-Xylol, m-Xylol, p-Xylol, Mesitylen, Ethylbenzol, 1-Phenylbutan, 2-Methyl-1-phenylpropan, 2-Phenbutan, Cumol, iso-Butylbenzol, Propylbenzol. Natürlich können auch Gemische von unpolaren organischen Lösungsmitteln eingesetzt werden.

Noch weiter bevorzugte unpolare organische Lösungsmittel mit einem Schmelzpunkt von <10°C, einer Dielektrizitätskonstante εᵣ bei 20°C von ≤ 3, einem logK_{OW} > 2,0, einem Siedepunkt von <150°C, einem Siedepunkt von >30°C, einem Dampfdruck bei 20°C zwischen 10 hPa und 600 hPa schließen ein, sind aber nicht beschränkt auf Pentan, Hexan, Heptan, Octan, Petrolether, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, Cyclopentan, Cyclohexan, Methylcyclopentan, Methylcyclohexan, Cycloheptan, 2,3-Dimethylcyclobutan, 1,2-Dimethylcyclobutan, Tetrachlorkohlenstoff, Tetradecafluorhexan, Hexafluorbenzol, Benzol, Toluol, o-Xylol, m-Xylol, p-Xylol, Ethylbenzol. Natürlich können auch Gemische von unpolaren organischen Lösungsmitteln eingesetzt werden.

Noch weiter bevorzugte unpolare organische Lösungsmittel mit einem Schmelzpunkt von <10°C, einer Dielektrizitätskonstante εᵣ bei 20°C von ≤ 2,5, einem logK_{OW} > 2,0, einem Siedepunkt von <100°C, einem Siedepunkt von >40°C, einem Dampfdruck bei 20°C zwischen 10 hPa und 300 hPa schließen ein, sind aber nicht beschränkt auf Hexan, Heptan, 2-Methylpentan, 3-Methylpentan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, Cyclohexan, Cycloheptan, 2,3-Dimethylcyclobutan, 1,2-Dimethylcyclobutan, Tetrachlorkohlenstoff, Tetradecafluorhexan, Hexafluorbenzol, Benzol. Natürlich können auch Gemische von unpolaren organischen Lösungsmitteln eingesetzt werden.

Noch weiter bevorzugte unpolare organische Lösungsmittel mit einem Schmelzpunkt von <10°C, einer Dielektrizitätskonstante εᵣ bei 20°C von ≤ 2,5, einem logK_{OW} ≥ 3,0, einem Siedepunkt von <100°C, einem Siedepunkt von >40°C, einem Dampfdruck bei 20°C zwischen 10 hPa und 300 hPa schließen ein, sind aber nicht beschränkt auf Hexan, Heptan, 2-Methylpentan, 3-Methylpentan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, Cyclohexan, Cycloheptan, 2,3-Dimethylcyclobutan, 1,2-Dimethylcyclobutan, Tetrachlorkohlenstoff. Natürlich können auch Gemische von unpolaren organischen Lösungsmitteln eingesetzt werden.

Bevorzugte unpolare organische Lösungsmittel sind zudem wasserfreie, d.h. getrocknete unpolare organische Lösungsmittel.

Bevorzugte unpolare organische Lösungsmittel weisen zudem bei Normaltemperatur (20°C) eine Dichte von <0,95 g/mL, weiter bevorzugt <0,9 g/mL, noch weiter bevorzugt von <0,8g/mL auf. Besonders bevorzugte unpolare organische Lösungsmittel stellen somit die hierin definierten Nichtlöser dar. Hierin besonders bevorzugte unpolare organische Lösungsmittel sind daher Hexan, Cyclohexan und Heptan.

Öle wie Kokosöl, Palmöl, Erdnussöl, Baumwollsamenöl, Rapsöl, Fischöl, Sojaöl, Leinsamenöl, Olivenöl sind im Allgemeinen unpolar und weisen eine Dielektrizitätskonstante εᵣ bei 20°C von ca. 2-5 auf. Solche Öle wie Rizinusöl, Kokosöl, Palmöl, Erdnussöl, Baumwollsamenöl, Rapsöl, Fischöl, Sojaöl, Leinsamenöl, Olivenöl sind hierin als unpolare organische Lösungsmittel weniger bevorzugt. Diese Öle sind viskos und weisen eine Viskosität bei 20°C von ca. 30 - 160 mPa s auf. Hierin bevorzugte unpolare organische Lösungsmittel weisen bei Normaltemperatur (20°C) eine Viskosität von <2,0 mPa s, weiter bevorzugt <1,5 mPa s, noch weiter bevorzugt <1,0 mPa s auf.

Der Begriff **"polares organisches Lösungsmittel",** wie hierin verwendet, bezieht sich auf ein Lösungsmittel auf Kohlenstoffbasis, das bei Normaltemperatur (20°C) und Normaldruck (101 hPa; 1 bar, 1 atm) flüssig ist, d.h. mindestens einen Schmelzpunkt von <20°C aufweist. Beispiele von gängigen polaren organischen Lösungsmitteln schließen ein, sind aber nicht beschränkt auf Acetonitril, Dimethylsulfoxid, Ether wie Dioxan, Tetrahydrofuran (THF), Diethylether, Methyl-tert-butylether (MTBE), Ketone wie beispielsweise Aceton, Butanon oder Pentanon, Alkohole wie Methanol, Ethanol, Propanol, Isopropanol, Carbonsäuren wie Ameisensäure, Essigsäure, Propionsäure, Amide wie Dimethylformamid (DMF) oder Dimethylacetamid, halogenierte Lösungsmittel wie Chloroform, Methylenchlorid sowie Carbonsäureester wie Essigsäuremethylester und Essigsäureethylester.

Polare organische Lösungsmittel, wie hierin definiert, weisen bevorzugt einen n-Octanol-Wasser-Verteilungskoeffizienten logK_{OW} von ≤ +2,0, bevorzugt von -1,0 bis +2,0 und vorzugsweise von -0,5 bis +1,5 auf, weiter bevorzugt von -0,4 bis +1,4, noch weiter bevorzugt von -0,4 bis +0,9 auf. Weiter bevorzugte polare organische Lösungsmittel weisen eine Dielektrizitätskonstante εᵣ bei 20°C von > 3, weiter bevorzugt von ≥ 5,0 auf. Bevorzugte polare organische Lösungsmittel weisen zudem eine Dielektrizitätskonstante εᵣ bei 20°C von < 50, weiter bevorzugt von < 40, noch weiter bevorzugt von < 35, am meisten bevorzugt von < 30 auf.

Hierin bevorzugte organische Lösungsmittel weisen somit einen n-Octanol-Wasser-Verteilungskoeffizienten logK_{OW} von ≤ +2,0, und eine Dielektrizitätskonstante εᵣ bei 20°C von > 3, noch weiter bevorzugt einen logK_{OW} von -1,0 bis +2,0 und eine Dielektrizitätskonstante εᵣ bei 20°C von ≥ 5,0 und < 40 auf, weiter bevorzugt einen logK_{OW} von -0,5 bis +1,5 und eine Dielektrizitätskonstante εᵣ bei 20°C von ≥ 5,0 und < 30 auf, und am meisten bevorzugt einen logK_{OW} von -0,4 bis +1,4 und eine Dielektrizitätskonstante εᵣ bei 20°C von ≥ 5,0 und < 30 auf. Bevorzugte organische Lösungsmittel weisen somit einen n-Octanol-Wasser-Verteilungskoeffizienten logK_{OW} von -1,0 bis +2,0 und eine Dielektrizitätskonstante εᵣ bei 20°C von > 3,0 bis 40, noch weiter bevorzugt einen logK_{OW} von -1,0 bis +2,0 und eine Dielektrizitätskonstante εᵣ bei 20°C von 5,0 bis 40, noch weiter bevorzugt einen logK_{OW} von -1,0 bis +2,0 und eine Dielektrizitätskonstante εᵣ bei 20°C von 5,0 bis 35, und am meisten bevorzugt einen logK_{OW} von -0,5 bis +1,5 und eine Dielektrizitätskonstante εᵣ bei 20°C von 5,0 bis 30 auf. Ein Lösungsmittel mit einer Dielektrizitätskonstante εᵣ bei 20°C von > 3 und einem logK_{OW} > 2,0, stellt hierin somit kein polares organisches Lösungsmittel dar. Beispielsweise hat Chlorbenzol eine Dielektrizitätskonstante εᵣ bei 20°C von ca. 5,6, aber einen logK_{OW} von ca. 2,9 und stellt hierin somit kein polares organisches Lösungsmittel dar.

Für die vorliegende Erfindung geeignete polare organische Lösungsmittel liegen bei Normaltemperatur (20°C) und Normaldruck (101 hPa; 1 bar, 1 atm) im flüssigen Aggregatzustand vor. Bevorzugte polare organische Lösungsmittel weisen einen Schmelzpunkt von <20°C, weiter bevorzugt <15°C, noch weiter bevorzugt <10°C auf.

Bevorzugte polare organische Lösungsmittel weisen zudem einen Siedepunkt von <200°C, weiter bevorzugt <150°C, noch weiter bevorzugt von <100°C auf. Bevorzugte polare organische Lösungsmittel weisen ebenfalls einen Siedepunkt von >25°C, weiter bevorzugt >30°C, noch weiter bevorzugt von >40°C auf. Bevorzugte polare organische Lösungsmittel weisen daher einen Siedepunkt zwischen 25°C und 200°C, weiter bevorzugt zwischen 30°C und 150°C, noch weiter bevorzugt zwischen 40°C und 100°C auf. Die Angaben zu den Schmelzpunkten und Siedepunkten beziehen sich hierbei auf den Normaldruck (101 hPa; 1 bar, 1atm).

Bevorzugte polare organische Lösungsmittel weisen zudem bei Normaltemperatur (20°C) einen Dampfdruck von <600 hPa, weiter bevorzugt <300 hPa, noch weiter bevorzugt <200 hPa auf. Bevorzugte polare organische Lösungsmittel weisen zudem einen Dampfdruck bei 20°C von >1 hPa, weiter bevorzugt >10 hPa, noch weiter bevorzugt >30hPa auf. Bevorzugte polare organische Lösungsmittel weisen daher einen Dampfdruck bei Normaltemperatur (20°C) zwischen 1 hPa bis 600 hPa, weiter bevorzugt zwischen 10 hPa und 300 hPa, noch weiter bevorzugt zwischen 30 hPa und 200 hPa auf.

Bevorzugte polare organische Lösungsmittel weisen ein Dipolmoment von ≥ 1,0 D (3,3·10⁻³⁰Cm), weiter bevorzugte polare organische Lösungsmittel weisen ein Dipolmoment von ≤ 3,0 D (9,9·10⁻³⁰Cm), noch weiter bevorzugt ≤ 2,0 D (6,6·10⁻³⁰Cm) auf.

Der Begriff "polares organisches Lösungsmittel", wie hierin verwendet, betrifft aprotisch-polare Lösungsmittel und protische Lösungsmittel. Bei aprotisch-polaren Lösungsmitteln ist das Molekül asymmetrisch substituiert, so dass dieses ein Dipolmoment aufweist. Beispiele von aprotisch-polaren Lösungsmitteln sind Ether, Ester, Säureanhydride, Ketone, z. B. Aceton, tertiäre Amine, Pyridin, Furan, Thiophen, asymmetrisch halogenierte Kohlenwasserstoffe, Nitromethan, Dimethylformamid (DMF), Dimethylsulfoxid (DMSO), Dimethylcarbonat, Tetramethylharnstoff, Tetraethylharnstoff, Dimethylpropylenharnstoff (DMPU), 1,3-Dimethyl-2-imidazolidinon (DMEU). Das wichtigste protische Lösungsmittel ist Wasser. Beispiele von weiteren protischen Lösungsmitteln sind Alkohole, Aldehyde und Carbonsäuren. Protisch sind somit Wasser, Methanol, Ethanol und andere Alkohole, primäre und sekundäre Amine, Formamid und Carbonsäuren wie Ameisensäure und Essigsäure.

Bevorzugte polare organische Lösungsmittel sind insbesondere die aprotisch-polaren Lösungsmittel, da diese im Allgemeinen mit den unpolaren organischen Lösungsmitteln, wie hierin definiert, insbesondere den Nichtlösern, wie hierin definiert, in jedem Mischungsverhältnis mischbar sind.

Eine Übersicht von Orientierungswerten (die angegebenen Werte sind gerundete Werte) der Dielektrizitätskonstanten und logK_{OW} für einige polare organische Lösungsmittel ist in Tabelle 5 gezeigt.

**Tabelle 5: Übersicht Dielektrizitätskonstanten und logK_{OW} für einige polare organische Lösungsmittel (gerundete Werte)**

| Lösungsmittel | Dielektrizitätskonstante εᵣ (20°C) | logK_{OW} |
|---|---|---|
| Halogenalkane | ca. 3-11 | ca. +1,0 - +1,98 |
| Alkylether | ca. 4-8 | ca. -0,4 - +1,98 |
| Alkylketone | ca. 15-25 | ca. -0,5 - +1,5 |
| Pyridine | ca. 9-13 | ca. +0,6 |
| Alkylnitrile | ca. 25-38 | ca. -0,3 - +0,5 |
| Alkylamine | ca. 3-7 | ca. -0,8 |
| Alkyldiole | ca. 30-50 | ca. -0,9 |
| höhere Alkylalkohole | ca. 5-17 | ca. +0,8 - ≥ +2,0 |
| niedere Alkylalkohole | ca. 19-32 | ca. -0,7 - +0,3 |
| Aldehyde | ca. 9-18 | ca. - 4,0 |
| Ameisensäure | ca. 50 | ca. -0,5 |
| Höhere Carbonsäuren | ca. 2-6 | ca. -0,2 |
| Alkylester | ca. 3 - 9 | ca. +0,0 - ≥ +2,0 |
| Wasser | ca. 80 | ca. -1,4 |
| DMF | ca. 37 | ca. -1,0 |
| DMSO | ca. 47 | ca. -1,4 |

Eine Übersicht einiger physikalischer Parameter (die angegebenen Werte sind gerundete Werte) von einigen polaren organischen Lösungsmitteln ist in der folgenden Tabelle 6 gezeigt.

**Tabelle 6: Übersicht einiger physikalischer Parameter für einige Beispiele von polaren organischen Lösungsmitteln**

| Lösungsmittel | Dielektrizitätskonstante εᵣ (20°C) | Siedepunkt (°C) | Schmelzpunkt (°C) | Dampf -druck (20°C, hPa) | logK_{OW} |
|---|---|---|---|---|---|
| Dichlormethan | 9,0 | 40 | -95 | 475 | +1,3 |
| Chloroform | 4,8 | 61 | -64 | 210 | +2,0 |
| Diethylether | 4,3 | 35 | -117 | 586 | +0,9 |
| Dipropylether | 3,3 | 90 | -122 | 73 | +2,0 |
| 2-Pentanon | 15,4 | 101 | -78 | | +0,9 |
| Tetrahydrofuran (THF) | 7,6 | 66 | --109 | 173 | +0,5 |
| 1,4-Dioxan | 2,3 | 101 | -12 | 38 | -0,4 |
| Aceton | 20,7 | 56 | -95 | 246 | -0,2 |
| Acetonitril | 37 | 82 | -44 | 94 | -0,3 |
| Nitromethan | 37 | 101 | -29 | 36 | -0,4 |
| Essigsäure | 6,2 | 118 | -17 | 15,8 | -0,2 |
| Methanol | 32,8 | 65 | -98 | 129 | -0,7 |
| Ethanol | 24,9 | 78 | -114 | 58 | -0,3 |
| 1-Propanol | 20,2 | 97 | -126 | 104 | +0,3 |
| iso-Propanol | 19,9 | 82 | -88 | 44 | +0,1 |
| Ethylenglycol | 37,0 | 197 | -16 | 22,3 | -1,4 |
| Glycerin | 47,0 | 290 | 18 | 77 | -1,8 |
| Methylacetat | 7,1 | 57 | -99 | 228 | +0,2 |
| Ethylacetat | 6,0 | 77 | -84 | 98 | +0,7 |
| n-Propylacetat | 5,6 | 102 | -95 | 33 | +1,4 |
| DMF | 37 | 153 | -60 | 3,8 | -1,0 |
| Dimethylacetamid | 38 | 166 | -20 | 3,3 | -0,8 |
| N-Methylpyrrolidon | 32 | 203 | -24 | 0,3 | -0,5 |
| DMSO | 47 | 189 | 18 | 0,6 | -1,4 |
| Wasser | 80 | 100 | 0 | 23 | |

Bevorzugte polare organische Lösungsmittel sind Tetrahydrofuran, Aceton, Methanol, Ethanol, n-Propanol, iso-Propanol Chloroform, Methylenchlorid (Dichlormethan) und Essigsäureethylester (Ethylacetat).

Weiter bevorzugte polare organische Lösungsmittel sind Tetrahydrofuran, Aceton, Ethanol, n-Propanol, iso-Propanol und Essigsäureethylester.

Am meisten bevorzugt sind die physiologisch weitgehend unbedenklichen Lösungsmittel Ethanol, iso-Propanol und Ethylacetat. Besonders bevorzugt ist Ethylacetat.

Natürlich können auch Gemische von polaren organischen Lösungsmitteln eingesetzt werden.

Wasser gilt als sehr polares organisches Lösungsmittel, ist aber zu vermeiden, da sich wasserenthaltende Beschichtungen schlecht trocknen lassen. Zudem sind die erfindungsgemäßen Tri-O-Acylglycerole in sehr polaren Lösungsmitteln wie Wasser schwer oder praktisch unlöslich. Eine Suspension, die als Lösungsmittel ausschließlich Wasser enthält, ist nicht erfindungsgemäß, da die Tri-O-Acylglycerole ausgewählt aus der Gruppe bestehend aus Trioctanoylglycerol, Trinonanoylglycerol, Tridecanoylglycerol und Triundecanoylglycerol in Wasser nicht in gelöster Form vorliegen können. Es können aber auch wasserhaltige Lösungsmittelgemische von mit Wasser mischbaren polaren organischen Lösungsmitteln bereitgestellt werden, in denen das mindestens eine Tri-O-Acylglycerol ausgewählt aus der Gruppe bestehend aus Trioctanoylglycerol, Trinonanoylglycerol, Tridecanoylglycerol und Triundecanoylglycerol in gelöster Form vorliegen, z.B. Lösungsmittelgemische wie Wasser/Methanol (25:75), Wasser/Isopropanol (35:65) oder auch Wasser/Acetonitril (15:85). Dennoch sind hierin wasserfreie Lösungsmittelgemische bevorzugt.

Besonders bevorzugt sind daher wasserfreie Suspensionen zur Beschichtung eines Medizinprodukts ausgewählt aus einem Katheterballon, einem Ballonkatheter, einem Stent oder einer Kanüle, enthaltend mindestens ein Tri-O-Acylglycerol ausgewählt aus der Gruppe bestehend aus Trioctanoylglycerol, Trinonanoylglycerol, Tridecanoylglycerol und Triundecanoylglycerol, und mindestens einen Limus-Wirkstoff in Form von Mikrokristallen; und ein Lösungsmittel oder ein Lösungsmittelgemisch, wobei das mindestens eine Tri-O-Acylglycerol in dem mindestens einen Lösungsmittel oder dem Lösungsmittelgemisch gelöst vorliegt, und wobei sich die Mikrokristalle des mindestens einen Limus-Wirkstoffs in dem Lösungsmittel oder dem Lösungsmittelgemisch enthaltend das gelöste mindestens eine Tri-O-Acylglycerol nicht lösen.

Eine "wasserfreie Suspension", wie hierin verwendet, enthält maximal 20 Vol.-% Wasser, bevorzugt weniger als 20 Vol.-%, weiter bevorzugt weniger als 10 Vol.-%, weiter bevorzugt weniger als 5 Vol.-%, weiter bevorzugt weniger als 3 Vol.-%, weiter bevorzugt weniger als 2 Vol.-%, weiter bevorzugt weniger als 1,5 Vol.-%, noch weiter bevorzugt weniger 1 Vol.-%, noch weiter bevorzugt weniger als 0,5 Vol.-% und am meistens bevorzugt weniger als 0,1 Vol.-% Wasser bezogen auf das Gesamtvolumen der Suspension.

In einigen bevorzugten Ausführungsformen enthält die Suspension der vorliegenden Erfindung ein Lösungsmittelgemisch, worin sich das mindestens eine Tri-O-Acylglycerol löst und sich die Mikrokristalle des mindestens einen Limus-Wirkstoffs nicht lösen. Bevorzugte Lösungsmittelgemische sind hierin Gemische von mindestens einem unpolaren organischen Lösungsmittel, bevorzugt mindestens einem Nichtlöser und mindestens einem polaren organischen Lösungsmittel. Unpolares organisches Lösungsmittel bzw. Nichtlöser und polares organischen Lösungsmittel müssen miteinander mischbar sein und vorzugsweise in jedem Verhältnis miteinander mischbar sein und eine homogene Mischung ergeben.

Die unpolaren organischen Lösungsmittel, wie hierin definiert, sind im Allgemeinen mit Wasser und anderen stark polaren organischen Lösungsmittel wie z.B. kurzkettigen Alkoholen wie Methanol nicht in jedem Verhältnis mischbar. Unpolare organische Lösungsmittel, die mit Wasser nicht mischbar sind, schließen ein, sind aber nicht beschränkt auf Benzol, Tetrachlorkohlenstoff, Cyclohexan, Heptan, Hexan, Isooctan, Pentan, Toluol und Xylol. Somit sind insbesondere die Nichtlöser, wie hierin definiert, mit Wasser nicht mischbar. Lösungsmittelgemische enthaltend mindestens einen Nichtlöser enthalten daher besonders bevorzugt kein Wasser als Mischungskomponente. Weiterhin sind einige unpolare organische Lösungsmittel wie Xylol, Cyclohexan, Heptan, Hexan, Isooctan und Pentan auch mit sehr polaren organischen Lösungsmitteln wie Dimethylsulfoxid und Dimethylformamid nicht in jedem Verhältnis mischbar. Zudem sind die Nichtlöser, wie hierin definiert, wie beispielsweise Cyclohexan, Heptan, Hexan, Isooctan und Pentan ebenfalls nicht mischbar mit polaren organischen Lösungsmitteln wie Acetonitril und Methanol.

Lösungsmittelgemische von mindestens einem Nichtlöser, wie hierin definiert, und mindestens einem polaren organischen Lösungsmittel enthalten daher besonders bevorzugt polare organische Lösungsmittel mit einem einen logK_{OW} von -0,5 bis +1,5 und einer Dielektrizitätskonstante εᵣ bei 20°C von < 30, weiter bevorzugt einen logK_{OW} von -0,4 bis +1,4 und einer Dielektrizitätskonstante εᵣ bei 20°C von < 30.

In einigen Ausführungsformen liegt das Volumenverhältnis in der Suspension zwischen dem unpolaren organischen Lösungsmittel und dem polaren organischen Lösungsmittel zwischen 25 : 75 bis 75 : 25, bevorzugt zwischen 30 : 70 bis 70 : 30 und weiter bevorzugt zwischen 35 : 65 und 65 : 35.

Bevorzugt liegt das Volumenverhältnis in der Suspension zwischen dem unpolaren organischen Lösungsmittel und dem polaren organischen Lösungsmittel zwischen 99:1 bis 65:35, bevorzugt zwischen 95:5 bis 70:30 und weiter bevorzugt zwischen 80:20 und 65:35, weiter bevorzugt zwischen 90:10 und 80:15 und am meisten bevorzugt bei 85:15.

Weiter bevorzugt sind Lösungsmittelgemische die mindestens 50 Vol.-% unpolares organisches Lösungsmittel enthalten, weiter bevorzugt mindestens 55 Vol.-% unpolares organisches Lösungsmittel, weiter bevorzugt mindestens 60 Vol.-% unpolares organisches Lösungsmittel, weiter bevorzugt mindestens 65 Vol.-% unpolares organisches Lösungsmittel, weiter bevorzugt mindestens 70 Vol.-% unpolares organisches Lösungsmittel, weiter bevorzugt mindestens 75 Vol.-% unpolares organisches Lösungsmittel und am meisten bevorzugt mindestens 80 Vol.-% unpolares organisches Lösungsmittel enthalten.

Weiter bevorzugt sind Lösungsmittelgemische die mindestens 50 Vol.-% Nichtlöser enthalten, weiter bevorzugt mindestens 55 Vol.-% Nichtlöser, weiter bevorzugt mindestens 60 Vol.-% Nichtlöser, weiter bevorzugt mindestens 65 Vol.-% Nichtlöser, weiter bevorzugt mindestens 70 Vol.-% Nichtlöser, weiter bevorzugt mindestens 75 Vol.-% Nichtlöser und am meisten bevorzugt mindestens 80 Vol.-% Nichtlöser enthalten.

In bevorzugten Ausführungsformen enthält das Lösungsmittelgemisch daher mindestens ein unpolares organisches Lösungsmittel mit einer Dielektrizitätskonstante εᵣ bei 20°C von ≤ 10 und einem n-Octanol-Wasser-Verteilungskoeffizienten logK_{OW} von > 2,0, weiter bevorzugt mit einer Dielektrizitätskonstante εᵣ bei 20°C von ≤ 5,0, und einem n-Octanol-Wasser-Verteilungskoeffizienten logK_{OW} von ≥ 2,5, weiter bevorzugt mit einer Dielektrizitätskonstante εᵣ bei 20°C von ≤ 3,0 und einem n-Octanol-Wasser-Verteilungskoeffizienten logK_{OW} von ≥ 3,0 und am meisten bevorzugt mit einer Dielektrizitätskonstante εᵣ bei 20°C von ≤ 2,0.

In weiter bevorzugten Ausführungsformen enthält das Lösungsmittelgemisch mindestens 50 Vol.-%, weiter bevorzugt mindestens 55 Vol.-%, weiter bevorzugt mindestens 60 Vol.-%, weiter bevorzugt mindestens 65 Vol.-%, weiter bevorzugt mindestens 70 Vol.-%, weiter bevorzugt mindestens 75 Vol.-%, und am meisten bevorzugt mindestens 80 Vol.-% unpolares organisches Lösungsmittel mit einer Dielektrizitätskonstante εᵣ bei 20°C von ≤ 10 und einem n-Octanol-Wasser-Verteilungskoeffizienten logK_{OW} von > 2,0, weiter bevorzugt mit einer Dielektrizitätskonstante εᵣ bei 20°C von ≤ 5,0 und einem n-Octanol-Wasser-Verteilungskoeffizienten logK_{OW} von ≥ 2,5, weiter bevorzugt mit einer Dielektrizitätskonstante εᵣ bei 20°C von von ≤ 3,0 und einem n-Octanol-Wasser-Verteilungskoeffizienten logK_{OW} von ≥ 3,0 und am meisten bevorzugt mit einer Dielektrizitätskonstante εᵣ bei 20°C von ≤ 2,0.

Eine bevorzugte Kombination aus polarem organischem Lösungsmittel und unpolarem organischem Lösungsmittel ist beispielsweise Ethanol und Cyclohexan oder Ethylacetat und Heptan.

Bevorzugt sind Lösungsmittelgemische von mindestens einem polaren organischen Lösungsmittel mit einem logK_{OW} von -1,0 bis +2,0 und einer Dielektrizitätskonstante εᵣ bei 20°C von 3,0 bis 40, noch weiter bevorzugt einem logK_{OW} von -1,0 bis +2,0 und einer Dielektrizitätskonstante εᵣ bei 20°C von 5,0 bis 40, noch weiter bevorzugt einen logK_{OW} von -1,0 bis +2,0 und einer Dielektrizitätskonstante εᵣ bei 20°C von 5,0 bis 35, weiter bevorzugt einen logK_{OW} von -0,5 bis +1,5 und einer Dielektrizitätskonstante εᵣ bei 20°C von 5,0 bis 30, und am meisten bevorzugt einen logK_{OW} von -0,4 bis +0,9 und einer Dielektrizitätskonstante εᵣ bei 20°C von 5,0 bis 30, und einem unpolaren organischen Lösungsmittel mit einer Dielektrizitätskonstante εᵣ bei 20°C von ≤ 10 und einem n-Octanol-Wasser-Verteilungskoeffizienten logK_{OW} von > 2,0, weiter bevorzugt mit einer Dielektrizitätskonstante εᵣ bei 20°C von ≤ 5,0, und einem n-Octanol-Wasser-Verteilungskoeffizienten logK_{OW} von ≥ 2,5, weiter bevorzugt mit einer Dielektrizitätskonstante εᵣ bei 20°C von ≤ 3,0 und einem n-Octanol-Wasser-Verteilungskoeffizienten logK_{OW} von ≥ 3,0 und am meisten bevorzugt mit einer Dielektrizitätskonstante εᵣ bei 20°C von ≤ 2,0.

Bevorzugt sind Lösungsmittelgemische von mindestens einem polaren organischen Lösungsmittel ausgewählt aus der Gruppe umfassend oder bestehend aus Tetrahydrofuran, Aceton, Methanol, Ethanol, n-Propanol, iso-Propanol, Chloroform, Dichlormethan, Ethylacetat, bevorzugt Tetrahydrofuran, Aceton, Ethanol, n-Propanol, iso-Propanol, und Ethylacetat, weiter bevorzugt Ethanol, iso-Propanol und Ethylacetat und einem unpolaren organischen Lösungsmittel mit einer Dielektrizitätskonstante εᵣ bei 20°C von ≤ 10 und einem n-Octanol-Wasser-Verteilungskoeffizienten logK_{OW} von > 2,0, weiter bevorzugt mit einer Dielektrizitätskonstante εᵣ bei 20°C von ≤ 5,0 und einem n-Octanol-Wasser-Verteilungskoeffizienten logK_{OW} von ≥2,5, weiter bevorzugt mit einer Dielektrizitätskonstante εᵣ bei 20°C von ≤ 3,0 und einem n-Octanol-Wasser-Verteilungskoeffizienten logK_{OW} von ≥ 3,0 und am meisten bevorzugt mit einer Dielektrizitätskonstante εᵣ bei 20°C von ≤ 2,0.

Eine besonders bevorzugte Kombination aus polarem organischem Lösungsmittel und unpolarem organischem Lösungsmittel ist ein Lösungsmittelgemisch von Ethylacetat und einem wie hierin definierten unpolaren organischen Lösungsmittel mit einer Dielektrizitätskonstante εᵣ bei 20°C von ≤ 10 und einem n-Octanol-Wasser-Verteilungskoeffizienten logK_{OW} von > 2,0, weiter bevorzugt mit einer Dielektrizitätskonstante εᵣ bei 20°C von ≤ 5,0 und einem n-Octanol-Wasser-Verteilungskoeffizienten logK_{OW} von ≥2,5, weiter bevorzugt mit einer Dielektrizitätskonstante εᵣ bei 20°C von ≤ 3,0 und einem n-Octanol-Wasser-Verteilungskoeffizienten logK_{OW} von ≥ 3,0 und am meisten bevorzugt mit einer Dielektrizitätskonstante εᵣ bei 20°C von ≤ 2,0.

Eine noch bevorzugtere Kombination aus polarem organischem Lösungsmittel und unpolarem organischem Lösungsmittel ist ein Lösungsmittelgemisch von Ethylacetat und einem wie hierin definierten Nichtlöser mit einer Dielektrizitätskonstante εᵣ bei 20°C von ≤ 2,0.

Bevorzugte Kombinationen aus polarem organischem Lösungsmittel und unpolarem organischem Lösungsmittel sind Ethylacetat und ein unpolares organisches Lösungsmittel ausgewählt aus der Gruppe umfassend oder bestehend aus Pentan, Hexan, Heptan, Octan, Nonan, Decan, Petrolether, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, Cyclopentan, Cyclohexan, Methylcyclopentan, tert-Butylcyclohexan, Methylcyclohexan, 2,3-Dimethylcyclobutan, Cycloheptan, Cyclooctan, Cyclononan, Cyclodecan, 1,2-Dimethylcyclobutan, Decalin, Pinan, Tetrachlorkohlenstoff, Tetradecafluorhexan, Hexafluorbenzol, Benzol, Toluol, o-Xylol, m-Xylol, p-Xylol, Mesitylen, Ethylbenzol, 1-Phenylbutan, 2-Methyl-1-phenylpropan, 2-Phenbutan, Cumol, iso-Butylbenzol, Propylbenzol, bevorzugt Pentan, Hexan, Heptan, Octan, Petrolether, Isooctan, 2-Methylpentan, 3-Methylpentan, 2,2-Dimethylbutan, 2,3-Dimethylbutan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, 2-Methyloctan, 2-Methylheptan, 3-Methylheptan, 4-Methylheptan, Tetraethylmethan, Cyclopentan, Cyclohexan, Methylcyclopentan, Methylcyclohexan, Cycloheptan, 2,3-Dimethylcyclobutan, 1,2-Dimethylcyclobutan, Tetrachlorkohlenstoff, Tetradecafluorhexan, Hexafluorbenzol, Benzol, Toluol, o-Xylol, m-Xylol, p-Xylol, Ethylbenzol, weiter bevorzugt Hexan, Heptan, 2-Methylpentan, 3-Methylpentan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, Cyclohexan, Cycloheptan, 2,3-Dimethylcyclobutan, 1,2-Dimethylcyclobutan, Tetrachlorkohlenstoff, Tetradecafluorhexan, Hexafluorbenzol, Benzol, noch weiter bevorzugt Hexan, Heptan, 2-Methylpentan, 3-Methylpentan, 2,2-Dimethylpentan, 2-Methylhexan, 3-Methylhexan, 2,3-Dimethylpentan, 2,4-Dimethylpentan, 3,3-Dimethylpentan, 3-Ethylpentan, 2,2,4-Trimethylpentan, 2,2,4-Trimethylbutan, Cyclohexan, Cycloheptan, 2,3-Dimethylcyclobutan, 1,2-Dimethylcyclobutan, Tetrachlorkohlenstoff, und am meistens bevorzugt Hexan, Heptan und Cyclohexan.

K_{OW} ist der n-Octanol-Wasser-Verteilungskoeffizient. Der K_{OW} ist somit der Verteilungskoeffizient einer Substanz in dem Zweiphasensystem aus n-Octanol und Wasser. Der logK_{OW} ist der dekadische Logarithmus des K_{OW}. Der logK_{OW} ist im englischsprachigen Raum auch als log P bekannt. Der Kow dient als Maß für das Verhältnis zwischen Lipophilie und Hydrophilie einer Substanz. Der Wert ist größer als eins, wenn eine Substanz besser in fettähnlichen Lösungsmitteln wie n-Octanol löslich ist und kleiner als eins, wenn sie besser in Wasser löslich ist.

n-Octanol-Wasser-Verteilungskoeffizienten logK_{OW} verschiedener Lösungsmittel sind dem Fachmann wohlbekannt, siehe beispielsweise James Sangster, "Octanol-Water Partition Coefficients of Simple Organic Compounds", J. Phys. Chem. Ref. Data 1989, Vol. 18, No. 3, S. 1111-1227, das hierin durch Verweis in seiner Gesamtheit aufgenommen ist. Als polare organische Lösungsmittel werden hierin solche bezeichnet, die einen logK_{OW} von -1,0 bis +2,0 und vorzugsweise von -0,5 bis +1,5 aufweisen. Als Nichtlöser bzw. unpolare organische Lösungsmittel werden solche bezeichnet, die einen logK_{OW} ≥ 2,8 bevorzugt einen logK_{OW} ≥ 3,3 oder einen logK_{OW} von +2,8 bis +7,5 und vorzugsweise von +3,3 bis +7,0 aufweisen.

Messverfahren zur Bestimmung von n-Octanol-Wasser-Verteilungskoeffizienten sind dem Fachmann ebenfalls wohlbekannt, siehe beispielsweise James Sangster, "Octanol-Water Partition Coefficients of Simple Organic Compounds", J. Phys. Chem. Ref. Data 1989, Vol. 18, No. 3, S. 1111-1227, im Abschnitt "Messverfahren" ("Methods of Measurement"). Eine praktische Bestimmung des logK_{OW}-Wertes kann so erfolgen, dass das jeweilige Lösungsmittel mit bekannter Konzentration c_{0b}^{Wasser} in wässriger Lösung bei bekanntem Volumen V^{Wasser} vorgelegt, mit einem genau abgemessenen Volumen an Octanol V^{Octanol} überschichtet und intensiv vermischt wird. Anschließend wird die Phasentrennung abgewartet und die Octanol-Phase abgetrennt. Damit während der Phasenvermischung keine Volumenänderung mehr eintritt, wird das eingesetzte Octanol vorab mit Wasser und das eingesetzte Wasser vorab mit Octanol gesättigt. Der logK_{OW} ist positiv für lipophile und negativ für hydrophile Lösungsmittel.

**Tabelle 7: Übersicht logK_{OW}-Werte einiger polarer organischer Lösungsmittel**

| Lösungsmittel | logK_{OW} | Lösungsmittel | logK_{OW} |
|---|---|---|---|
| Acetonitril | -0,34 | Aceton | -0,24 |
| Dimethylsulfoxid | -1,35 | 2-Butanon | 0,29 |
| Dioxan | -0,42 | 2-Pentanon | 0,84 |
| Tetrahydrofuran (THF) | 0,46 | Methanol | -0,74 |
| Essigsäureethylester | 0,73 | Ethanol | -0,30 |
| Diethylether | 0,89 | 1-Propanol | 0,25 |
| Methyl-tert.-butylether (MTBE) | 0,94 | 2-Propanol | 0,05 |
| Dimethylformamid (DMF) | -1,01 | 1-Butanol | 0,84 |
| Dimethylacetamid | -0,77 | tert-Butylalkohol | 0,35 |
| Methylenchlorid | 1,25 | Essigsäure | -0,17 |
| Chloroform | 1,97 | Propionsäure | 0,32 |
| Ameisensäure | -0,54 | | |

**Tabelle 8: Übersicht logK_{OW}-Werte einiger unpolarer organischer Lösungsmittel**

| Lösungsmittel | logK_{OW} | Lösungsmittel | logK_{OW} |
|---|---|---|---|
| Toluol | 2,73 | Pentan | 3,45 |
| Benzol | 2,13 | Cyclopentan | 3,00 |
| Cyclohexan | 2,86 | Hexan | 4,00 |
| Nonan | 5,65 | Heptan | 4,50 |
| Decan | 6,25 | Octan | 5,15 |
| o-Xylol | 3,12 | Mesitylen | 3,42 |
| m-Xylol | 3,20 | Petrolether | 4,20 |
| p-Xylol | 3,15 | | |

Zwischen dem logK_{OW}-Wert des polaren organischen Lösungsmittels und dem logK_{OW}-Wert des unpolaren organischen Lösungsmittels sollte mindestens eine Differenz von 1,0, vorzugsweise mindestens 1,5 und insbesondere bevorzugt mindestens eine Differenz von 2,0 liegen.

Zur Bestimmung des K_{OW} eines Gemisches aus unpolaren organischen Lösungsmitteln werden die K_{OW}-Werte der einzelnen unpolaren organischen Lösungsmittel gewichtet nach dem Volumenanteil am Gemisch und aus den gewichteten K_{OW}-Werten der Mittelwert bestimmt.

Die Dielektrizitätskonstante (relative Dielektrizitätskonstante, Formelzeichen εᵣ) ist eine physikalische Stoffkonstante, mit deren Hilfe bestimmte Eigenschaften von Lösungsmitteln beschrieben werden können. Lösungsmittel mit hoher Dielektrizitätskonstante sind gute Lösungsmittel für ionische und andere polare Verbindungen, solche mit niedriger Dielektrizitätskonstante sind bessere Lösungsmittel für unpolare Verbindungen. Der Begriff "Dielektrizitätskonstante" wird im Stand der Technik auch als Permittivität, dielektrische Leitfähigkeit, Dielektrizität, oder dielektrische Funktion bezeichnet. Die relative Dielektrizitätskonstante εᵣ eines Mediums, auch Permittivitäts- oder Dielektrizitätszahl genannt, ist das dimensionslose Verhältnis der Permittivität ε zur Permittivität ε₀ des Vakuums. Für gasförmige, flüssige und feste Materie ist εᵣ > 1. Messverfahren zur Bestimmung der relativen Dielektrizitätskonstante sind dem Fachmann aus dem Stand der Technik wohlbekannt. Es gibt viele Methoden, die für die Messung der Dielektrizitätskonstante entwickelt wurden. Für Flüssigkeiten eignet sich beispielsweise die Methode der offenen Koaxialsonde. Bei dieser Methode wird die Sonde in die Flüssigkeiten eingetaucht und der Reflexionskoeffizient wird gemessen und zur Bestimmung der Dielektrizitätskonstante verwendet.

Gemäß der vorliegenden Erfindung enthält die erfindungsgemäße Suspension ein Lösungsmittel oder ein Lösungsmittelgemisch. Erfindungsgemäß ist mindestens ein Tri-O-Acylglycerol ausgewählt aus der Gruppe bestehend aus Trioctanoylglycerol, Trinonanoylglycerol, Tridecanoylglycerol und Triundecanoylglycerol in dem Lösungsmittel oder in dem Lösungsmittelgemisch gelöst. Erfindungsgemäß enthält die Suspension daher ein Lösungsmittel oder ein Lösungsmittelgemisch, in dem mindestens ein Tri-O-Acylglycerol ausgewählt aus der Gruppe bestehend aus Trioctanoylglycerol, Trinonanoylglycerol, Tridecanoylglycerol und Triundecanoylglycerol gelöst ist, worin sich die Mikrokristalle des mindestens einen Limus-Wirkstoffs nicht lösen oder bei Anwesenheit des mindestens einen Tri-O-Acylglycerol nicht lösen oder nicht mehr lösen.

Das Lösungsmittel oder das Lösungsmittelgemisch bildet mit dem gelösten mindestens einen Tri-O-Acylglycerol ausgewählt aus der Gruppe bestehend aus Trioctanoylglycerol, Trinonanoylglycerol, Tridecanoylglycerol und Triundecanoylglycerol somit definitionsgemäß eine Lösung, und stellt ein homogenes Gemisch dar. Die Lösung aus mindestens einem Tri-O-Acylglycerol ausgewählt aus der Gruppe bestehend aus Trioctanoylglycerol, Trinonanoylglycerol, Tridecanoylglycerol und Triundecanoylglycerol in dem Lösungsmittel oder Lösungsmittelgemisch weist somit nur eine Phase auf und das gelöste mindestens eine Tri-O-Acylglycerol ausgewählt aus der Gruppe bestehend aus Trioctanoylglycerol, Trinonanoylglycerol, Tridecanoylglycerol und Triundecanoylglycerol ist gleichmäßig in dem Lösungsmittel oder Lösungsmittelgemisch verteilt.

Somit ist die Suspension gemäß der vorliegenden Erfindung eine Kombination von:
1) einer Lösung von mindestens einem Tri-O-Acylglycerol ausgewählt aus der Gruppe bestehend aus Trioctanoylglycerol, Trinonanoylglycerol, Tridecanoylglycerol und Triundecanoylglycerol in einem Lösungsmittel oder einem Lösungsmittelgemisch, und
2) mindestens einem Limus-Wirkstoff in Form von Mikrokristallen, wobei sich die Mikrokristalle des mindestens einen Limus-Wirkstoffs in der Lösung gemäß 1) nicht lösen.

Der mindestens eine Limus-Wirkstoff in Form von Mikrokristallen liegt fein verteilt als Feststoff, also "suspendiert" in der Lösung von mindestens einem Tri-O-Acylglycerol ausgewählt aus der Gruppe bestehend aus Trioctanoylglycerol, Trinonanoylglycerol, Tridecanoylglycerol und Triundecanoylglycerol in mindestens einem Lösungsmittel oder einem Lösungsmittelgemisch vor. Somit sind die Mikrokristalle des mindestens einen Limus-Wirkstoffs erfindungsgemäß in der Lösung von mindestens einem Tri-O-Acylglycerol ausgewählt aus der Gruppe bestehend aus Trioctanoylglycerol, Trinonanoylglycerol, Tridecanoylglycerol und Triundecanoylglycerol in einem Lösungsmittel oder einem Lösungsmittelgemisch "suspendiert".

Somit betrifft die vorliegende Erfindung ebenfalls eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol;
b) mindestens einen Limus-Wirkstoff in Form von Mikrokristallen; und
c) ein Lösungsmittel oder ein Lösungsmittelgemisch,
   wobei das mindestens eine Tri-O-Acylglycerol in dem Lösungsmittel oder dem Lösungsmittelgemisch gelöst vorliegt, und
   wobei sich die Mikrokristalle des mindestens einen Limus-Wirkstoffs in dem Lösungsmittel oder dem Lösungsmittelgemisch enthaltend das gelöste mindestens eine Tri-O-Acylglycerol nicht lösen.

Anders formuliert betrifft die vorliegende Erfindung eine Suspension zur Beschichtung eines Medizinprodukts ausgewählt aus einem Katheterballon, einem Ballonkatheter, einem Stent oder einer Kanüle, die Suspension enthaltend:
a) eine Lösung von mindestens einem Trioctanoylglycerol, Trinonanoylglycerol, Tridecanoylglycerol und Triundecanoylglycerol; und
b) mindestens einen Limus-Wirkstoff in Form von Mikrokristallen, wobei die Mikrokristalle in dieser Lösung suspendiert sind.

Noch anders formuliert betrifft die vorliegende Erfindung eine Suspension zur Beschichtung eines Medizinprodukts ausgewählt aus einem Katheterballon, einem Ballonkatheter, einem Stent oder einer Kanüle, die Suspension enthaltend:
a) eine Lösung von mindestens einem Trioctanoylglycerol, Trinonanoylglycerol, Tridecanoylglycerol und Triundecanoylglycerol, und
b) in dieser Lösung suspendierte Mikrokristalle mindestens eines Limus-Wirkstoffs.

Die vorliegende Erfindung betrifft daher eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff ausgewählt wird aus Rapamycin (Sirolimus) und Everolimus.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
wobei der mindestens eine Limus-Wirkstoff eine Kristallinität von mindestens 90 Gew% aufweist,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus Rapamycin (Sirolimus) und Everolimus.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
wobei der mindestens eine Limus-Wirkstoff eine Kristallinität von mindestens 90 Gew% aufweist,
wobei der mindestens eine Limus-Wirkstoff ausgewählt wird aus Rapamycin (Sirolimus) und Everolimus.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei das mindestens eine Tri-O-Acylglycerol und der mindestens eine Limus-Wirkstoff mit einem Massenanteil von 10% - 30% Tri-O-Acylglycerol zu 90% - 70% Limus-Wirkstoff vorliegen.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das mindestens eine Tri-O-Acylglycerol und der mindestens eine Limus-Wirkstoff mit einem Massenanteil von 10% - 30 % Tri-O-Acylglycerol zu 90% - 70% Limus-Wirkstoff vorliegen.

Die vorliegende Erfindung betrifft eine Suspension zur Beschichtung eines Medizinprodukts ausgewählt aus einem Katheterballon, einem Ballonkatheter, einem Stent odereiner Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das mindestens eine Tri-O-Acylglycerol und der mindestens eine Limus-Wirkstoff mit einem Massenanteil von 10% - 30% Tri-O-Acylglycerol zu 90% - 70% Limus-Wirkstoff vorliegen.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das mindestens eine Tri-O-Acylglycerol und der mindestens eine Limus-Wirkstoff mit einem Massenanteil von 10% - 30% Tri-O-Acylglycerol zu 90% - 70% Limus-Wirkstoff vorliegen.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das mindestens eine Tri-O-Acylglycerol und der mindestens eine Limus-Wirkstoff mit einem Massenanteil von 10% - 30% Tri-O-Acylglycerol zu 90% - 70% Limus-Wirkstoff vorliegen.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei das Lösungsmittel ein Nichtlöser mit einer Dielektrizitätskonstante εᵣ bei 20°C von ≤ 2,0 ist oder das Lösungsmittelgemisch zu mindestens 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-O-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0 ist.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0 ist.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens
   eine Tri-O-Acylglycerol löst und sich die Mikrokristalle des mindestens einen Limus-Wirkstoffs nicht lösen,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das Lösungsmittel ein Nichtlöser mit einer Dielektrizitätskonstante εᵣ bei 20°C von ≤ 2,0 ist oder das Lösungsmittelgemisch zu mindestens 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff ausgewählt wird aus Rapamycin (Sirolimus) und Everolimus,
   wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff ausgewählt wird aus Rapamycin (Sirolimus) und Everolimus,
   wobei das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0 ist.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-O-Acylglycerol löst und sich die Mikrokristalle des mindestens einen Limus-Wirkstoffs nicht lösen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0 ist.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
      wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-O-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-O-Acylglycerol löst und sich die Mikrokristalle des mindestens einen Limus-Wirkstoffs nicht lösen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-*O*-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das Lösungsmittelgemisch ausgewählt wird aus Ethanol und Cyclohexan oder Essigsäureethylester und Heptan.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
      wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-*O*-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
   wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-*O*-Acylglycerol löst und sich die Mikrokristalle des mindestens einen Limus-Wirkstoffs nicht lösen,
   wobei das mindestens eine Tri-*O*-Acylglycerol und der mindestens eine Limus-Wirkstoff mit einem Massenanteil von 10 - 30 % Tri-O-Acylglycerol zu 90 - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
   wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-*O*-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus, und Temsirolimus,
   wobei das mindestens eine Tri-*O*-Acylglycerol und der mindestens eine Limus-Wirkstoff in einem Massenverhältnis von 10 - 30 % Tri-*O*-Acylglycerol zu 90 - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
      wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-*O*-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
   wobei das mindestens eine Tri-O-Acylglycerol und der mindestens eine Limus-Wirkstoff mit einem Massenanteil von 10% - 30% Tri-*O*-Acylglycerol zu 90% - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von von ≤ 3,0 und einem n-Octanol-Wasser-Verteilungskoeffizienten logK_{Ow} von ≥ 3,0 ist.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
      wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-*O*-Acylglycerol löst und sich die Mikrokristalle des mindestens einen Limus-Wirkstoffs nicht lösen,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10 - 30 % Tri-*O*-Acylglycerol zu 90 - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
      wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Die vorliegende Erfindung betrifft eine Suspension zur Beschichtung eines Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittelgemisch, worin sich das mindestens eine Tri-*O*-Acylglycerol löst und sich die Mikrokristalle des mindestens einen Limus-Wirkstoffs nicht lösen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Die Medizinprodukts ausgewählt aus einem Katheterballon, einem Ballonkatheter, einem Stent, oder einer Kanüle, die Suspension enthaltend:
a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen oder bei Anwesenheit des mindestens einen Tri-O-Acylglycerols nicht lösen, und
   wobei die Suspension 1-6% Limus-Wirkstoff enthält.

In einigen weiteren Ausführungsformen kann die Beschichtungssuspension nur aus den drei Bestandteilen a) mindestens ein Tri-*O*-Acylglycerol ausgewählt aus der Gruppe bestehend aus Trioctanoylglycerol, Trinonanoylglycerol, Tridecanoylglycerol und Triundecanoylglycerol; b) mindestens einen Limus-Wirkstoff in Form von Mikrokristallen; und c) mindestens ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen, bestehen.

### Zusatzstoffe

Neben dem vorgenannten mindestens einen Tri-*O*-Acylglycerol ausgewählt aus der Gruppe bestehend aus Trioctanoylglycerol, Trinonanoylglycerol, Tridecanoylglycerol oder Triundecanoylglycerol oder einem Gemisch aus Trioctanoylglycerol, Trinonanoylglycerol, Tridecanoylglycerol und/oder Triundecanoylglycerol können in der erfindungsgemäßen Suspension auch ein oder mehrere Zusatzstoffe enthalten sein. Der eine oder die mehreren Zusatzstoffe liegen dabei insbesondere bevorzugt gelöst in der Suspension vor.

In bevorzugten Ausführungsformen sind in der erfindungsgemäßen Suspension keine Polymere, Oligomere, Metalle oder Metallpartikel, metallorganische Verbindungen und Salze enthalten. In einigen Ausführungsformen ist die erfindungsgemäße Suspension zur Beschichtung von Medizinprodukten daher frei von Polymeren, Oligomeren, Metallen oder Metallpartikeln, metallorganischen Verbindungen und Salzen.

In bevorzugten Ausführungsformen kann in der erfindungsgemäßen Suspension als Zusatzstoff ein Antioxidans anwesend sein. Ein Antioxidans kann der Suspension zwecks Haltbarkeit des mindestens einen Limus-Wirkstoffes zugegeben werden.

Als Antioxidantien eignen sich vor allem Butylhydroxyltoluol (BHT), Butylhydroxyanisol, Ascorbylpalmitat, Ascorbylstearat, Tocopherolacetat, Ascorbinsäure, Tocopherole und Tocotrienole (z.B. alpha-Tocopherol), Carotinoide wie ß-Carotin, Zeaxanthin, Lycopin und Lutein, Vitamin C, Nordihydroguajaretsäure, Probucol, Propylgallat, sekundäre Pflanzenstoffe (Flavonoide) wie beispielsweise Catechin, Gallocatechin, Epicatechin, Epigallocatechingallat, Taxifolin, Isoliquiritigenin, Xanthohumol, Morin, Quercetin (Glycosid Rutin und Methylether Isorhamnetin), Kaempferol, Myricetin, Fisetin, Aureusidin, Luteolin, Apigenin, Hesperetin, Naringenin, Eriodictyol, Genistein, Daidzein, Licoricidin Anthocyane, Allicin, Astaxanthin Glutathion, Resveratrol, deren Derivate und deren Kombinationen.

Bevorzugte Antioxidantien sind daher Butylhydroxytoluol (BHT), das besonders in Fettphasen nach Kontakt mit Luft das Ranzig werden verhindert bzw. verzögert, Butylhydroxyanisol (BHA), Tocopherole, Carotinoide, Flavonoide, und natürlich auch Mischungen der Antioxidantien.

Besonders bevorzugt wird Butylhydroxytoluol (BHT) als Antioxidans eingesetzt.

Werden ein oder mehrere Antioxidantien der Suspension zugesetzt, berechnet sich deren Gesamtanteil zwischen 0,001 - 15,0 Gew% bzgl. Limus-Wirkstoff, bevorzugt werden 0,01 - 10,0 Gew.% und besonders bevorzugt werden 0,05 - 5,0 Gew.%.

In einigen Ausführungsformen kann in der erfindungsgemäßen Suspension als Zusatzstoff ein Flokkulationsinhibitor anwesend sein, der die Sedimentation der Mikrokristalle des mindestens einen Limus-Wirkstoffs in der Suspension verhindern kann. Geeignete Flokkulationsinhibitoren schließen ein, sind aber nicht beschränkt auf Polysorbate wie Tween 80. Flokkulationsinhibitoren werden bevorzugt bei der Herstellung von Kristallsuspensionen bei sehr niedrigem Wirkstoffgehalt eingesetzt.

Beispielsweise lässt sich eine 3%ige Everolimus enthaltende Suspension mit gleichmäßiger Verteilung der Kristalle ohne Flokkulationsinhibitor herstellen, ab ca. 1,5-1,0%iger Suspension (w/v) und niedriger kann der Zusatzstoff von Flokkulationsinhibitoren vorteilhaft sein, da diese die Sedimentation der Mikrokristalle zusätzlich verhindern und somit weiterhin eine gleichmäßige Beschichtung möglich ist.

Werden ein oder mehrere Flokkulationsinhibitoren der Suspension zugesetzt, muss die Menge an Zusatzstoff, die die Mikrokristalle in der Schwebe halten kann, individuell für den jeweiligen Limus-Wirkstoff ermittelt werden. Dabei liegt der Gesamtanteil des mikrokristallinen Limus-Wirkstoffs in der Suspension bevorzugt sehr niedrig zwischen 1,0 - 0,001 Gew.-% weiterhin bevorzugt sind 0,5 - 0,005 Gew.-% und besonders bevorzugt sind 0,1 - 0,01 % Gew.-%.

Es ist auch möglich, einen weiteren nicht polymeren Zusatzstoff als Matrix in die Lösung zu geben. Es eignen sich beispielsweise Kontrastmittel oder Kontrastmittelanaloga sowie biologisch verträgliche organische Stoffe, die die Beschichtungseigenschaften ebenfalls verbessern bzw. nicht negativ verändern.

In einigen Ausführungsformen kann die Suspension der vorliegenden Erfindung auch als Zusatzstoff ein oder mehrere Polymere enthalten, wie z.B. Polyvinylpyrrolidon (PVP). Als Zusatzstoffe sind Polymere geeignet, die in organischen Lösungsmitteln, insbesondere in unpolaren Lösungsmitteln gelöst werden können. Sehr hydrophile, wasserlösliche Polymere, die in organischen Lösungsmitteln kaum löslich oder praktisch unlöslich sind, sind nicht bevorzugt. Zudem muss darauf geachtet werden, dass sich die Mikrokristalle des mindestens einen Limus-Wirkstoff bei Anwesenheit eines gelösten Polymers in der Suspension nicht an- oder auflösen. Polymere für Beschichtungen von Medizinprodukten sind aus dem Stand der Technik bekannt. Der Fachmann auf dem technischen Gebiet ist somit leicht in der Lage ein geeignetes Polymer als Zusatzstoff auszuwählen.

Jedoch sind hierin polymerfreie Suspensionen zur Beschichtung von Medizinprodukten besonders bevorzugt.

Somit betrifft die vorliegende Erfindung eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-O-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet.

Als Zusatzstoffe kommen die weiter unten genannten Substanzen in Frage, bevorzugt Antioxidantien, Polyvinylpyrrolidon (PVP) und Flokkulationsinhibitoren.

Somit betrifft die vorliegende Erfindung eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen.

Somit betrifft die vorliegende Erfindung eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet, wobei der mindestens eine Limus-Wirkstoff eine Kristallinität von mindestens 90 Gew% aufweist.

Somit betrifft die vorliegende Erfindung eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet, wobei der mindestens eine Limus-Wirkstoff ausgewählt wird aus Rapamycin (Sirolimus) und Everolimus.

Somit betrifft die vorliegende Erfindung eine Suspension zur Beschichtung eines Medizinprodukts ausgewählt aus einem Katheterballon, einem Ballonkatheter, einem Stent oder einer Kanüle, die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus Rapamycin (Sirolimus) und Everolimus.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei das Lösungsmittel ein Nichtlöser mit einer Dielektrizitätskonstante εᵣ bei 20°C von ≤ 2,0 ist oder das Lösungsmittelgemisch zu mindestens 50 Vol.-% einen Nichtlöser mit einer Dielektrizitätskonstante εᵣ bei 20°C von ≤ 2,0 enthält.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei der mindestens eine Limus-Wirkstoff ausgewählt wird aus Rapamycin (Sirolimus) und Everolimus,
   wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-O-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei der mindestens eine Limus-Wirkstoff ausgewählt wird aus Rapamycin (Sirolimus) und Everolimus,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-O-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-O-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das Lösungsmittelgemisch ausgewählt wird aus Ethanol und Cyclohexan oder Essigsäureethylester und Heptan.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
      wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-O-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-O-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
   wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei das mindestens eine Tri-*O*-Acylglycerol und der mindestens eine Limus-Wirkstoff mit einem Massenanteil von 10 - 30 % Tri-*O*-Acylglycerol zu 90 - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
   wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-O-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei die Mikrokristalle des mindestens einen Limus-Wirkstoffs eine Kristallgröße im Bereich von 1 µm bis 300 µm aufweisen,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10 - 30 % Tri-*O*-Acylglycerol zu 90 - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet, wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
      wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält.

Somit betrifft die vorliegende Erfindung bevorzugt eine Suspension zur Beschichtung eines Medizinprodukts ausgewählt aus einem Katheterballon, einem Ballonkatheter, einem Stent oder einer Kanüle , die Suspension bestehend aus:
a) mindestens einem Tri-O-Acylglycerol ausgewählt aus der Gruppe bestehend aus Trioctanoylglycerol, Trinonanoylglycerol, Tridecanoylglycerol und Triundecanoylglycerol, und
b) mindestens einem Limus-Wirkstoff in Form von Mikrokristallen, wobei der Limus-Wirkstoff ausgewählt ist aus der Gruppe bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus, und
c) einem Lösungsmittel oder einem Lösungsmittelgemisch, worin sich das mindestens eine Tri-*O*-Acylglycerol löst und sich die Mikrokristalle des mindestens einen Limus-Wirkstoffs bei Anwesenheit des mindestens einen Tri-*O*-Acylglycerols nicht lösen, und
d) bis zu 5,0 Gew.% bezogen auf den Limus-Wirkstoff an Zusatzstoffen oder bis zu 15,0 Gew.% bezogen auf den Limus-Wirkstoff an Antioxidantien als Zusatzstoffe und bis zu 5,0 Gew.-% bezogen auf den Limus-Wirkstoff an Zusatzstoffen, welche keine Antioxidantien sind, wobei die Gesamtmenge an Zusatzstoffen 15,0 Gew.% bezogen auf den Limus-Wirkstoff nicht überschreitet,
   wobei der mindestens eine Limus-Wirkstoff eine Kristallinität von mindestens 90 Gew% aufweist,
   wobei der mindestens eine Limus-Wirkstoff ausgewählt wird aus Rapamycin (Sirolimus) und Everolimus,
   wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
   wobei das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{Ow} von ≥ 3,0 ist.

### Verfahren zur Herstellung der Suspension

Die vorliegende Erfindung betrifft weiterhin ein Verfahren zur Herstellung einer Suspension zur Beschichtung eines Medizinprodukts ausgewählt aus einem Katheterballon, einem Ballonkatheter, einem Stent, oder einer Kanüle, umfassend die folgenden Schritte:
a) Lösen mindestens eines Tri-*O*-Acylglycerols ausgewählt aus der Gruppe bestehend aus Trioctanoylglycerol, Trinonanoylglycerol, Tridecanoylglycerol und Triundecanoylglycerol in einem Lösungsmittel oder Lösungsmittelgemisch;
b) Zugabe von mindestens einem Limus-Wirkstoff in Form von Mikrokristallen zu der Lösung aus Schritt a) oder Zugabe der Lösung aus Schritt a) zu mindestens einem Limus-Wirkstoff in Form von Mikrokristallen,
wobei sich die Mikrokristalle des mindestens einen Limus-Wirkstoffs in der Lösung aus Schritt a) nicht lösen.

Mit anderen Worten betrifft die vorliegende Erfindung weiterhin ein Verfahren zur Herstellung einer Suspension zur Beschichtung eines Medizinprodukts ausgewählt aus einem Katheterballon, einem Ballonkatheter, einem Stent, oder einer Kanüle, umfassend die folgenden Schritte:
a) Lösen mindestens eines Tri-*O*-Acylglycerols ausgewählt aus der Gruppe bestehend aus Trioctanoylglycerol, Trinonanoylglycerol, Tridecanoylglycerol und Triundecanoylglycerol in einem Lösungsmittel oder Lösungsmittelgemisch;
b) Herstellen einer Suspension aus mindestens einem Limus-Wirkstoff in Form von Mikrokristallen und der Lösung aus Schritt a),
   wobei sich die Mikrokristalle des mindestens einen Limus-Wirkstoffs in der Lösung aus Schritt a) nicht lösen.

Bevorzugt weist der mindestens eine Limus-Wirkstoff eine Kristallinität von mindestens 90 Gew% aufweist. Bevorzugt weisen die Mikrokristalle des mindestens einen Limus-Wirkstoffs eine Kristallgröße im Bereich von 1 µm bis 300 µm aufweisen, weiter bevorzugt eine Kristallgröße von maximal 100 µm, weiter bevorzugt eine Kristallgröße im Bereich von 10 µm bis 50 µm. Bevorzugt wird der mindestens eine Limus-Wirkstoff ausgewählt aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus, und Temsirolimus. Noch bevorzugter wird der mindestens eine Limus-Wirkstoff ausgewählt aus Rapamycin (Sirolimus) und Everolimus. Insbesondere bevorzugt ist der mindestens eine Limus-Wirkstoff Everolimus. Bevorzugt ist das Lösungsmittel ein Nichtlöser mit einer Dielektrizitätskonstante εᵣ bei 20°C von ≤ 2,0 ist oder das Lösungsmittelgemisch enthält mindestens zu 50 Vol.-% einen Nichtlöser mit einer Dielektrizitätskonstante εᵣ bei 20°C von ≤ 2,0. Bevorzugt ist das Lösungsmittelgemisch ein Gemisch von mindestens einem polaren organischen Lösungsmittel mit einem n-Octanol-Wasser-Verteilungskoeffizienten logK_{Ow} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von von ≤ 3,0 und einem n-Octanol-Wasser-Verteilungskoeffizienten logK_{OW} von ≥ 3,0.

Noch anders formuliert betrifft die vorliegende Erfindung ein Verfahren zur Herstellung einer Suspension zur Beschichtung eines Medizinprodukts ausgewählt aus einem Katheterballon, einem Ballonkatheter, einem Stent, oder einer Kanüle, umfassend die folgenden Schritte:
a) Bereitstellen einer Lösung von mindestens einem Tri-O-Acylglycerol ausgewählt aus der Gruppe bestehend aus Trioctanoylglycerol, Trinonanoylglycerol, Tridecanoylglycerol und Triundecanoylglycerol in einem Lösungsmittel oder einem Lösungsmittelgemisch;
b) Bereitstellen mindestens eines Limus-Wirkstoffs in Form von Mikrokristallen,
c) Herstellen einer Suspension durch Zusammengeben der Lösung gemäß Schritt a) und dem mindestens einem Limus-Wirkstoff in Form von Mikrokristallen gemäß Schritt b),
   wobei sich die Mikrokristalle des mindestens einen Limus-Wirkstoffs gemäß Schritt b) in der Lösung gemäß Schritt a) nicht lösen.

Bevorzugt weist der mindestens eine Limus-Wirkstoff eine Kristallinität von mindestens 90 Gew% aufweist. Bevorzugt weisen die Mikrokristalle des mindestens einen Limus-Wirkstoffs eine Kristallgröße im Bereich von 1 µm bis 300 µm aufweisen, weiter bevorzugt eine Kristallgröße von maximal 100 µm, weiter bevorzugt eine Kristallgröße im Bereich von 10 µm bis 50 µm. Bevorzugt wird der mindestens eine Limus-Wirkstoff ausgewählt aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus. Noch bevorzugter wird der mindestens eine Limus-Wirkstoff ausgewählt aus Rapamycin (Sirolimus) und Everolimus. Insbesondere bevorzugt ist der mindestens eine Limus-Wirkstoff Everolimus. Bevorzugt ist das Lösungsmittel ein Nichtlöser mit einer Dielektrizitätskonstante εᵣ bei 20°C von ≤ 2,0 ist oder das Lösungsmittelgemisch enthält mindestens zu 50 Vol.-% einen Nichtlöser mit einer Dielektrizitätskonstante εᵣ bei 20°C von ≤ 2,0. Bevorzugt ist das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0.

Es hat sich als essentiell erwiesen, den mindestens einen Limus-Wirkstoff in Form von Mikrokristallen bereitzustellen und den mindestens einem Limus-Wirkstoff in Form von Mikrokristallen mit der Lösung des mindestens einen Tri-O-Acylglycerols in dem Lösungsmittel oder Lösungsmittelgemisch zusammenzugeben, damit eine stabile Kristallsuspension entsteht. Werden hingegen zuerst die Mikrokristalle des Limus-Wirkstoffs in das Lösungsmittel oder Lösungsmittelgemisch gegeben und danach erst das mindestens eine Tri-O-Acylglycerol hinzugefügt, entsteht keine erfindungsgemäße Suspension der Mikrokristalle des Limus-Wirkstoffs, woraus sich fest haftende Beschichtungen auf Medizinprodukten herstellen lassen. Die Reihenfolge der Schritte zur Herstellung der Kristallsuspension des Limus-Wirkstoffs ist daher essentiell und nicht vertauschbar. Ebenso entstehen ungeeignete Beschichtungen wenn versucht wird, aus einer Lösung des Limus-Wirkstoffs in einem Lösungsmittel oder Lösungsmittelgemisch enthaltend das mindestens eine Tri-O-Acylglycerol nach Beschichtung auf der Oberfläche des Medizinproduktes Limus-Wirkstoffkristalle zu erzeugen.

Die vorliegende Erfindung betrifft ebenfalls ein Verfahren zur Herstellung einer Suspension zur Beschichtung eines Medizinprodukts ausgewählt aus einem Katheterballon, einem Ballonkatheter, einem Stent, oder einer Kanüle, umfassend die folgenden Schritte:
a') Lösen mindestens eines Tri-*O*-Acylglycerols ausgewählt aus der Gruppe bestehend aus Trioctanoylglycerol, Trinonanoylglycerol, Tridecanoylglycerol und Triundecanoylglycerol in einem Lösungsmittel, bevorzugt in einem polaren organischen Lösungsmittel,
a") Zugabe eines unpolaren organischen Lösungsmittels, bevorzugt eines Nichtlösers, zu der Lösung aus Schritt a'); und optional Homogenisieren und Filtrieren,
b) Zugabe von mindestens einem Limus-Wirkstoff in Form von Mikrokristallen zu der Lösung aus Schritt a") oder Zugabe der Lösung aus Schritt a") zu mindestens einem Limus-Wirkstoff in Form von Mikrokristallen,
   wobei die Mikrokristalle des mindestens einen Limus-Wirkstoffs in der Lösung aus Schritt a") nicht löslich sind.

Die vorliegende Erfindung betrifft ebenfalls ein Verfahren zur Herstellung einer Suspension zur Beschichtung eines Medizinprodukts ausgewählt aus einem Katheterballon, einem Katheterballon, einem Ballonkatheter, einem Stent, oder einer Kanüle, umfassend die folgenden Schritte:
a') Lösen mindestens eines Tri-*O*-Acylglycerols ausgewählt aus der Gruppe bestehend aus Trioctanoylglycerol, Trinonanoylglycerol, Tridecanoylglycerol und Triundecanoylglycerol in einem Lösungsmittel, bevorzugt in einem polaren organischen Lösungsmittel,
a") Zugabe eines unpolaren organischen Lösungsmittels, bevorzugt eines Nichtlösers, zu der Lösung aus Schritt a'); und optional Homogenisieren und Filtrieren,
b) Bereitstellen mindestens eines Limus-Wirkstoffs in Form von Mikrokristallen,
c) Herstellen einer Suspension durch Zusammengeben der Lösung gemäß Schritt a) und dem mindestens einem Limus-Wirkstoff in Form von Mikrokristallen gemäß Schritt b),
   wobei sich die Mikrokristalle des mindestens einen Limus-Wirkstoffs gemäß Schritt b) in der Lösung gemäß Schritt a) nicht lösen.

Bevorzugt weist der mindestens eine Limus-Wirkstoff eine Kristallinität von mindestens 90 Gew% aufweist. Bevorzugt weisen die Mikrokristalle des mindestens einen Limus-Wirkstoffs eine Kristallgröße im Bereich von 1 µm bis 300 µm aufweisen, weiter bevorzugt eine Kristallgröße von maximal 100 µm, weiter bevorzugt eine Kristallgröße im Bereich von 10 µm bis 50 µm. Bevorzugt wird der mindestens eine Limus-Wirkstoff ausgewählt aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus. Noch bevorzugter wird der mindestens eine Limus-Wirkstoff ausgewählt aus Rapamycin (Sirolimus) und Everolimus. Insbesondere bevorzugt ist der mindestens eine Limus-Wirkstoff Everolimus. Bevorzugt weist der Nichtlöser eine Dielektrizitätskonstante εᵣ bei 20°C von ≤ 2,0 auf. Bevorzugt enthält die Lösung zu mindestens 50 Vol.-% Nichtlöser mit einer Dielektrizitätskonstante εᵣ bei 20°C von ≤ 2,0. Bevorzugt ist das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0.

### Beschichtungsverfahren

Die vorliegende Erfindung betrifft ebenfalls ein Verfahren zur Beschichtung eines Medizinprodukts ausgewählt aus einem Katheterballon, einem Ballonkatheter, einem Stent, oder einer Kanüle, mit einer Suspension umfassend die folgenden Schritte:
a) Bereitstellen eines Medizinprodukts mit einer Medizinprodukteoberfläche,
b) Bereitstellen einer Suspension enthaltend mindestens ein Tri-*O*-Acylglycerol ausgewählt aus der Gruppe bestehend aus Trioctanoylglycerol, Trinonanoylglycerol, Tridecanoylglycerol und Triundecanoylglycerol, mindestens einen Limus-Wirkstoff in Form von Mikrokristallen, und ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen oder bei Anwesenheit des mindestens einen Tri-O-Acylglycerols nicht lösen; und
c) Auftragen der Suspension auf die Medizinprodukteoberfläche mittels Spritzenverfahren, Pipettierverfahren, Kapillarverfahren, Faltensprühverfahren, Tauchverfahren, Sprühverfahren, Schleppverfahren, Fadenschleppverfahren, Tropfenschleppverfahren oder Rollverfahren.

Die vorliegende Erfindung betrifft ebenfalls ein Verfahren zur Beschichtung eines Medizinprodukts ausgewählt aus einem Katheterballon, einem Katheterballon, einem Ballonkatheter, einem Stent, oder einer Kanüle, mit einer Suspension umfassend die folgenden Schritte:
a) Bereitstellen eines Medizinprodukts mit einer Medizinprodukteoberfläche, optional mit vorbehandelter Oberfläche (Konditionierung der Oberfläche), wobei die Medizinprodukteoberfläche unbeschichtet oder beschichtet ist;
b) Bereitstellen einer Suspension enthaltend mindestens ein Tri-*O*-Acylglycerol ausgewählt aus der Gruppe bestehend aus Trioctanoylglycerol, Trinonanoylglycerol, Tridecanoylglycerol und Triundecanoylglycerol, mindestens einen Limus-Wirkstoff in Form von Mikrokristallen, und ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen oder bei Anwesenheit des mindestens einen Tri-O-Acylglycerols nicht lösen; und
c) Aufbringen der Suspension auf die Medizinprodukteoberfläche mittels Spritzenverfahren, Pipettierverfahren, Kapillarverfahren, Faltensprühverfahren, Tauchverfahren, Sprühverfahren, Schleppverfahren, Fadenschleppverfahren, Tropfenschleppverfahren oder Rollverfahren.

In bevorzugten Ausführungsformen weist das Verfahren nach Schritt c) noch einen Schritt d) Trocknen der Beschichtung auf.

Hierin bevorzugt werden spezielle Beschichtungsverfahren zur Beschichtung von Medizinprodukten verwendet, in welchen das Medizinprodukt mit einer definierten Menge an mikrokristallinem Limus-Wirkstoff beschichtet werden kann, wobei in diesen Beschichtungsverfahren bevorzugt eine Beschichtungsvorrichtung mit einer Volumenmesseinrichtung zur gezielten Abgabe einer definierten Menge der erfindungsgemäßen Beschichtungssuspension auf die Medizinprodukteoberfläche mittels einer Abgabevorrichtung eingesetzt wird.

Als Volumenmesseinrichtung kann jede beliebige Vorrichtung dienen, welche in der Lage ist, eine definierte Menge Beschichtungssuspension bereitzustellen oder die Menge an abgegebener Beschichtungssuspension zu messen oder anzuzeigen. Volumenmesseinrichtungen sind daher im einfachsten Fall Skalen, skalierte Pipetten, skalierte Büretten, skalierte Behälter, skalierte Kavitäten als auch Pumpen, Ventile, Spritzen oder andere kolbenförmige Behälter, welche in der Lage sind, eine definierte Menge an Beschichtungssuspension bereitzustellen oder zu befördern oder auszugeben. Somit dient die Volumenmesseinrichtung dazu, entweder eine definierte Menge einer Beschichtungssuspension bereitzustellen oder abzugeben oder eine abgegebene Menge an Beschichtungssuspension zu messen und/oder anzuzeigen. Die Volumenmesseinrichtung dient somit dazu, die von der Abgabevorrichtung auf die Medizinprodukteoberfläche übertragene Menge an Beschichtungssuspension und damit an mikrokristallinem Limus-Wirkstoff zu bestimmen bzw. zu messen.

Der wichtigste Aspekt bei der Beschichtungsvorrichtung ist jedoch die Abgabevorrichtung, welche als Düse, Vielzahl von Düsen, Faden, Netz aus Fäden, ein Stück Textil, Lederstreifen, Schwamm, Kugel, Spritze, Nadel, Kanüle oder als Kapillare ausgestaltet sein kann. Je nachdem welche Ausgestaltung die Abgabevorrichtung aufweist, ergeben sich leicht abgewandelte Beschichtungsverfahren, welche alle auf dem Grundprinzip beruhen, eine messbare bzw. definierte Menge an mikrokristallinem Limus-Wirkstoff verlustfrei auf die Medizinprodukteoberfläche zu übertragen. Auf diese Weise wird eine Beschichtung mit definierter Wirkstoffkonzentration oder Wirkstoffmenge an mikrokristallinem Limus-Wirkstoff und damit eine reproduzierbare Beschichtung bereitgestellt. Zur Unterscheidung der Verfahren werden hierin verschiedene Begriffe benutzt, nämlich Spritzenverfahren, Pipettierverfahren, Kapillarverfahren, Faltensprühverfahren, Tauchverfahren, Sprühverfahren, Schleppverfahren, Fadenschleppverfahren, Tropfenschleppverfahren oder Rollverfahren, welche die bevorzugten Ausführungsformen der vorliegenden Erfindung darstellen.

Als besonders bevorzugtes Beschichtungsverfahren von Medizinprodukten mit Kristallsuspensionen wird hierin die Tropfendosiertechnik im Mikrodosierverfahren wie das Pipettierverfahren oder Tropfenschleppverfahren verwendet. Mit diesen können besonders gleichmäßige Beschichtungen mit ebenfalls gleichmäßiger Wirkstoffkonzentration des mikrokristallinen Limus-Wirkstoffs auf der Medizinprodukteoberfläche erhalten werden, solange gewährleistet ist, dass die Mikrokristalle des Limus-Wirkstoffs gleichmäßig verteilt bleiben.

Die Untersuchungen zur Wiederfindungsrate von Wirkstoff auf in gleich großen Segmenten zerteilten Ballonkathetern bestätigt die Gleichmäßigkeit der Beschichtung und damit den Erfolg bei der Nutzung einer Kristallsuspension (siehe Beispiel 7) als auch eine 100%ige Wiederfindungsrate.

Um die gleichmäßige Verteilung der Kristalle zu gewährleisten, kann zusätzlich ein pneumatischer Schwenker die Suspension während des Beschichtungsprozesses schütteln, um eine eventuelle Sedimentation zu verhindern, was bei Kristallanteilen von unter 2% (Gew./Vol.) als Vorsichtsmaßnahme vorteilhaft sein kann.

Für eine eventuelle Vorbehandlung der Medizinprodukteoberfläche z.B. Konditionierung oder Aufbringen einer Basisschicht können dagegen auch die weiteren gängigen Beschichtungsverfahren wie Sprühen, Tauchen, Streichen, Pinseln, Pipettieren, Tropfenschleppen, Aufrollen, Spinnen, in situ-Abscheidung, Siebdrucken, Gasphasenabscheidung oder Spritzen verwendet werden. Die genannten Verfahren können auch kombiniert werden.

### Beschichtetes Medizinprodukt

Die Suspension der vorliegenden Erfindung eignet sich insbesondere für die Bereitstellung von beschichteten Medizinprodukten, die eine wirkstofffreisetzende Beschichtung umfassend mindestens ein Tri-*O*-Acylglycerol ausgewählt aus der Gruppe bestehend aus Trioctanoylglycerol, Trinonanoylglycerol, Tridecanoylglycerol und Triundecanoylglycerol, und mindestens einen Limus-Wirkstoff in Form von Mikrokristallen aufweisen.

Alle nachstehenden Ausführungsformen, die sich auf ein beschichtetes Medizinprodukt ausgewählt aus einem Katheterballon, einem Ballonkatheter, einem Stent oder einer Kanüle, beziehen, sind gemäß den beiligenden Ansprüchen so auszulegen, dass diese erhältlich sind nach einem hierin definierten Verfahren; oder mit einer hierin definierten Suspension beschichtet sind und nachfolgender Trocknung der Beschichtung.

Die vorliegende Erfindung betrifft daher ebenfalls beschichtete Medizinprodukte ausgewählt aus einem Katheterballon, einem Ballonkatheter, einem Stent oder einer Kanüle, mit einer Beschichtung umfassend mindestens ein Tri-*O*-Acylglycerol ausgewählt aus der Gruppe bestehend aus Trioctanoylglycerol, Trinonanoylglycerol, Tridecanoylglycerol und Triundecanoylglycerol, und mindestens einen Limus-Wirkstoff in Form von Mikrokristallen.

Die vorliegende Erfindung betrifft daher weiterhin beschichtete Medizinprodukte ausgewählt aus einem Katheterballon, einem Ballonkatheter, einem Stent, oder einer Kanüle, mit einer Beschichtung bestehend mindestens ein Tri-*O*-Acylglycerol ausgewählt aus der Gruppe bestehend aus Trioctanoylglycerol, Trinonanoylglycerol, Tridecanoylglycerol und Triundecanoylglycerol, und mindestens einen Limus-Wirkstoff in Form von Mikrokristallen.

Der Begriff "**Beschichtung**" soll nicht nur eine Beschichtung auf der Medizinprodukteoberfläche, sondern auch eine Befüllung oder Beschichtung von Falten, Kavitäten, Poren, Mikronadeln oder anderen befüllbaren Räumen auf oder zwischen oder in dem Material umfassen, ebenso wie bei expandierbaren, gefalteten oder eingeklappten Medizinprodukte deflatierte, partiell inflatierte und vollständig inflatierte oder aufgeklappte oder teilweise aufgeklappte Medizinprodukte betreffen.

Der Begriff "**auf die Medizinprodukteoberfläche**", wie hierin verwendet, bedeutet vorzugsweise, dass eine Auftragung unmittelbar auf die Medizinprodukteoberfläche erfolgt, d.h. unmittelbar auf das Material des Medizinprodukts. Ist das Medizinprodukt beispielsweise aus Polyamid hergestellt, so bedeutet dies, dass die Auftragung auf das Polyamid, aus dem das Medizinprodukt gefertigt ist, erfolgt. Sollte das Medizinprodukt aus beispielsweise Polyamid hergestellt sein und anschließend mit einem Polymer beschichtet werden, dann würde die Auftragung nicht auf der Medizinprodukteoberfläche erfolgen.

Bevorzugt ist, wenn die gesamte Medizinprodukteoberfläche gleichmäßig beschichtet ist. Ferner ist bevorzugt, wenn eine gleichmäßige Verteilung des mikrokristallinen Limus-Wirkstoffs auf der Medizinprodukteoberfläche vorliegt. Die Oberfläche kann aber auch nur teilweise beschichtet sein bzw. an verschiedenen Stellen unterschiedlich beschichtet sein (z.B. verschiedene Schichtdicke, verschiedene Beschichtungen, verschiedene Wirkstoffkonzentrationen, nur ausgewählte abgegrenzte Bereiche, etc.).

Der Begriff "**Medizinprodukt**", wie hierin verwendet, bezieht sich auf Gegenstände oder Stoffe, die der Erkennung, Verhütung, Überwachung, Behandlung oder Linderung von Krankheiten dienen, diesen Zweck jedoch vorwiegend ("bestimmungsgemäße Hauptwirkung") auf physikalischem Wege erreichen und nicht auf pharmakologische/immunologische Weise oder durch metabolische Wirkung. Die physikalische Wirkung der Medizinprodukte kann aber durchaus durch pharmakologische, immunologische oder metabolische Wirkungen unterstützt werden. Medizinprodukte können in langfristig einsetzbare Medizinprodukt und kurzfristig einsetzbare Medizinprodukte eingeteilt werden, je nachdem ob das Medizinprodukt kurzfristig oder langfristig Kontakt mit dem Organismus hat. Als langfristig einsetzbar werde alle Medizinprodukte verstanden, die im Körper verbleiben sollen. Weniger langfristig bis sehr kurzfristig einsetzbare Medizinprodukte sind nach einer gewissen Zeit entfernbare über einen begrenzten Zeitraum eingesetzte Medizinprodukte.

Beispiele von langfristig einsetzbaren Medizinprodukten schließen ein, sind aber nicht beschränkt auf nicht bioabbaubare, biostabile Stents, Implantate, Gelenkimplantate, Gefäßprothesen, Hirnschrittmacher (wie sie z.B. bei Parkinson eingesetzt werden), Kunstherzen, Portkatheter, Sehimplantate, Ersatz für Augenlinsen, Retina, Glaskörper, Cornea, Zahnimplantate, Cochleaimplantate, Rekonstruktionsimplantate, Schädelrekonstruktionen, Knochenersatz, Penisprothesen, Schließmuskelprothesen, und dergleichen.

Beispiele von weniger langfristig bis sehr kurzfristig einsetzbaren Medizinprodukten schließen ein, sind aber nicht beschränkt auf alle Formen und Arten von Kathetern, Ballonkatheter, Angioplastiekatheter, Blasenkatheter, Beatmungsschläuche, Venenkatheter, Kanülen aller Art, Nadeln, Flügelkanülen (Butterflies), Wirkstoffdepots, Fixierungen z.B. zur operativen Behandlung von Knochenbrüchen, künstliche Zugänge, Tubusse, Nahtmaterial, Klammern und dergleichen.

Der Begriff "Ballon" oder "**Katheterballon**" bezeichnet grundsätzlich jede expandierbare und wieder komprimierbare sowie temporär implantierbare medizinische Vorrichtung, welche in der Regel zusammen mit einem Katheter verwendet wird. Der Begriff "Katheterballon", wie hierin verwendet, bezieht sich auf den dilatierbaren Teil, d.h. den Ballon eines Ballonkatheters. Unter "**Ballonkatheter**" wird ein Dilatationsballonkatheter verstanden. Ballonkatheter ist in der Medizin eine Bezeichnung für Katheter, die mit einem Ballon versehen sind. Beispiele von Ballonkathetern schließen ein, sind aber nicht beschränkt auf Angioplastieballonkatheter, die in der perkutanen transluminalen Angioplastie zur Erweiterung und Öffnung von verengten oder verschlossenen Blutgefäßen zum Einsatz kommen, Blasenkatheter, Thrombektomiekatheter, die in der Behandlung in der Gefäßchirurgie, Neuroradiologie und der Kardiologie in der Versorgung embolisierter und sekundär thrombosierter peripherer Arterien zum Einsatz kommt, aber auch bei der Neurothrombektomie bei der Schlaganfalltherapie eingesetzt wird, Embolektomiekatheter, die in der Gefäßchirurgie zur Entfernung von frischen und weichen Emboli im peripheren Arteriensystem zum Einsatz kommen, Fogarty-Katheter, Doppelballonkatheter, Ballonkatheter, die in der Pneumologie verwendet werde, Mikro-Ballonkatheter.

In der vorliegenden Erfindung wird das Medizinprodukt ausgewählt aus der Gruppe bestehend aus einem Katheterballon, einem Ballonkatheter, einem Stent, einer Kanüle, einer Flügelkanüle (Butterfly).

In der vorliegenden Erfindung wird das Medizinprodukt ausgewählt aus der Gruppe bestehend aus einem Katheterballon, einem Ballonkatheter, einem Stent, einer Kanüle. In diesen Ausführungsformen wird das Medizinprodukt daher bevorzugt ausgewählt aus der Gruppe bestehend aus einem Katheterballon, einem Ballonkatheter, einem Stent, einem bioresorbierbaren Stent, einer Kanüle, einer Flügelkanüle (Butterfly), einer peripheren Venenverweilkanüle, einer Epiduralkanüle. Weiter bevorzugt wird das Medizinprodukt ausgewählt aus der Gruppe bestehend aus einem Katheterballon, einem Ballonkatheter, einem Angioplastiekatheter, einem Stent. Noch weiter bevorzugt wird das Medizinprodukt ausgewählt aus der Gruppe bestehend aus einem Katheterballon, einem Ballonkatheter, einem Angioplastiekatheter. Insbesondere bevorzugt ist das Medizinprodukt ein Katheterballon.

Katheterballone können unabhängig von ihrem Anwendungsbereich aus den gängigen biokompatiblen flexiblen Materialien, insbesondere Polymeren bestehen, wie sie weiter unter beschrieben werden und insbesondere aus Polyamid, wie z.B. PA 12, Polyester, Polyurethane, Polyacrylate, Polyether, Pebax, usw., aber auch aus Kombinationen geeigneter Polymere z.B. aus übereinander liegenden Schichten dieser Materialien aufgebaut sein wie aus Copolymeren dieser Materialien, Mischungen und Kombinationen der Ausführungsformen aus Schichten und Copolymeren und deren Mischungen.

Ein Implantat kann aus den gängigen biokompatiblen Materialien wie beispielsweise medizinischem Edelstahl, Titan, Chrom, Vanadium, Wolfram, Molybdän, Gold, Eisen, Nitinol, Magnesium, Eisen, Zink, Legierungen der vorgenannten Metalle, Keramiken als auch aus polymerem biostabilem oder bioresorbierbarem Material wie z.B. PTFE, Polysulfone, Polyvinylpyrrolidon, Polyamid, z.B. PA 12, Polyester, Polyurethan, Polyacrylate, Polyether, Silikon, PMMA, Kombinationen derselben, etc. bestehen. Die Materialien sind entweder bioinert, biostabil und/oder biologisch abbaubar, das Implantat expandierbar, komprimierbar oder nicht formveränderbar.

Die vorliegende Erfindung betrifft bevorzugt ein Medizinprodukt ausgewählt aus einem Katheterballon, einem Ballonkatheter, einem Stent oder einer Kanüle beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff ausgewählt wird aus Rapamycin (Sirolimus) und Everolimus.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus Rapamycin (Sirolimus) und Everolimus.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff eine Kristallinität von mindestens 90 Gew% aufweist,
wobei der mindestens eine Limus-Wirkstoff ausgewählt wird aus Rapamycin (Sirolimus) und Everolimus.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei das mindestens eine Tri-*O*-Acylglycerol und der mindestens eine Limus-Wirkstoff mit einem Massenanteil von 10 - 30% Tri-*O*-Acylglycerol zu 90 - 70% Limus-Wirkstoff vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das mindestens eine Tri-*O*-Acylglycerol und der mindestens eine Limus-Wirkstoff mit einem Massenanteil von 10% - 30% Tri-O-Acylglycerol zu 90% - 70% Limus-Wirkstoff vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus Rapamycin (Sirolimus) und Everolimus,
wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei mindestens 70% des Limus-Wirkstoffs in Form von Mikrokristallen mit einer Kristallgröße im Bereich von 10 µm bis 50 µm vorliegt.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei mindestens 70% des Limus-Wirkstoffs in Form von Mikrokristallen mit einer Kristallgröße im Bereich von 10 µm bis 50 µm vorliegt.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff ausgewählt wird aus Rapamycin (Sirolimus) und Everolimus,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus Rapamycin (Sirolimus) und Everolimus,
wobei mindestens 70% des Limus-Wirkstoffs in Form von Mikrokristallen mit einer Kristallgröße im Bereich von 10 µm bis 50 µm vorliegt.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff eine Kristallinität von mindestens 90 Gew% aufweist,
wobei der mindestens eine Limus-Wirkstoff ausgewählt wird aus Rapamycin (Sirolimus) und Everolimus,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
wobei mindestens 70% des Limus-Wirkstoffs in Form von Mikrokristallen mit einer Kristallgröße im Bereich von 10 µm bis 50 µm vorliegt.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus der Gruppe umfassend oder bestehend aus Rapamycin (Sirolimus), Everolimus, Zotarolimus, Umirolimus, Deforolimus, Myolimus, Novolimus, Pimecrolimus Ridaforolimus, Tacrolimus und Temsirolimus,
wobei das mindestens eine Tri-*O*-Acylglycerol und der mindestens eine Limus-Wirkstoff mit einem Massenanteil von 10% - 30 % Tri-*O*-Acylglycerol zu 90% - 70% Limus-Wirkstoff vorliegen,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei die Mikrokristalle des mindestens einen Limus-Wirkstoffs eine Kristallgröße im Bereich von 1 µm bis 300 µm aufweisen,
wobei der mindestens eine Limus-Wirkstoff ausgewählt wird aus Rapamycin (Sirolimus) und Everolimus,
wobei das mindestens eine Tri-*O*-Acylglycerol und der mindestens eine Limus-Wirkstoff mit einem Massenanteil von 10% - 30 % Tri-*O*-Acylglycerol zu 90% - 70% Limus-Wirkstoff vorliegen,
wobei mindestens 70% des Limus-Wirkstoffs in Form von Mikrokristallen mit einer Kristallgröße im Bereich von 10 µm bis 50 µm vorliegen.

Die vorliegende Erfindung betrifft bevorzugt ein Medizinprodukt ausgewählt aus einem Katheterballon, einem Ballonkatheter, einem Stent oder einer Kanüle, beschichtet mit mindestens einem Tri-*O*-Acylglycerol ausgewählt aus der Gruppe bestehend aus Trioctanoylglycerol, Trinonanoylglycerol, Tridecanoylglycerol und Triundecanoylglycerol, und mindestens einem Limus-Wirkstoff in Form von Mikrokristallen,
wobei der mindestens eine Limus-Wirkstoff eine Kristallinität von mindestens 90 Gew% aufweist,
wobei der mindestens eine Limus-Wirkstoff ausgewählt wird aus Rapamycin (Sirolimus) und Everolimus,
wobei das mindestens eine Tri-*O*-Acylglycerol und der mindestens eine Limus-Wirkstoff mit einem Massenanteil von 10% - 30% Tri-*O*-Acylglycerol zu 90% - 70% Limus-Wirkstoff vorliegen,
wobei mindestens 70% des Limus-Wirkstoffs in Form von Mikrokristallen mit einer Kristallgröße im Bereich von 10 µm bis 50 µm vorliegen

Zur Herstellung der Beschichtungen werden erfindungsgemäße Suspensionen verwendet, welche den Limus-Wirkstoff in Form von Mikrokristallen zusammen mit mindestens einem gelösten Tri-*O*-Acylglycerol ausgewählt aus der Gruppe bestehend aus Trioctanoylglycerol, Trinonanoylglycerol, Tridecanoylglycerol und Triundecanoylglycerol in einem Lösungsmittel bzw. Lösungsmittelgemisch enthalten.

Die vorliegende Erfindung betrifft daher ein Medizinprodukt ausgewählt aus einem Katheterballon, einem Ballonkatheter, einem Stent oder einer Kanüle, beschichtet mit einer Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol;
b) mindestens einen Limus-Wirkstoff in Form von Mikrokristallen; und
c) ein Lösungsmittel oder ein Lösungsmittelgemisch, worin sich das mindestens eine Tri-*O*-Acylglycerol löst und sich die Mikrokristalle des mindestens einen Limus-Wirkstoffs nicht lösen oder bei Anwesenheit des mindestens einen Tri-O-Acylglycerols nicht lösen.

Die vorliegende Erfindung betrifft daher ein Medizinprodukt ausgewählt aus einem Katheterballon, einem Ballonkatheter, einem Stent oder einer Kanüle, erhältlich gemäß einem Verfahren umfassend die folgenden Schritte:
a) Bereitstellen eines Medizinprodukts ausgewählt aus einem Katheterballon, einem Ballonkatheter, einem Stent oder einer Kanüle mit einer Medizinprodukteoberfläche;
b) Bereitstellen einer Suspension enthaltend ein Tri-*O*-Acylglycerol ausgewählt aus der Gruppe bestehend aus Trioctanoylglycerol, Trinonanoylglycerol, Tridecanoylglycerol und Triundecanoylglycerol gelöst in einem Lösungsmittel oder einem Lösungsmittelgemisch und mindestens einen Limus-Wirkstoff in Form von Mikrokristallen, wobei sich die Mikrokristalle des mindestens einen Limus-Wirkstoffs in dem Lösungsmittel oder dem Lösungsmittelgemisch nicht lösen oder bei Anwesenheit des mindestens einen Tri-O-Acylglycerols nicht lösen;
c) Aufbringen der Suspension auf die Medizinprodukteoberfläche mittels Spritzenverfahren, Pipettierverfahren, Kapillarverfahren, Faltensprühverfahren, Tauchverfahren, Sprühverfahren, Schleppverfahren, Fadenschleppverfahren, Tropfenschleppverfahren oder Rollverfahren,
d) Trocknen der Beschichtung.

In einigen Ausführungsformen der vorliegenden Erfindung kann ein Medizinprodukt mit einer Medizinprodukteoberfläche bereitgestellt werden, das auf der Medizinprodukteoberfläche eine Basisbeschichtung aufweist. In solchen Ausführungsformen wird die erfindungsgemäße Suspension enthaltend mindestens ein Tri-*O*-Acylglycerol ausgewählt aus der Gruppe bestehend aus Trioctanoylglycerol, Trinonanoylglycerol, Tridecanoylglycerol und Triundecanoylglycerol, und mindestens ein Limus-Wirkstoff in Form von Mikrokristallen auf diese Basisbeschichtung aufgetragen.

Die Medizinprodukteoberfläche kann beispielsweise zusätzlich mit einer hämokompatiblen, athrombogenen Schicht als Basisbeschichtung versehen sein, die durch kovalente Immobilisierung von semisynthetischen Heparinderivaten wie z.B. desulfatiertem, reacetyliertem Heparin oder Chitosanderivaten wie z.B. N-carboxymethyliertes, partiell N-acetyliertes Chitosan aufgebracht wird.

Falls erforderlich oder vorteilhaft, kann die Implantat-Oberfläche z.B. durch eine Oberflächenaktivierung z.B. über Plasmaverfahren, Temperaturbehandlung, Benetzen mit geeigneten Lösungsmitteln, DLC-Beschichtung ("diamond like carbon"), Teflonbeschichtung, oder Silikonisierung, etc. vorbehandelt werden. Es sich gezeigt, dass ein Benetzen mit einem geeigneten Lösungsmittel einen positiven Effekt auf die Haftfähigkeit hat.

Ebenso ist eine polymere Basisbeschichtung mit bioabbaubaren und/oder biostabilen Polymeren realisierbar. Diese polymeren Schichten können natürlich auch Zusatzstoffe enthalten, z.B. weitere Wirkstoffe oder Wirkstoffgemische, Metalle, Salze etc. Als Wirkstoffe bzw. Wirkstoffkombinationen eignen sich antiinflammatorische, cytostatische, cytotoxische, antiproliferative, anti-mikrotubuli, antiangiogene, antirestenotische (anti-Restenose), antifungizide, antineoplastische, antimigrative, athrombogene und antithrombogene Substanzen.

Wird der Limus-Wirkstoff in Form von Mikrokristallen nicht direkt auf oder in die Medizinprodukteoberfläche aufgebracht, können geeignete biokompatible Substanzen synthetischer, semisynthetischer und/oder nativer Herkunft biostabile oder bioabbaubare Polymere bzw. Polysaccharide als Träger bzw. als Matrix auf der Oberfläche oder als Oberfläche des Medizinproduktes verwendet werden.

Als in der Regel biologisch stabile und nur langsam biologisch abbaubare Polymere können genannt werden: Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorsilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Polyethersulfone, Epoxyharze, ABS-Harze, EPDM-Gummis, Siliconpräpolymere, Silicone wie Polysiloxane, Polyvinylhalogene und Copolymere, Celluloseether, Cellulosetriacetate, Chitosan, Chitosanderivate, polymerisierbare Öle wie z.B. Leinöl und Copolymere und/oder Mischungen derselben.

Als in der Regel biologisch abbaubare, biodegradierbare oder resorbierbare Polymere können beispielsweise verwendet werden: Polyvalerolactone, Poly-ε-Decalactone, Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherester-multiblockpolymere wie z.B. PEG und Poly(butylenterephtalat. Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethylcarbonate, Polytrimethylcarbonate, Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan], Polyhydroxypentansäure, Polyethylenoxid-propylenoxid, weiche Polyurethane, Polyurethane mit Aminosäurereste im Backbone, Polyetherester wie das Polyethylenoxid, Polyalkenoxalate, Polyorthoester sowie deren Copolymere, Carrageenane, Fibrinogen, Stärke, Kollagen, protein-basierte Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Heparansulfat, Heparin, Chondroitinsulfat, Dextran, b-Cyclodextrine, Copolymere mit PEG und Polypropylenglycol, Gummi arabicum, Guar, Gelatine, Collagen, Collagen-N-Hydroxysuccinimid, Lipide und Lipoide, polymerisierbare Öle mit geringem Vernetzungsgrad, Modifikationen und Copolymere und/oder Mischungen der vorgenannten Substanzen.

### Figurenbeschreibung

**Figur 1** a) Querschnittansicht eines umlaufend beschichteten und teilweise gefalteten Ballons; b) mikrokristalline Struktur der Everolimus-Beschichtung unter SEM bei 1000-facher Vergrößerung.
**Figur 2** zeigt mit 200-facher Vergrößerung Rapamycin in Form von Mikrokristallen in Stäbchenform mit sehr enger Partikelgrößenverteilung hauptsächlich im Bereich von 10 µm bis 30 µm.
**Figur 3** zeigt mit 1000-facher Vergrößerung Rapamycin in Form von Mikrokristallen in regelmäßiger Stäbchenform mit sehr enger Partikelgrößenverteilung hauptsächlich im Bereich von 10 µm bis 30 µm.
**Figur 4** zeigt mit 200-facher Vergrößerung Rapamycin in Form von Mikrokristallen in fast identischer Stäbchenform mit extrem enger Partikelgrößenverteilung hauptsächlich im Bereich von 15 µm bis 30 µm. Es sind keinerlei größere Kristalle oder Agglomerate zu erkennen.
**Figur 5** zeigt mit 1000-facher Vergrößerung Rapamycin in Form von Mikrokristallen in fast perfekt regelmäßiger Stäbchenform mit sehr enger Partikelgrößenverteilung hauptsächlich im Bereich von 15 µm bis 30 µm. Die Form von rhomboedrischen Prismen ist sehr anschaulich zu erkennen.
**Figur 6** zeigt mit 200-facher Vergrößerung Everolimus in Form von Mikrokristallen in Nadelform mit extrem enger Partikelgrößenverteilung hauptsächlich im Bereich von 20 µm bis 40 µm. Es sind keinerlei größere Kristalle oder Agglomerate zu erkennen.
**Figur 7** zeigt mit 1000-facher Vergrößerung Everolimus in Form von Mikrokristallen in Nadelform mit extrem enger Partikelgrößenverteilung hauptsächlich im Bereich von 20 µm bis 40 µm. Die Nadelform ist sehr anschaulich zu erkennen.
**Figur 8** zeigt mit 1000-facher Vergrößerung Rapamycin in Form von Mikrokristallen in fast identischer Stäbchenform mit extrem enger Partikelgrößenverteilung hauptsächlich im Bereich von 20 µm bis 40 µm. Es sind keinerlei größere Kristalle oder Agglomerate zu erkennen.
**Figur 9** zeigt mit 1000-facher Vergrößerung Rapamycin in Form von Mikrokristallen in fast perfekt regelmäßiger Stäbchenform mit sehr enger Partikelgrößenverteilung hauptsächlich im Bereich von 20 µm bis 40 µm. Die Form von rhomboedrischen Prismen ist sehr anschaulich zu erkennen.
**Figur 10** zeigt die mikrokristalline Struktur der Rapamycin-Beschichtung mit im Wesentlichen in rhomboedrischen Prismen vorliegenden Mikrokristallen von Rapamycin unter REM bei 1000-facher Vergrößerung.
**Figur 11** zeigt die mikrokristalline Struktur der Rapamycin-Beschichtung mit vermahlenen Mikrokristallen von Rapamycin unter REM bei 1000-facher Vergrößerung.
**Figur 12** zeigt das Modell aus Silikonschlauch geformt, dass dem natürlichen Verlauf der Gefäße im Organismus nachempfunden ist a) Simulierter Peripherer Katheter; b) Simulierte Femorale Arterie.

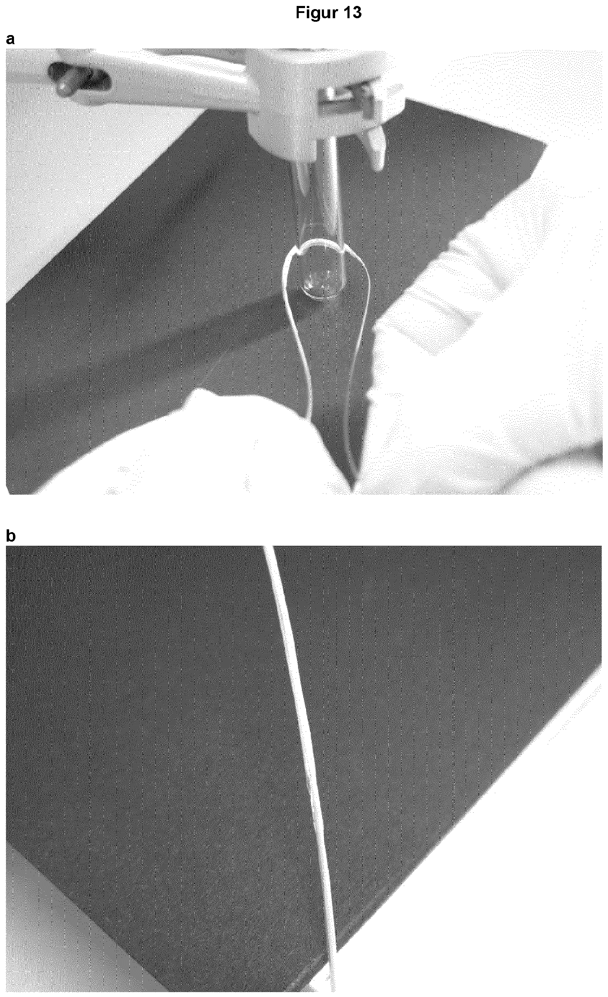

**Figur 13** a) zeigt einen Biegeversuch zur Ermittlung der Partikelfreisetzung eines beschichteten Katheterballons. b) zeigt einen Kantenschlagtest zur Ermittlung der Partikelfreisetzung eines beschichteten Katheterballons.
**Figur 14** zeigt nicht erfindungsgemäße Rapamycin-Beschichtungen hergestellt gemäß WO 2015/039969 A1 bei 1000-facher Vergrößerung. Es ist ein zu großer fast runder Kristall umgeben von vielen recht kleinen Kristallen unregelmäßiger Form und breiter Partikelgrößenverteilung zu sehen.
**Figur 15** zeigt nicht erfindungsgemäße Rapamycin-Beschichtungen hergestellt gemäß WO 2015/039969 A1 bei 1200-facher Vergrößerung. Es sind zahlreiche recht große Kristalle umgeben von vielen recht kleinen Kristallen unregelmäßiger Form und breiter Partikelgrößenverteilung zu sehen.
**Figur 16** a) zeigt nicht erfindungsgemäße Rapamycin-Kristallbeschichtung mit Trockenkristallen. b) zeigt die Beschichtung aus a) nach "Solvent-Bonding". Die Kristalle sind nicht mehr intakt.
**Figur 17** a) zeigt eine nicht erfindungsgemäße Rapamycin-Kristallbeschichtung mit Trockenkristallen und Basisbeschichtung von Kleber und Topcoat mit Trioctanoylglycerol. b) zeigt eine nicht erfindungsgemäße Rapamycin-Kristallbeschichtung mit Trockenkristallen und Basisbeschichtung und Topcoat mit Trioctanoylglycerol, c) zeigt eine Vergrößerung der Fig. b. Es ist deutlich zu erkennen, dass die Beschichtung nicht gleichmäßig ist und Bereiche aufweist, auf denen keine Mikrokristalle vorliegen.

### Beispiele

### Beispiel 1

### Herstellung von mikrokristallinem Rapamycin und mikrokristallinem Everolimus

Für die Herstellung der Kristallsuspensionen gemäß der vorliegenden Erfindung wurden zunächst Mikrokristalle von Rapamycin und Everolimus bereitgestellt. Aus dem Stand der Technik sind Kristallisationsverfahren zur Herstellung von kristallinem Sirolimus (Rapamycin) und kristallinem Everolimus bekannt. Zu aus dem Stand der Technik wohlbekannte Kristallisationsverfahren gehören:
Kristallisation durch Kühlung: Der Limus-Wirkstoff kann bei Raumtemperatur oder höherer Temperatur bis zur Sättigung in einem Lösungsmittel gelöst und bei niedrigerer Temperatur z.B. bei 0°C zur Kristallisation gebracht werden. Die Kristallgrößenverteilung kann über eine kontrollierte Abkühlungsrate beeinflusst werden. Als Lösungsmittel zur Kristallisation eignen sich für Limus-Wirkstoffe sowohl polare als auch unpolare organische Lösungsmittel, wie z.B. Toluol, Acetonitril, Ethylformiat, Isopropylacetat, Isobutylacetat, Ethanol, Dimethylformamid, Anisol, Ethylacetat, Methylethylketon, Methylisopropylketon, Tetrahydrofuran, Nitromethan, Propionitril.

Kristallisation durch Zugabe von Impfkristallen: Der Limus-Wirkstoff wird bis zur Sättigung in einem Lösungsmittel gelöst und die Kristallisation durch Zugabe von Impfkristallen initiiert, um einen kontrollierten Abbau der Übersättigung zu erzielen.

Kristallisation durch Zugabe von Antilösungsmitteln: Der Wirkstoff wird in einem Lösungsmittel gelöst und anschließend ein Nichtlöser oder Wasser hinzugegeben. Hierbei sind auch Zwei-Phasen-Gemische möglich. Als Lösungsmittel zum Lösen des Limus-Wirkstoffs können polare organische Lösungsmittel wie beispielsweise Aceton, Acetonitril, Ethylacetat, Methanol, Ethanol, Isopropylalkohol, Butanol, Butylmethylether, Tetrahydrofuran, Dimethylformamid oder Dimethylsulfoxid eingesetzt werden. Als Nichtlöser eignen sich beispielsweise Pentan, Hexan, Cyclohexan oder Heptan. Das Lösungsmittelgemisch kann zur Kristallisation stehengelassen, gerührt oder langsam i. Vak. eingeengt bzw. verdampft werden. Die Kristallgröße und Kristallinität des Wirkstoffs kann durch kontrollierte Zugabe des unpolaren Lösungsmittels beeinflusst werden. Für die Erzeugung großer Kristalle sollte die Übersättigung langsamer und zur Erzeugung kleiner Kristalle schneller erfolgen. Die Steuerung der Zugaberate des Antilösungsmittels zur Kontrolle der Kristallgröße ist allgemein bekannt.

Zur Herstellung von Mikrokristallen kann die Kristallisation auch durch Ultraschall unterstützt werden. Es ist allgemein bekannt, dass die Kristallgröße mittels Ultraschall beeinflusst werden kann. Ultraschall kann dabei zu Beginn der Kristallisation zur Initiierung der Kristallisation und Keimbildung eingesetzt werden, wobei das weitere Kristallwachstum dann ungehindert verläuft, so dass größere Kristalle heranwachsen können. Die Anwendung einer kontinuierlichen Beschallung einer übersättigten Lösung mit Ultraschall führt hingegen zu kleineren Kristallen, da hierbei viele Keime entstehen, wodurch zahlreiche kleine Kristalle heranwachsen. Eine weitere Option besteht in der Beschallung mit Ultraschall im Pulsmodus, um das Kristallwachstum so zu beeinflussen, dass maßgeschneiderte Kristallgrößen erzielt werden.

Zur Bereitstellung der gewünschten Kristallgrößen können auch noch weitere aus dem Stand der Technik bekannte Verfahren wie Mikronisierung, Vermahlung oder Siebung eingesetzt werden. Eine Möglichkeit ist die Vermahlung der Kristalle, die auch während der Kristallisation durch ein Nassvermahlen erfolgen kann. Das Vermahlen kann vorteilhaft sein, um verschiedene Kristallgrößen, d.h. eine breitere Kristallgrößenverteilung, zu erhalten. Das Vermahlen lässt alle Wunschgrößen im Kristallgrößenbereich zu. Einheitlichere Kristallgrößen können bereitgestellt werden, indem nach der Isolierung und Trocknung beispielsweise ein spezielles Siebverfahren durchgeführt wird. Hierzu kann auf aus dem Stand der Technik bekannte spezielle Siebvorrichtungen zurückgegriffen werden. Im Siebverfahren können die Limus-Wirkstoffkristalle z.B. durch einen Stapel von Sieben gesiebt und in verschiedene Größenbereiche aufgeteilt werden.

Zur Herstellung von mikrokristallinem Rapamycin und mikrokristallinem Everolimus wurden Kristallisationsverfahren mit kontrolliert ablaufenden Kristallisationen durchgeführt. Rapamycin und Everolimus konnten so direkt in Form von Mikrokristallen erhalten werden, so dass eine anschließende Vermahlung oder Mikronisierung vermieden werden kann. Die Kristallisation erfolgte durch Zugabe von Antilösungsmitteln (Ethylacetat/Heptan). Nach der Kristallisation wurden die Mikrokristalle von Rapamycin bzw. Everolimus isoliert, gewaschen (Heptan) und getrocknet. Optional wurde anschließend noch eine Trennung in verschiedene Kristallgrößen mittels Siebverfahren durchgeführt, um engere Kristallgrößenverteilungen der Mikrokristalle bereitzustellen.

Zur Bewertung der Kristallgröße, der Kristallgrößenverteilung und der Form der Kristalle eine Probe auf die Folie ein REM-Probentellers gegeben. Zur Bewertung wurden repräsentative Aufnahmen bei 200, 1000 und 3000-facher Vergrößerung gemacht, wobei die 200-fache Vergrößerung geeignet ist, um sog. Überkörner (Grobpartikel) gut zu erkennen. Die Größenabschätzung erfolgt mit Hilfe der Skalierung der REM-Aufnahmen.

Beispielaufnahmen von hierin eingesetztem Rapamycin in Form von Mikrokristallen und Everolimus in Form von Mikrokristallen sind in Fig. 2 bis Fig. 9 gezeigt. In Fig. 2 und 3 ist Rapamycin in Form von Mikrokristallen in Stäbchenform mit sehr enger Partikelgrößenverteilung hauptsächlich im Bereich von 10 µm bis 30 µm gezeigt. In Fig. 4 und 5 ist Rapamycin in Form von Mikrokristallen in fast identischer Stäbchenform mit extrem enger Partikelgrößenverteilung hauptsächlich im Bereich von 15 µm bis 30 µm gezeigt. In Fig. 7 und 8 ist Rapamycin in Form von Mikrokristallen mit einer Partikelgrößenverteilung hauptsächlich im Bereich von 20 µm bis 40 µm gezeigt. In Fig. 6 und 7 ist Everolimus in Form von Mikrokristallen in Nadelform mit einer Partikelgrößenverteilung hauptsächlich im Bereich von 20 µm bis 40 µm gezeigt. In allen Fig. 2 bis 9 ist zu erkennen, dass keinerlei größere Kristalle oder Agglomerate vorliegen. Es ist zudem deutlich zu erkennen, dass Everolimus in Form von Nadeln vorliegt, während Rapamycin in Form von rhomboedrischen Prismen vorliegt.

Die erhaltenen Mikrokristalle von Rapamycin bzw. Everolimus wurden in den folgenden Beispielen zur Herstellung von Kristallsuspensionen eingesetzt.

### Beispiel 2

### Herstellung von Kristallsuspensionen mit Tri-O-Acylglycerolen und mikrokristallinem Rapamycin (SIR) und Everolimus (EVR)

Im ersten Schritt wurden zunächst Lösungen von Tri-O-Acylglycerolen in einem Lösungsmittelgemisch hergestellt. Anschließend wurden die Lösungen mit Mikrokristallen von Rapamycin und Everolimus zusammengegeben und untersucht, mit welchen Tri-O-Acylglycerolen stabile Kristallsuspension erhalten werden können. Die Zusammensetzung der Lösungsmittel und des Lösungsmittelgemisch variiert in Abhängigkeit vom eingesetzten Wirkstoff. Die im Beispiel hergestellten Lösungen und Lösungsmittelgemische gelten für Rapamycin (SIR) und Everolimus (EVR). Für die Herstellung der Lösungen wurde hier exemplarisch ein Ethylacetat/Heptan-Lösungsmittelgemisch verwendet.

### 1. Herstellung von Lösungen mit Tri-O-Acylglycerolen

Zur Herstellung der Lösungen wurde das jeweilige Tri-*O*-Acylglycerol zunächst in einem polaren organischen Lösungsmittel gelöst und anschließend ein unpolares Lösungsmittel zugegeben. Zudem wurden noch alternativ Lösungen mit Zugabe eines Antioxidans (BHT) hergestellt.

Zur Herstellung der Lösungen wurden in 14 g Ethylacetat (15,6 mL) 770 mg des jeweiligen Tri-*O*-Acylglycerols und optional 150 mg BHT gelöst. Anschließend wurden 57,4 g n-Heptan (84,4 mL) zugegeben. Anschließend wurde homogenisiert und filtriert. Das Gesamtvolumen des Lösungsmittelgemisches ist 100 mL.
Lösung 1a)

| | |
|---|---|
| Tri-O-Acylglycerol: | Trioctanoylglycerol |
| Antioxidans: | --- |

Lösung 1b)

| | |
|---|---|
| Tri-*O*-Acylglycerol: | Trioctanoylglycerol |
| Antioxidans: | BHT |

Lösung 1c)

| | |
|---|---|
| Tri-*O*-Acylglycerol: | Tridecanoylglycerol |
| Antioxidans: | --- |

Lösung 1d)

| | |
|---|---|
| Tri-*O*-Acylglycerol: | Tridecanoylglycerol |
| Antioxidans: | BHT |

Lösung 1e)

| | |
|---|---|
| Tri-*O*-Acylglycerol: | Trihexanoylglycerol |
| Antioxidans: | --- |

Lösung 1f)

| | |
|---|---|
| Tri-*O*-Acylglycerol: | Trihexanoylglycerol |
| Antioxidans: | BHT |

Lösung 1g)

| | |
|---|---|
| Tri-*O*-Acylglycerol: | Tributanoylglycerol |
| Antioxidans: | --- |

Lösung 1h)

| | |
|---|---|
| Tri-*O*-Acylglycerol: | Tributanoylglycerol |
| Antioxidans: | BHT |

Lösung 1i)

| | |
|---|---|
| Tri-*O*-Acylglycerol: | 770mg Triacetin |
| Antioxidans: | --- |

Lösung 1j)

| | |
|---|---|
| Tri-*O*-Acylglycerol: | Triacetin |
| Antioxidans: | BHT |

Lösung 1k)

| | |
|---|---|
| Tri-*O*-Acylglycerol: | Tridodecanoylglycerol |
| Antioxidans: | --- |

Lösung 11)

| | |
|---|---|
| Tri-*O*-Acylglycerol: | Tridodecanoylglycerol |
| Antioxidans: | BHT |

Lösung 1m)

| | |
|---|---|
| Tri-*O*-Acylglycerol: | Citryl/Lactyl/Linoleyl/Oleyl-O-Glycerolen (IMWITOR^{®}) |
| Antioxidans: | --- |

Lösung 1n)

| | |
|---|---|
| Tri-*O*-Acylglycerol: | Citryl/Lactyl/Linoleyl/Oleyl-O-Glycerolen (IMWITOR^{®}) |
| Antioxidans: | BHT |

Lösung 1o)

| | |
|---|---|
| Tri-*O*-Acylglycerol: | Dioctanoylglycerol |
| Antioxidans: | --- |

Lösung 1p)

| | |
|---|---|
| Tri-*O*-Acylglycerol: | Dioctanoylglycerol |
| Antioxidans: | BHT |

Lösung 1q)

| | |
|---|---|
| Tri-*O*-Acylglycerol: | Monooctanoylglycerol |
| Antioxidans: | --- |

Lösung 1r)

| | |
|---|---|
| Tri-*O*-Acylglycerol: | Monooctanoylglycerol |
| Antioxidans: | BHT |

Lösung 1s)

| | |
|---|---|
| Tri-*O*-Acylglycerol: | Tritetradecanoylglycerol |
| Antioxidans: | --- |

Lösung 1t)

| | |
|---|---|
| Tri-*O*-Acylglycerol: | Tritetradecanoylglycerol |
| Antioxidans: | BHT |

### 2. Redispergierung von Mikrokristallen von Rapamycin bzw. Everolimus

Zu einer genau abgewogenen Menge an im Vorfeld hergestellten, trockenen Mikrokristallen des Limus-Wirkstoffs wird vorsichtig eine definierte Menge der Lösungen enthaltend Tri-O-Acylglycerol und optional Antioxidans gegeben. Es wurde untersucht, ob die Mikrokristalle des Limus-Wirkstoffs in den Lösungen unlöslich sind und ob sich Suspensionen ergeben.

Zur Überprüfung ob sich Kristallsuspension herstellen lassen, wurden zu jeweils 200 mg Rapamycin in Form von Mikrokristallen bzw. 200 mg Everolimus in Form von Mikrokristallen vorsichtig 10 mL einer der Lösungen 1a) bis 1t) bei Raumtemperatur gegeben. Es wurden jeweils 3 Ansätze mit je 10 mL Lösung für jede Lösung hergestellt. Nach Zusammenführung wurde getestet, ob sich die Mikrokristalle der Limus-Wirkstoffe in den Lösungen direkt lösen. Für Lösungen, bei denen kein sofortiges Lösen der Mikrokristalle der Limus-Wirkstoffe auftrat, wurden die Suspensionen der Lösungen ohne Antioxidans über einen Zeitraum von 100 h stehen gelassen und erneut geprüft, ob sich die Mikrokristalle der Limus-Wirkstoffe gelöst haben. Für die Suspensionen der Lösungen mit Antioxidans wurde das Gemisch auf 50°C erhitzt, um zu überprüfen, ob die Suspensionen auch unter Sterilisationsbedingungen stabil bleiben.

**Tabelle 9: Übersicht Ergebnisse zur Herstellung von Kristallsuspensionen mit Lösungen mit verschiedenen Tri-O-Acylglycerolen (+++++ = stabile Kristallsuspension, gleichmäßige Verteilung der Mikrokristalle, intakte Mikrokristalle, "schweben" der Mikrokristalle; +++ = Suspension, kein vollständiges Auflösen der Mikrokristalle; + = Sedimentation oder teilweises Auflösen der Mikrokristalle, keine intakten Kristalle; --- = keine Suspension; -- = keine Suspension; - = keine Suspension; -/- = nicht untersucht)**

| Lösung (10 mL) | Wirkstoff (000 mg) | Suspension | Suspension ohne BHT (100 h) | Suspension mit BHT (50°C) |
|---|---|---|---|---|
| 1a) | Rapamycin | +++++ | +++++ | +++++ |
| 1b) | Rapamycin | +++++ | +++++ | +++++ |
| 1c) | Rapamycin | +++++ | +++++ | +++++ |
| 1d) | Rapamycin | +++++ | +++++ | +++++ |
| 1e) | Rapamycin | + | - | - |
| 1f) | Rapamycin | + | --- | - |
| 1g) | Rapamycin | - | -/- | -/- |
| 1h) | Rapamycin | - | -/- | -/- |
| 1i) | Rapamycin | - | - | -/- |
| 1j) | Rapamycin | - | -/- | -/- |
| 1k) | Rapamycin | +++ | + | + |
| 1l) | Rapamycin | +++ | + | + |
| 1m) | Rapamycin | - | -/- | -/- |
| 1n) | Rapamycin | - | -/- | -/- |
| 1o) | Rapamycin | - | -/- | -/- |
| 1p) | Rapamycin | - | -/- | -/- |
| 1q) | Rapamycin | - | -/- | -/- |
| 1r) | Rapamycin | - | -/- | -/- |
| 1s) | Rapamycin | +++ | + | + |
| 1t) | Rapamycin | +++ | + | + |
| 1a) | Everolimus | +++++ | +++++ | +++++ |
| 1b) | Everolimus | +++++ | +++++ | +++++ |
| 1c) | Everolimus | +++++ | +++++ | +++++ |
| 1d) | Everolimus | +++++ | +++++ | +++++ |
| 1e) | Everolimus | + | --- | - |
| 1f) | Everolimus | + | - | - |
| 1g) | Everolimus | - | -/- | -/- |
| 1h) | Everolimus | - | -/- | -/- |
| 1i) | Everolimus | - | -/- | -/- |
| 1j) | Everolimus | - | -/- | -/- |
| 1k) | Everolimus | +++ | + | + |
| 1l) | Everolimus | +++ | + | + |
| 1m) | Everolimus | - | -/- | -/- |
| 1n) | Everolimus | - | -/- | -/- |
| 1o) | Everolimus | - | -/- | -/- |
| 1p) | Everolimus | - | -/- | -/- |
| 1q) | Everolimus | - | -/- | -/- |
| 1r) | Everolimus | - | -/- | -/- |
| 1s) | Everolimus | +++ | + | + |
| 1t) | Everolimus | +++ | + | + |

### Ergebnis:

Mit den Tri-*O*-Acylglycerolen Trioctanoylglycerol und Tridecanoylglycerol konnten stabile Kristallsuspensionen hergestellt werden, die auch noch nach 100 Stunden und unter Temperaturerhöhung stabil blieben. Die Anwesenheit des Antioxidans hatte keinen Einfluss auf die Stabilität der Kristallsuspension. Es wurde mit und ohne Anwesenheit von BHT eine stabile Kristallsuspension mit den Tri-*O*-Acylglycerolen Trioctanoylglycerol und Tridecanoylglycerol erhalten. Bei den Lösungen mit Trioctanoylglycerol und Tridecanoylglycerol trat keine Sedimentation der Mikrokristalle auf, die Mikrokristalle "schweben" in der Kristallsuspension und sind gleichmäßig verteilt.

Zur Bewertung der Kristallgröße, der Partikelgrößenverteilung (d.h. der Kristallgrößenverteilung) und der Form der Kristalle wurde jeweils mit einer Pasteur-Pipette eine Probe entnommen und ein Tropfen auf die Folie des REM-Probentellers gegeben. Zur Bewertung wurden REM-Aufnahmen bei 200 und 1000-facher Vergrößerung gemacht.

Die REM-Aufnahmen zeigten, dass die Mikrokristalle der Kristallsuspensionen mit den Tri-*O*-Acylglycerolen Trioctanoylglycerol und Tridecanoylglycerol intakt blieben. Die Mikrokristalle von Everolimus lagen durchgehend in Form von Nadeln vor, während die Mikrokristalle von Rapamycin weiterhin in Form von rhomboedrischen Prismen vorlagen. Auch die Kristallgrößenverteilung entsprach weiterhin der Kristallgrößenverteilung des ursprünglich eingesetzten mikrokristallinen Everolimus bzw. Rapamycin. Somit trat in diesen Kristallsuspension kein Kristallwachstum und kein Zusammenlagern der Mikrokristalle ein.

Bei den Lösungen mit Trihexanoylglycerol lösten sich die Mikrokristalle von Everolimus und Rapamycin nicht direkt auf. In diesen Suspensionen lagen die Mikrokristalle im Gegensatz zu den Kristallsuspensionen mit Trioctanoylglycerol und Tridecanoylglycerol nicht so gleichmäßig vor. Im Fall von Trihexanoylglycerol waren die Mikrokristalle von Everolimus und Rapamycin nach 100 Stunden fast vollständig gelöst, und unter Erhöhung der Temperatur lösten sich die Mikrokristalle von Everolimus und Rapamycin rasch auf.

Bei den Lösungen mit Tridodecanoylglycerol und Tritetradecanoylglycerol lösten sich die Mikrokristalle von Everolimus und Rapamycin nicht direkt auf. Jedoch zeigte sich auch hier, dass diese "Suspensionen" instabil sind. Im Fall von Tridodecanoylglycerol und Tritetradecanoylglycerol lösten sich die Mikrokristalle von Everolimus und Rapamycin nach 100 Stunden nicht vollständig auf. Jedoch lagen die Mikrokristalle im Gegensatz zu den Kristallsuspensionen mit Trioctanoylglycerol und Tridecanoylglycerol nicht so gleichmäßig verteilt vor und es trat eine Sedimentation der Kristalle auf. Eine Erhöhung der Temperatur beschleunigte diesen Prozess.

Zur Bewertung der Kristallgröße, der Partikelgrößenverteilung (d.h. der Kristallgrößenverteilung) und der Form der Kristalle wurde jeweils mit einer Pasteur-Pipette eine Probe entnommen und ein Tropfen auf die Folie des REM-Probentellers gegeben. Es wurde eine zusätzliche Probe vom Bodensatz entnommen und ein Tropfen auf die Folie des REM-Probentellers gegeben. Zur Bewertung wurden REM-Aufnahmen bei 200 und 1000-facher Vergrößerung gemacht.

Die REM-Aufnahmen zeigten, dass die Mikrokristalle der Kristallsuspensionen mit Tridodecanoylglycerol und Tritetradecanoylglycerol nicht intakt blieben. Die Kristallgrößenverteilung entsprach nicht mehr der Kristallgrößenverteilung des ursprünglich eingesetzten mikrokristallinen Everolimus bzw. Rapamycin, es wurden größere Kristalle detektiert, insbesondere in der Probe, die vom Bodensatz entnommen wurde.

Mit den weiteren Lösungen von Tri-*O*-Acylglycerolen konnten keine Kristallsuspensionen hergestellt werden. Somit konnten nur mit Trioctanoylglycerol und Tridecanoylglycerol stabile Kristallsuspensionen hergestellt werden.

### Beispiel 3

### Herstellung von Kristallsuspensionen mit weiteren Tri-O-Acylglycerolen und mikrokristallinem Rapamycin (SIR) und Everolimus (EVR)

Auf Basis der Ergebnisse aus Beispiel 2 wurden noch weitere Lösungen von den drei Tri-*O*-Acylglycerolen Triheptanoylglycerol, Trinonanoylglycerol und Triundecanoylglycerol hergestellt, um zu untersuchen ob mit diesen stabile Kristallsuspension erhalten werden können. Für die Herstellung der Lösungen wurde hier ebenfalls ein Ethylacetat/Heptan-Lösungsmittelgemisch verwendet.

### 1. Herstellung von Lösungen mit Tri-O-Acylglycerolen

Zur Herstellung der Lösungen wurden in 14 g Ethylacetat (15,6 mL) 770 mg des jeweiligen Tri-*O*-Acylglycerols und optional 150 mg BHT gelöst. Anschließend wurden 57,4 g n-Heptan (84,4 mL) zugegeben. Anschließend wurde homogenisiert und filtriert. Das Gesamtvolumen des Lösungsmittelgemisches ist 100 mL.
Lösung 2a)

| | |
|---|---|
| Tri-*O*-Acylglycerol: | Triheptanoylglycerol |
| Antioxidans: | --- |

Lösung 2b)

| | |
|---|---|
| Tri-*O*-Acylglycerol: | Triheptanoylglycerol |
| Antioxidans: | BHT |

Lösung 2c)

| | |
|---|---|
| Tri-*O*-Acylglycerol: | Trinonanoylglycerol |
| Antioxidans: | --- |

Lösung 2d)

| | |
|---|---|
| Tri-*O*-Acylglycerol: | Trinonanoylglycerol |
| Antioxidans: | BHT |

Lösung 2e)

| | |
|---|---|
| Tri-*O*-Acylglycerol: | Triundecanoylglycerol |
| Antioxidans: | --- |

Lösung 2f)

| | |
|---|---|
| Tri-*O*-Acylglycerol: | Triundecanoylglycerol |
| Antioxidans: | BHT |

### 2. Redispergierung von Mikrokristallen von Rapamycin bzw. Everolimus

Zur Herstellung von Kristallsuspension wurden wie in Beispiel 2 zu jeweils 200 mg Rapamycin in Form von Mikrokristallen bzw. 200 mg Everolimus in Form von Mikrokristallen vorsichtig 10 mL einer der Lösungen 2a) bis 2f) bei Raumtemperatur gegeben. Es wurden jeweils 3 Ansätze mit je 10 mL Lösung für jede Lösung hergestellt. Nach Zusammenführung wurde getestet, ob sich die Mikrokristalle der Limus-Wirkstoffe in diesen Lösungen direkt lösen. Für Lösungen, bei denen kein sofortiges Lösen der Mikrokristalle der Limus-Wirkstoffe auftrat, wurden die Suspensionen der Lösungen ohne Antioxidans über einen Zeitraum von 100 Stunden stehen gelassen und erneut geprüft, ob sich die Mikrokristalle der Limus-Wirkstoffe gelöst haben. Für die Suspensionen der Lösungen mit Antioxidans wurde das Gemisch auf 50°C erhitzt, um zu überprüfen, ob die Suspensionen auch unter Sterilisationsbedingungen stabil bleiben.

Zum direkten Vergleich sind die Ergebnisse aus Beispiel 2 für die Lösungen 1a) bis 1d) in der folgenden Tabelle mit aufgeführt.

**Tabelle 10: Übersicht Ergebnisse zur Herstellung von Kristallsuspensionen mit Lösungen mit verschiedenen Tri-O-Acylglycerolen (+++++ = exzellent stabile Kristallsuspension, gleichmäßige Verteilung der Mikrokristalle, intakte Mikrokristalle, "schweben" der Mikrokristalle; ++++ = stabile Kristallsuspension, gleichmäßige Verteilung der Mikrokristalle, intakte Mikrokristalle, "schweben" der Mikrokristalle; +++ = Kristallsuspension,; ++ = Suspension, kein vollständiges Auflösen der Mikrokristalle; + = Sedimentation oder teilweises Auflösen der Mikrokristalle, keine intakten Kristalle)**

| Lösung (10 mL) | Wirkstoff (200 mg) | Suspension | Suspension ohne BHT (100 h) | Suspension mit BHT (50°C) |
|---|---|---|---|---|
| 1a) | Rapamycin | +++++ | +++++ | +++++ |
| 1b) | Rapamycin | +++++ | +++++ | +++++ |
| 1c) | Rapamycin | +++++ | +++++ | +++++ |
| 1d) | Rapamycin | +++++ | +++++ | +++++ |
| 2a) | Rapamycin | +++ | + | - |
| 2b) | Rapamycin | +++ | + | - |
| 2c) | Rapamycin | +++++ | ++++ | ++++ |
| 2d) | Rapamycin | +++++ | ++++ | ++++ |
| 2e) | Rapamycin | +++++ | ++++ | ++++ |
| 2f) | Rapamycin | +++++ | ++++ | ++++ |
| 1a) | Everolimus | +++++ | +++++ | +++++ |
| 1b) | Everolimus | +++++ | +++++ | +++++ |
| 1c) | Everolimus | +++++ | +++++ | +++++ |
| 1d) | Everolimus | +++++ | +++++ | +++++ |
| 2a) | Everolimus | +++ | + | - |
| 2b) | Everolimus | +++ | + | - |
| 2c) | Everolimus | +++++ | ++++ | ++++ |
| 2d) | Everolimus | +++++ | ++++ | ++++ |
| 2e) | Everolimus | +++++ | ++++ | ++++ |
| 2f) | Everolimus | +++++ | ++++ | ++++ |

### Ergebnis:

Mit den Tri-*O*-Acylglycerolen Trinonanoylglycerol und Triundecanoylglycerol konnten stabile Kristallsuspensionen hergestellt werden, die auch noch nach 100 Stunden und unter Temperaturerhöhung stabil blieben. Die Anwesenheit des Antioxidans hatte keinen Einfluss auf die Stabilität der Kristallsuspension. Bei den Lösungen mit Trinonanoylglycerol und Triundecanoylglycerol trat keine Sedimentation der Mikrokristalle auf, die Mikrokristalle "schweben" in der Kristallsuspension und sind gleichmäßig verteilt.

Zur Bewertung der Kristallgröße, der Partikelgrößenverteilung (d.h. der Kristallgrößenverteilung) und der Form der Kristalle wurde jeweils mit einer Pasteur-Pipette eine Probe entnommen und ein Tropfen auf die Folie des REM-Probentellers gegeben. Zur Bewertung wurden REM-Aufnahmen bei 200 und 1000-facher Vergrößerung gemacht.

Die REM-Aufnahmen zeigten, dass die Mikrokristalle der Kristallsuspensionen mit den Tri-*O*-Acylglycerolen Trinonanoylglycerol und Triundecanoylglycerol intakt blieben. Die Mikrokristalle von Everolimus lagen durchgehend in Form von Nadeln vor, während die Mikrokristalle von Rapamycin weiterhin in Form von rhomboedrischen Prismen vorlagen. Auch die Kristallgrößenverteilung entsprach weiterhin der Kristallgrößenverteilung des ursprünglich eingesetzten mikrokristallinen Everolimus bzw. Rapamycin.

Bei den Lösungen mit Triheptanoylglycerol lösten sich die Mikrokristalle von Everolimus und Rapamycin nicht direkt auf. Jedoch waren die Mikrokristalle von Everolimus und Rapamycin nach 100 Stunden zu einem Teil gelöst, und unter Erhöhung der Temperatur lösten sich die Mikrokristalle von Everolimus und Rapamycin auf.

Zur Bewertung der Kristallgröße, der Partikelgrößenverteilung (d.h. der Kristallgrößenverteilung) und der Form der Kristalle wurde jeweils mit einer Pasteur-Pipette eine Probe entnommen und ein Tropfen auf die Folie des REM-Probentellers gegeben. Zur Bewertung wurden REM-Aufnahmen bei 200 und 1000-facher Vergrößerung gemacht.

Die REM-Aufnahmen zeigten, dass die Mikrokristalle der Kristallsuspensionen mit den Triheptanoylglycerol nicht intakt blieben. Die Kristallgrößenverteilung entsprach nicht mehr der Kristallgrößenverteilung des ursprünglich eingesetzten mikrokristallinen Everolimus bzw. Rapamycin.

Mit den weiteren Tri-*O*-Acylglycerolen Trinonanoylglycerol und Triundecanoylglycerol konnten somit stabile Kristallsuspensionen hergestellt werden.

### Beispiel 4

### Herstellung von 3%igen und 1%igen-Kristallsuspensionen enthaltend Trioctanoylglycerol und mikrokristallines Everolimus (EVR)

### I. Herstellung der Lösungen von Trioctanoylglycerol

Ia)
   Lösungsmittelgemischbeispiel für einen Ansatz von 100 ml mit 3% EVR Kristallanteil. In 14 g Ethylacetat werden 770 mg Trioctanoylglycerol gelöst. Zu dieser Lösung werden 57,4 g n-Heptan hinzugegeben, homogenisiert und filtriert.
Ib) Lösungsmittelgemischbeispiel für einen Ansatz von 100 ml mit 3% EVR Kristallanteil und BHT. In 14 g Ethylacetat werden 770 mg Trioctanoylglycerol und 150 mg BHT gelöst. Zu dieser Lösung werden 57,4 g n-Heptan hinzugegeben, homogenisiert und filtriert.
Ic) Lösungsgemischbeispiel für einen Ansatz von 100 ml mit 1% EVR Kristallanteil. In 14 g Ethylacetat werden 250 mg Trioctanoylglycerol, und 20mg Tween 80 gelöst. Zu dieser Lösung werden 57,4 g n-Heptan hinzugegeben, homogenisiert und filtriert.
Id) Lösungsgemischbeispiel für einen Ansatz von 100 ml 1% EVR Kristallanteil. In 14 g Ethylacetat werden 250mg Trioctanoylglycerol, 50 mg BHT und 20mg Tween 80 gelöst. Zu dieser Lösung werden 57,4 g n-Heptan hinzugegeben, homogenisiert und filtriert.

### II. Herstellung der Kristallsuspension

Zu einer genau abgewogenen Menge an im Vorfeld hergestellten, trockenen Wirkstoffkristallen wird vorsichtig eine definierte Menge des Lösungsmittelgemisches gegeben. Die in dem Lösungsmittelgemisch unlöslichen Kristalle bilden mit dem Lösungsmittelgemisch eine Suspension. Für die Lösungen Ia) und Ib) wurden 3 g Everolimus in Form von Mikrokristallen eingesetzt und für die Lösungen Ic) und Id) wurden 1 g Everolimus in Form von Mikrokristallen eingesetzt.

### Beispiel 5

### Herstellung von Kristallsuspensionen mit Mikrokristallen von Everolimus (EVR) und Rapamycin mit verschiedenen Anteilen von-Tri-O-Acylglycerol

In den Beispielen 2 und 3 wurde gezeigt, dass bei den Tri-*O*-Acylglycerolen Trioctanoylglycerol, Tridecanoylglycerol, Trinonanoylglycerol und Triundecanoylglycerol, bei einem Massenverhältnis von Tri-*O*-Acylglycerolen zu Mikrokristallen des Limus-Wirkstoffs von 20:80 stabile Kristallsuspensionen erhalten werden konnten.

Es wurden hierzu weitere Untersuchung mit Lösungen von Trioctanoylglycerol bzw. Tridecanoylglycerol mit verschiedenen Anteilen an Tri-O-Acylglycerol durchgeführt, um das optimale Massenverhältnis von Tri-*O*-Acylglycerol zu mikrokristallinem Limus-Wirkstoff herauszufinden. Zur Herstellung der Lösungen wurde in 14 g Ethylacetat (15,6 mL) die entsprechende Menge des jeweiligen Tri-*O*-Acylglycerols gelöst. Anschließend wurden 57,4 g n-Heptan (84,4 mL) zugegeben. Anschließend wurde homogenisiert und filtriert. Das Gesamtvolumen des Lösungsmittelgemisches ist 100 mL.
Lösung 3a)

| | |
|---|---|
| Tri-*O*-Acylglycerol: | Trioctanoylglycerol |
| Einwaage: | 300 mg |

Lösung 3b)

| | |
|---|---|
| Tri-*O*-Acylglycerol: | Trioctanoylglycerol |
| Einwaage: | 450 mg |

Lösung 3c)

| | |
|---|---|
| Tri-*O*-Acylglycerol: | Trioctanoylglycerol |
| Einwaage: | 600 mg |

Lösung 3d)

| | |
|---|---|
| Tri-*O*-Acylglycerol: | Trioctanoylglycerol |
| Antioxidans: | 900 mg |

Lösung 3e)

| | |
|---|---|
| Tri-*O*-Acylglycerol: | Trioctanoylglycerol |
| Antioxidans: | 1200 mg |

Lösung 3f)

| | |
|---|---|
| Tri-*O*-Acylglycerol: | Trioctanoylglycerol |
| Antioxidans: | 1500 mg |

Lösung 4a)

| | |
|---|---|
| Tri-*O*-Acylglycerol: | Tridecanoylglycerol |
| Einwaage: | 300 mg |

Lösung 4b)

| | |
|---|---|
| Tri-*O*-Acylglycerol: | Tridecanoylglycerol |
| Einwaage: | 450 mg |

Lösung 4c)

| | |
|---|---|
| Tri-*O*-Acylglycerol: | Tridecanoylglycerol |
| Einwaage: | 600 mg |

Lösung 4d)

| | |
|---|---|
| Tri-*O*-Acylglycerol: | Tridecanoylglycerol |
| Antioxidans: | 900 mg |

Lösung 4e)

| | |
|---|---|
| Tri-*O*-Acylglycerol: | Tridecanoylglycerol |
| Antioxidans: | 1200 mg |

Lösung 4f)

| | |
|---|---|
| Tri-*O*-Acylglycerol: | Tridecanoylglycerol |
| Antioxidans: | 1500 mg |

**Tabelle 11: Übersicht Ergebnisse zur Herstellung von Kristallsuspensionen mit Lösungen mit verschiedenen Anteilen an Tri-O-Acylglycerol (+++++ = exzellent stabile Kristallsuspension, gleichmäßige Verteilung der Mikrokristalle, intakte Mikrokristalle, "schweben" der Mikrokristalle; ++++ = stabile Kristallsuspension, gleichmäßige Verteilung der Mikrokristalle, intakte Mikrokristalle, "schweben" der Mikrokristalle; +++ = Kristallsuspension; ++ = weniger stabile Kristallsuspension; + = weniger stabile Kristallsuspension, zu viskos)**

| Lösung (10 mL) | Wirkstoff (300 mg) | Suspension (100 h) |
|---|---|---|
| 1a) | Rapamycin | +++++ |
| 3a) | Rapamycin | +++ |
| 3b) | Rapamycin | ++++ |
| 3c) | Rapamycin | +++++ |
| 3d) | Rapamycin | ++++ |
| 3e) | Rapamycin | +++ |
| 3f) | Rapamycin | ++ |
| 1c) | Rapamycin | +++++ |
| 4a) | Rapamycin | +++ |
| 4b) | Rapamycin | ++++ |
| 4c) | Rapamycin | +++++ |
| 4d) | Rapamycin | ++++ |
| 4e) | Rapamycin | +++ |
| 4f) | Rapamycin | ++ |
| 1a) | Everolimus | +++++ |
| 3a) | Everolimus | +++ |
| 3b) | Everolimus | ++++ |
| 3c) | Everolimus | +++++ |
| 3d) | Everolimus | ++++ |
| 3e) | Everolimus | +++ |
| 3f) | Everolimus | ++ |
| 1c) | Everolimus | +++++ |
| 4a) | Everolimus | +++ |
| 4b) | Everolimus | ++++ |
| 4c) | Everolimus | +++++ |
| 4d) | Everolimus | ++++ |
| 4e) | Everolimus | +++ |
| 4f) | Everolimus | ++ |

### Ergebnis:

Mit verschiedenen Anteilen der Tri-*O*-Acylglycerolen Trioctanoylglycerol und Tridecanoylglycerol konnten mit mikrokristallinem Everolimus und mikrokristallinem Rapamycin stabile Kristallsuspensionen hergestellt werden. Bei den Lösungen mit verschiedenen Anteilen an Trioctanoylglycerol und Tridecanoylglycerol war insbesondere die Kristallsuspension mit einem Verhältnis von 20:80 nach 100 Stunden noch exzellent stabil.

### Beispiel 6

### Herstellung von Kristallsuspensionen mit mikrokristallinem Rapamycin (SIR) und Everolimus (EVR) in verschiedenen Lösungsmittelgemischen

Auf Basis der Ergebnisse der vorhergehenden Beispiele wurden verschiedene Lösungsmittelgemische für die Herstellung von Kristallsuspensionen von mikrokristallinem Rapamycin und Everolimus getestet. Das in den vorhergehenden Beispielen eingesetzte Lösungsmittelgemisch von Ethylacetat/Heptan weist ein Verhältnis von ca. 85:15 (Heptan : Ethylacetat) auf.

Zur Untersuchung von weiteren Lösungsmittelgemischen für die Herstellung der Kristallsuspensionen wurden Lösungsmittelgemische der polaren organischen Lösungsmittel Aceton, Ethanol, iso-Propanol und Ethylacetat und der unpolaren organischen Lösungsmittel Hexan, Heptan und Cyclohexan in verschiedenen Anteilen hergestellt.

Zur Herstellung der Lösungen wurden 770 mg von Trioctanoylglycerol im polaren Lösungsmittel gelöst. Anschließend wurde das unpolare Lösungsmittel zugegeben, homogenisiert und filtriert. Das Gesamtvolumen des Lösungsmittelgemisches ist jeweils 100 mL.

Zur Überprüfung, ob sich Kristallsuspensionen herstellen lassen, wurden zu jeweils 200 mg Everolimus in Form von Mikrokristallen vorsichtig 10 mL einer der Lösungen bei Raumtemperatur gegeben. Nach Zusammenführung wurde getestet, ob stabile Kristallsuspensionen erhalten werden.

**Tabelle 12: Übersicht Ergebnisse zur Herstellung von Kristallsuspensionen verschiedener Lösungsmittelgemische (+++ = gut; +/- = mittel,-= schlecht)**

| Polares organisches LM | Unpolares organisches LM | Verhältnis polares/unpolares LM (Vol./Vol.) | Kristallsuspension |
|---|---|---|---|
| Ethylacetat | Heptan | 10:90 | +++ |
| Ethylacetat | Heptan | 30:70 | +++ |
| Ethylacetat | Heptan | 40:60 | +++ |
| Ethylacetat | Heptan | 50:50 | +/- |
| Ethylacetat | Heptan | 60:40 | +/- |
| Ethylacetat | Hexan | 10:90 | +++ |
| Ethylacetat | Hexan | 30:70 | +++ |
| Ethylacetat | Hexan | 40:60 | +++ |
| Ethylacetat | Hexan | 50:50 | +/- |
| Ethylacetat | Hexan | 60:40 | +/- |
| Ethylacetat | Cyclohexan | 10:90 | +++ |
| Ethylacetat | Cyclohexan | 30:70 | +++ |
| Ethylacetat | Cyclohexan | 40:60 | +++ |
| Ethylacetat | Cyclohexan | 50:50 | +/- |
| Ethylacetat | Cyclohexan | 60:40 | +/- |
| Ethanol | Heptan | 10:90 | +++ |
| Ethanol | Heptan | 30:70 | ++ |
| Ethanol | Heptan | 40:60 | +/- |
| Ethanol | Heptan | 50:50 | - |
| Ethanol | Heptan | 60:40 | - |
| Ethanol | Hexan | 10:90 | +++ |
| Ethanol | Hexan | 30:70 | ++ |
| Ethanol | Hexan | 40:60 | +/- |
| Ethanol | Hexan | 50:50 | - |
| Ethanol | Hexan | 60:40 | - |
| Ethanol | Cyclohexan | 10:90 | +++ |
| Ethanol | Cyclohexan | 30:70 | ++ |
| Ethanol | Cyclohexan | 40:60 | +/- |
| Ethanol | Cyclohexan | 50:50 | - |
| Ethanol | Cyclohexan | 60:40 | - |
| Aceton | Heptan | 10:90 | +++ |
| Aceton | Heptan | 30:70 | +/- |
| Aceton | Heptan | 40:60 | +/- |
| Aceton | Heptan | 50:50 | --- |
| Aceton | Heptan | 60:40 | - |
| Aceton | Hexan | 10:90 | +++ |
| Aceton | Hexan | 30:70 | +/- |
| Aceton | Hexan | 40:60 | +/- |
| Aceton | Hexan | 50:50 | - |
| Aceton | Hexan | 60:40 | - |
| Aceton | Cyclohexan | 10:90 | +++ |
| Aceton | Cyclohexan | 30:70 | +/- |
| Aceton | Cyclohexan | 40:60 | +/- |
| Aceton | Cyclohexan | 50:50 | - |
| Aceton | Cyclohexan | 60:40 | - |
| iso-Propanol | Heptan | 10:90 | +++ |
| iso-Propanol | Heptan | 30:70 | +/- |
| iso-Propanol | Heptan | 40:60 | +/- |
| iso-Propanol | Heptan | 50:50 | - |
| iso-Propanol | Heptan | 60:40 | - |
| iso-Propanol | Hexan | 10:90 | +++ |
| iso-Propanol | Hexan | 30:70 | +/- |
| iso-Propanol | Hexan | 40:60 | +/- |
| iso-Propanol | Hexan | 50:50 | - |
| iso-Propanol | Hexan | 60:40 | - |
| iso-Propanol | Cyclohexan | 10:90 | +++ |
| iso-Propanol | Cyclohexan | 30:70 | +/- |
| iso-Propanol | Cyclohexan | 40:60 | +/- |
| iso-Propanol | Cyclohexan | 50:50 | - |
| iso-Propanol | Cyclohexan | 60:40 | - |

Mit verschiedenen Lösungsmittelgemischen konnten mit mikrokristallinem Everolimus stabile Kristallsuspensionen hergestellt werden. Es hat sich gezeigt, dass ein Anteil von mindestens 50 Vol.-% an unpolarem Lösungsmittel zu sehr stabilen Kristallsuspensionen führt.

### Beispiel 7

### Beschichtungen von Ballonkathetern mit Kristallsuspensionen von mikrokristallinem Everolimus

Ballonkatheter 4x40mm wurden mit einer 2%igen EVR-Suspension enthaltend Trioctanoylglycerol (20Gew.-% bezogen auf EVR) mit einer Tropfendosiertechnik im Mikrodosierverfahren wie das Pipettierverfahren, oder Tropfenschleppverfahren beschichtet. Es ließ sich eine durchgängig gleichmäßige Beschichtung mit ebenfalls gleichmäßiger Wirkstoffkonzentration auf der Ballonoberfläche herstellen, bei der die Kristalle gleichmäßig verteilt sind. Die Untersuchungen zur Wiederfindungsrate von Wirkstoff auf in gleich großen Segmenten zerteilten Ballonkathetern bestätigt die Gleichmäßigkeit der Beschichtung und damit den Erfolg bei der Nutzung einer Kristallsuspension als auch eine 100%ige Wiederfindungsrate (siehe Tab. 13).

**Tabelle 13: Ballonkatheter 4x40mm, beschichtet mit einer 2%igen EVR-Suspension (3 möglichst gleichgroße Schnitte in 4 Segmente)**

| Proben Kathetersegmente | EVR-Gehalt [µg/segm] | Wiederfindung [%/segm] | Gesamt [%/total] Ziel 100% |
|---|---|---|---|
| Ballon 1: | | | |
| Distal 1 | 359,1 | 95,3 | 108,9 |
| Mid 1 | 402,6 | 106,8 | |
| Mid 1 | 436,8 | 115,9 | |
| Proximal 1 | 429,9 | 114,0 | |

| Ballon 2: | | | |
|---|---|---|---|
| Distal 2 | 357,9 | 94,9 | 111,3 |
| Mid 2 | 453,3 | 120,2 | |
| Mid 2 | 511,3 | 135,6 | |
| Proximal 2 | 353,7 | 93,8 | |

| Ballon 3: | | | |
|---|---|---|---|
| Distal 3 | 380,5 | 100,9 | 109,4 |
| Mid 3 | 421,9 | 111,9 | |
| Mid 3 | 452,2 | 119,9 | |
| Proximal 3 | 389,3 | 103,3 | |

Ballonkatheter 7x150mm wurden mit einer 2%igen EVR-Suspension enthaltend Trioctanoylglycerol (20Gew.-% bezogen auf EVR) mit einer Tropfendosiertechnik im Mikrodosierverfahren wie das Pipettierverfahren, oder Tropfenschleppverfahren beschichtet. Es ließ sich eine durchgängig gleichmäßige Beschichtung mit ebenfalls gleichmäßiger Wirkstoffkonzentration auf der Ballonoberfläche herstellen, bei der gewährleistet ist, dass die Kristalle gleichmäßig verteilt sind. Die Untersuchungen zur Wiederfindungsrate von Wirkstoff auf in gleich großen Segmenten zerteilten Ballonkathetern bestätigt die Gleichmäßigkeit der Beschichtung und damit den Erfolg bei der Nutzung einer Kristallsuspension als auch eine 100%ige Wiederfindungsrate (siehe Tab. 14).

**Tabelle 14: Ballonkatheter 7x150mm, beschichtet mit einer 2%igen EVR-Suspension (14 möglichst gleichgroße Schnitte in 15 Segmente)**

| Proben Kathetersegmente | EVR-Gehalt [µg/segm] | Wiederfindung [%/segm] | Gesamt [%/total] Ziel 100% |
|---|---|---|---|
| Distal 1 | 731,6 | 110,9 | 102,3% |
| mid 2 | 790,2 | 119,8 | |
| mid 3 | 792,4 | 120,1 | |
| mid 4 | 1027,6 | 155,8 | |
| mid 5 | 732,9 | 111,1 | |
| mid 6 | 625,1 | 94,8 | |
| mid 7 | 572,2 | 86,7 | |
| mid 8 | 682,4 | 103,4 | |
| mid 9 | 527,7 | 80,0 | |
| mid 10 | 562,0 | 85,2 | |
| mid 11 | 594,5 | 90,1 | |
| mid 12 | 780,1 | 118,2 | |
| mid 13 | 759,8 | 115,2 | |
| mid14 | 650,7 | 98,6 | |
| Proximal 15 | 194,8 | 44,7 | |

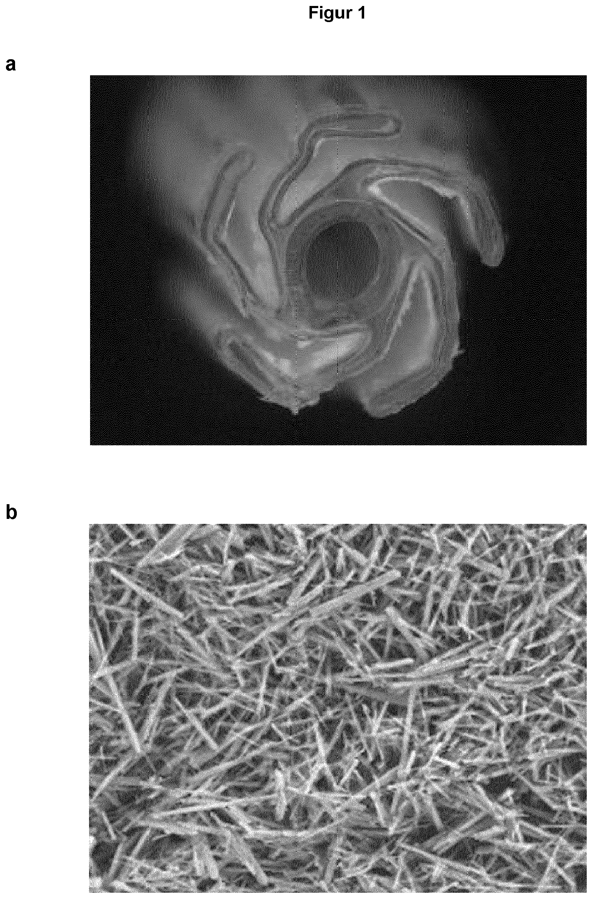

SEM-Aufnahmen der Beschichtungen. Figur 1 a) zeigt eine Querschnittansicht eines umlaufend beschichteten und teilweise gefalteten Ballons und b) zeigt die mikrokristalline Struktur der Everolimus-Beschichtung unter SEM bei 1000-facher Vergrößerung.

### Beispiel 8

### Beschichtungen von Ballonkathetern mit Kristallsuspensionen von mikrokristallinem Rapamycin (SIR)

### Kristallsuspension von Rapamycin (SIR) enthaltend Trioctanoylalycerol

Für die Beschichtungen von Ballonkathetern mit Kristallsuspensionen von mikrokristallinem Rapamycin (SIR) enthaltend Trioctanoylglycerol (20Gew.-% bezogen auf EVR) wurden zwei verschiedene 2%ige-Kristallsuspension bereitgestellt.

Die erste Kristallsuspension wurde mit Rapamycin in Form von Mikrokristallen mit einer Partikelgrößenverteilung im Bereich von 20 µm bis 40µm hergestellt. Rapamycin liegt hier im Wesentlichen vollständig in Form von rhomboedrischen Prismen vor.

Die zweite Kristallsuspension wurde mit Rapamycin in Form von Mikrokristallen hergestellt, wobei die Kristalle von Rapamycin zuvor vermahlen wurden, um eine breitere Kristallgrößenverteilung bereitzustellen.

Ballonkatheter 4x40mm wurden jeweils mit einer 2%igen SIR-Suspensionen enthaltend Trioctanoylglycerol (20Gew.-% bezogen auf EVR) mit einer Tropfendosiertechnik im Mikrodosierverfahren wie das Pipettierverfahren, oder Tropfenschleppverfahren beschichtet. Es ließ sich eine durchgängig gleichmäßige Beschichtung mit ebenfalls gleichmäßiger Wirkstoffkonzentration auf der Ballonoberfläche herstellen. Die Untersuchungen zur Wiederfindungsrate von Wirkstoff auf in gleich großen Segmenten zerteilten Ballonkathetern bestätigt die Gleichmäßigkeit der Beschichtung und damit den Erfolg bei der Nutzung einer Kristallsuspension als auch eine 100%ige Wiederfindungsrate.

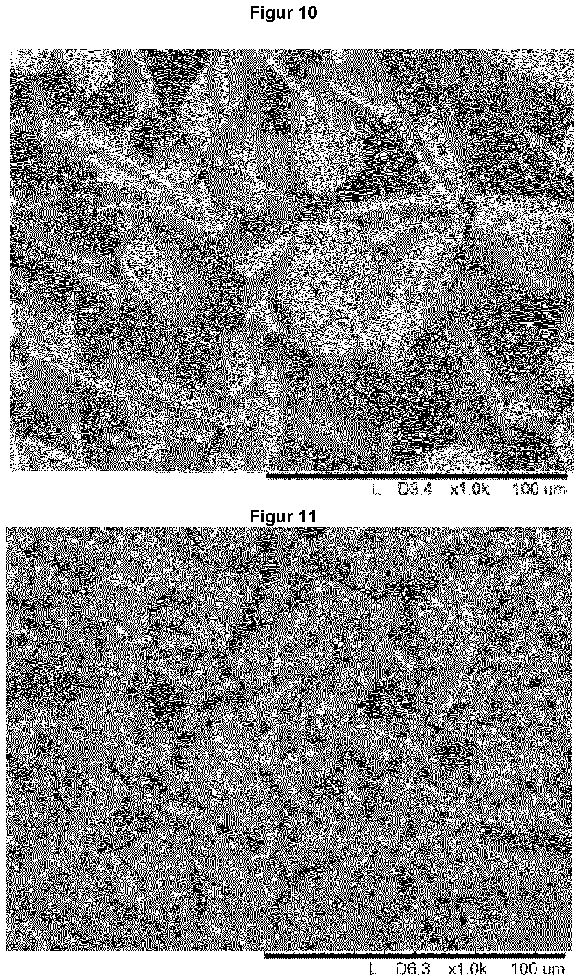

REM-Aufnahmen der Beschichtungen. In Fig. 10 ist die mikrokristalline Struktur der Rapamycin-Beschichtung im Wesentlichen vollständig in Form von rhomboedrischen Prismen vorliegenden Mikrokristallen von Rapamycin (ohne Vermahlung) unter REM bei 1000-facher Vergrößerung gezeigt.

In Fig. 11 ist die mikrokristalline Struktur der Rapamycin-Beschichtung mit vermahlenen Mikrokristallen von Rapamycin unter REM bei 1000-facher Vergrößerung gezeigt.

Anschließend wurden diese beschichteten Ballonkatheter über einer schwarzen Unterlage gegen eine Kante eines geeigneten Gegenstandes geschlagen (Kantenschlagtest). Die auf der Unterlage aufgefangenen Partikel wurden anschließend mikroskopisch bestimmt und die Größenverteilung der abgelösten Beschichtung ermittelt. Der inflatierte Ballon wurde anschließend in PBS-Lösung getaucht, damit restliche noch locker anhaftende Partikel ebenfalls abfallen und in die Bewertung aufgenommen werden können. In einer weiteren Untersuchung wurden weitere beschichtete Ballonkatheter über einer schwarzen Unterlage vorschriftsmäßig inflatiert und in verschiedene Richtungen verbogen (Biegeversuch). Auf der Unterlage aufgefangene Partikel wurden anschließend mikroskopisch bestimmt und die Größenverteilung der abgelösten Beschichtung ermittelt. Der inflatierte Ballon wurde anschließend noch in PBS-Lösung getaucht, damit restliche noch locker anhaftende Partikel ebenfalls abfallen und in die Bewertung aufgenommen werden können.

### Kristallsuspension von Rapamycin (SIR) enthaltend nicht erfindungsgemäße Triacylglycerole

In Beispiel 2 hat sich gezeigt, dass der mikrokristalline Wirkstoff sich nicht vollständig in den Lösungen enthaltend Tridodecanoylglycerol oder Tritetradecanoylglycerol gelöst hat. Die Suspensionen enthaltend Tridodecanoylglycerol oder Tritetradecanoylglycerol erwiesen sich als instabil. Zur Untersuchung der Stabilität, Flexibilität und Haftfähigkeit von Beschichtungen von Ballonkathetern mit von mikrokristallinem Rapamycin wurden nicht erfindungsgemäßen Suspensionen von mikrokristallinem Rapamycin (SIR) enthaltend Tridodecanoylglycerol oder Tritetradecanoylglycerol (20Gew.-% bezogen auf EVR) frisch hergestellt und direkt für die Beschichtung verwendet. Rapamycin wurde hierzu in Form von Mikrokristallen mit einer Partikelgrößenverteilung im Bereich von 20 µm bis 40µm bereitgestellt.

Ballonkatheter 4x40mm wurden jeweils mit einer 2%igen SIR-Suspension enthaltend Tridodecanoylglycerol oder Tritetradecanoylglycerol (20Gew.-% bezogen auf EVR) mit einer Tropfendosiertechnik im Mikrodosierverfahren wie das Pipettierverfahren oder Tropfenschleppverfahren beschichtet. Es zeigte sich, dass die Beschichtung mit diesen Suspensionen nicht ausreichend gleichmäßig aufgetragen werden.

Anschließend wurden diese beschichteten Ballonkatheter über einer schwarzen Unterlage gegen eine Kante eines geeigneten Gegenstandes geschlagen (Kantenschlagtest). Die auf der Unterlage aufgefangenen Partikel wurden anschließend mikroskopisch bestimmt und die Größenverteilung der abgelösten Beschichtung ermittelt. Der inflatierte Ballon wurde anschließend in PBS-Lösung getaucht, damit restliche noch locker anhaftende Partikel ebenfalls abfallen und in die Bewertung aufgenommen werden können. In einer weiteren Untersuchung wurden weitere beschichtete Ballonkatheter über einer schwarzen Unterlage vorschriftsmäßig inflatiert und in verschiedene Richtungen verbogen (Biegeversuch). Auf der Unterlage aufgefangene Partikel wurden anschließend mikroskopisch bestimmt und die Größenverteilung der abgelösten Beschichtung ermittelt. Der inflatierte Ballon wurde anschließend noch in PBS-Lösung getaucht, damit restliche noch locker anhaftende Partikel ebenfalls abfallen und in die Bewertung aufgenommen werden können.

### Vergleich der Ergebnisse des Kantenschlagtests und Biegeversuch der erfindungsgemäßen Beschichtungen und der nicht erfindungsgemäßen Beschichtungen

Der Kantenschlagtest und der Biegetest zeigten deutlich, dass der Gesamtpartikelverlust als auch die Partikelgrößenverteilung/Ballonoberfläche bei der erfindungsgemäßen Beschichtung mit Trioctanoylglycerol weit unter dem der nicht erfindungsgemäßen Beschichtungen mit Tridodecanoylglycerol oder Tritetradecanoylglycerol liegt. Die Partikelfreisetzung für die erfindungsgemäße Beschichtung mit Mikrokristallen von Rapamycin und Trioctanoylglycerol zeigt, dass sich so gut wie keine Partikel ablösen lassen. Die erfindungsgemäß beschichteten Ballonkatheter liegen mit einer ermittelten Partikelanzahl weit unter dem Stand der Technik.

### Beispiel 9

### Partikel-Freisetzung ("Bröseltest"), Ermittlung des Verlustes von Wirkstoff bzw. Beschichtung bei Implantation anhand eines in-Vitro-Modells, Vorbenetzung von Implantatoberflächen, Bestimmung einer uniformen Beschichtung

### 1. Partikel-Freisetzung ("Bröseltest")

Als Kontrolle der mechanischen Haftfähigkeit einer Beschichtung auf einer Oberfläche wird die Partikel-Freisetzung gemessen ("Bröseltest"), wobei ermittelt wird, wie viele Partikel und welcher Größe bei Stoß auf Kanten und bei Verbiegung (während und nach der Inflation des Ballons) des beschichteten Medizinproduktes von der Oberfläche gelöst werden und damit verloren gehen. Hierzu werden die beschichteten Implantate bis zu drei mechanischen Prüfungen unterzogen. Das gewichtsbestimmte beschichtete Implantat wird vor und nach der Prüfung gewogen.

### a) Kantenschlagtest

Der beschichtete Ballonkatheter wird über einer schwarzen Unterlage leicht gegen eine harte (scharfe) Kante eines geeigneten Gegenstandes geschlagen. Auf der Unterlage aufgefangene Partikel werden anschließend mikroskopisch bestimmt und die Größenverteilung der abgelösten Beschichtung ermittelt. Der inflatierte Ballon wird anschließend noch in PBS-Lösung getaucht. Damit fallen restliche noch locker anhaftende Partikel ebenfalls ab und können in die Bewertung aufgenommen werden.

### b) Biegeversuch

Der beschichtete Ballonkatheter wird über einer schwarzen Unterlage vorschriftsmäßig inflatiert per Hand in verschiedene Richtungen verbogen. Auf der Unterlage aufgefangene Partikel werden anschließend mikroskopisch bestimmt und die Größenverteilung der abgelösten Beschichtung ermittelt. Der inflatierte Ballon wird anschließend noch in PBS-Lösung getaucht. Damit fallen restliche noch locker anhaftende Partikel ebenfalls ab und können in die Bewertung aufgenommen werden.

### c) Adhäsionstest

Hierzu werden vor allem bei längeren Ballonkathetern, wie z.B. periphere Ballons mit einer Länge von 150 mm deflatiert als auch inflatiert, um ein vom Durchmesser passendes rundes Gefäß (z.B. Reagenzglas, Standzylinder o.ä. gewickelt und überprüft, ob zum einen keine Brösel entstehen und zum anderen wird geprüft, ob die Beschichtung sich vom Ballonkatheter löst und an der Oberfläche des Gefäßes haftet oder nicht. Biegen um einen glatten Gegenstand (bevorzugt ein Laborgefäß aus Glas, das vom Umfang passt, damit der Katheter genügend gebogen werden kann und prüfen, ob und wenn wieviel auf die schwarze Unterlage fällt. Die Hydrolyseröhrchen haben einen Durchmesser von 12,8 mm. Die Durchführung erfolgt dergestalt, dass der Ballon um das Hydrolyseröhrchen gebogen wird und dabei Kontakt zur Wandung besteht. Schmieriger Abrieb am Glas ist tolerabel, abbröckelnde Masse nicht tolerabel.

Die "Bröseltests" zeigen deutlich, dass der Gesamtpartikelverlust als auch die Partikelgrößenverteilung/Ballonoberfläche aller Proben weit unter den Richtlinien der FDA liegt. Mit Trioctanoylglycerol lässt sich in der Tabelle 15 deutlich erkennen, dass dieses unabhängig von der Beladung mit 1 µg/mm² EVR-Mikrokristallen oder 3 µg/mm² EVR-Mikrokristallen wesentlich weniger Partikel freigibt und weit unter den Werten liegt, die sich für käuflich erwerbbare und damit zugelassenen beschichtete Ballonkatheter ergeben. Die Partikelfreisetzung in allen gemessenen Partikelgrößenbereichen für die erfindungsgemäße Beschichtung mit Mikrokristallen von Everolimus und Trioctanoylglycerol (hier auf verschiedenen BMT-Katheterballons) zeigt, dass sich so gut wie keine Partikel ablösen lassen. Alle erfindungsgemäß beschichteten Ballonkatheter liegen mit einer ermittelten Partikelanzahl weit unter dem Stand der Technik.

Es zeigt sich also, dass die Flexibilität und Stabilität der erfindungsgemäßen Kristallbeschichtungen von Mikrokristallen von Everolimus-beschichteten Ballons weit über dem zugelassenen Standard und Stand der Technik liegen.

**Tabelle 15: Anzahl Partikelverlust/mm² Ballonoberfläche in Abhängigkeit von der Partikelgröße bei unterschiedlicher Wirkstoffbeladung mit 1 µg EVR/mm² und 3 µg EVR/mm² mit Trioctanoylglycerol (HTQ* = Hemoteq, Trioctanoylglycerol 20Gew.% bzgl. EVR)**

| | | | | | |
|---|---|---|---|---|---|
| **Freigesetzte Partikel**/mm² Ballonoberfläch e | **HTQ*** Trioctanoyl -glycerol | **HTQ** Trioctanoyl -glycerol | **HTQ** Trioctanoyl -glycerol | **HTQ** Trioctanoyl -glycerol | **HTQ** Trioctanoyl -glycerol |
| Ballongröße [mm] | 6,0x40 | 6,0x40 | 6,0x40 | 5,0x40 | 2,0x40 |
| Beladung EVR [µg] | 3 | 1 | 1 | 1 | 1 |
| **≥ 10 µm** | 28,4 | 12,8 | 13,2 | 12,9 | 58,4 |
| **≥ 25 µm** | 4,2 | 1,5 | 2,1 | 2,4 | 6,5 |
| **≥ 65 µm** | 0,1 | 0,2 | 0,1 | 0,1 | 0,2 |
| **≥ 100 µm** | 0 | 0 | 0 | 0 | 0 |

Die Kristallbeschichtung mit Trioctanoylglycerol/EVR während und nach Inflation zeigt bei optischer Überprüfung eine gleichmäßige Beschichtung mit gleichmäßiger Oberflächenstruktur auf. Die Beschichtung bröselt nicht bei der Inflation des Ballons ab.

Neben hervorragender Flexibilität und nahezu verlustfreier Haftfähigkeit zeigt die erfindungsgemäße Kristallbeschichtung auch die erforderliche und notwendige Temperaturstabilität, Sterilisierbarkeit (bevorzugt wird die ETO-Sterilisierung) und Lagerfähigkeit (shelf life).

### 2. Ermittlung des Verlustes von Wirkstoff bzw. Beschichtung bei Implantation anhand eines in-Vitro-Modells

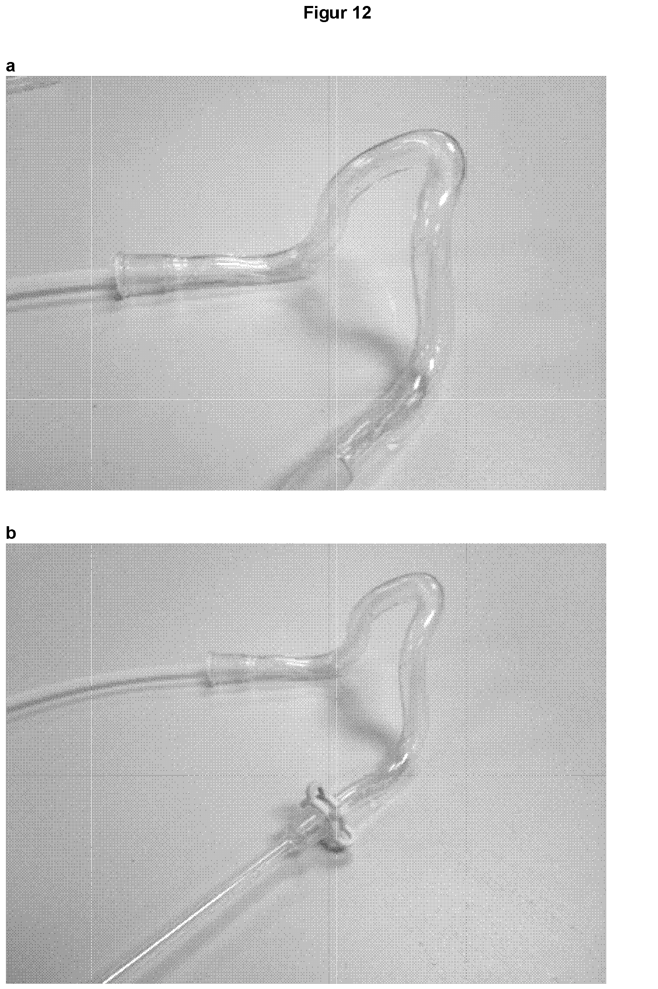

Zur Simulierung der natürlichen oft gekrümmten Wege durch die Gefäße, die ein Ballonkatheter zum Implantationsort zurücklegen muss, wird ein Modell aus Silikonschlauch geformt (siehe Fig. 12a und Fig. 12b), dass dem natürlichen Verlauf der Gefäße im Organismus nachempfunden ist.

Der Katheter wird in den die Arterie simulierenden Silikonschlauch eingeführt und inflatiert. Der Silikonschlauch wurde vorher mit einem definierten Volumen pyrogenfreiem Wasser gefüllt. Nach 60 sec. wird der Ballon deflatiert (Vakuum ziehen) und vorsichtig herausgezogen. Dabei wird darauf geachtet, dass die Flüssigkeit aus dem Schlauch vollständig in einem Behälter aufgefangen wird. Anschließend wird mit einer definierten Menge Wasser nachgespült und ebenfalls aufgefangen. Die Partikelanalyse (Partikelgrößenverteilung und Quantifizierung) erfolgt über LPC (Liquid Partikel Counter).

**Tabelle 16: Partikel-Freisetzung verschiedener Beschichtungen mit Everolimus Kristallsuspensionen enthaltend 20% Trioctanoylglycerol bezogen auf Everolimus während der Inflation in PBS nach in-vitro-Bestimmung mittels LPC**

| Probe | Partikelanzahl gesamt | | | |
|---|---|---|---|---|
| | ≥10 µm | ≥25 µm | ≥65 µm | ≥100 µm |
| 6,0x40, 3µg, 20% Trioctanoylglycerol | 369 | 59 | 0 | 0 |
| 6,0x40, 1µg, 20% Trioctanoylglycerol | 24 | 25 | 0 | 0 |
| 5,0x40, 1µg, 20% Trioctanoylglycerol | 123 | 0 | 0 | 0 |
| 2,0x40, 1µg, 20% Trioctanoylglycerol | 89 | 26 | 0 | 0 |

### 3. Bestimmung einer uniformen Beschichtung

Hierzu wird der beschichtete gewogene Ballon fixiert und inflatiert. Anschließend wird der Ballon mit einem Skalpell in möglichst gleich große Stücke geschnitten, z.B. ein 40 mm langer Ballon in 4 Stücke, ein 120 mm langer Ballon kann in 6 Stücke aufgeteilt werden. Als erstes wird in Längsrichtung halbiert wird und die Schichtdicke mit einem Mikrometer gemessen. Anschließend wird der Ballon zerteilt und die Stücke gewogen und ebenfalls die Schichtdicken mit dem Mikrometer gemessen.

Die Beschichtungen werden je in einer definierten Menge Aceton gelöst und die Wirkstoffmenge über HPLC bestimmt. Die Ergebnisse werden unter Berücksichtigung der Ballonabschnittflächen miteinander verglichen.

In allen Fällen hat sich herausgestellt, dass die Anwesenheit von Trioctanoylglycerol, oder Tridecanoylglycerol eine besonders stabile und flexible Beschichtung ergeben, wobei der Wirkstoff auch in Form von Kristallen sehr gut haftet und dieser erst während der Kontaktzeit mit dem Zielort abgegeben wird.

### Beispiel 10

### Vergleich mit nicht erfindungsgemäßen Kristallbeschichtungen

### Kristallbeschichtungen gemäß WO 2015/039969 A1

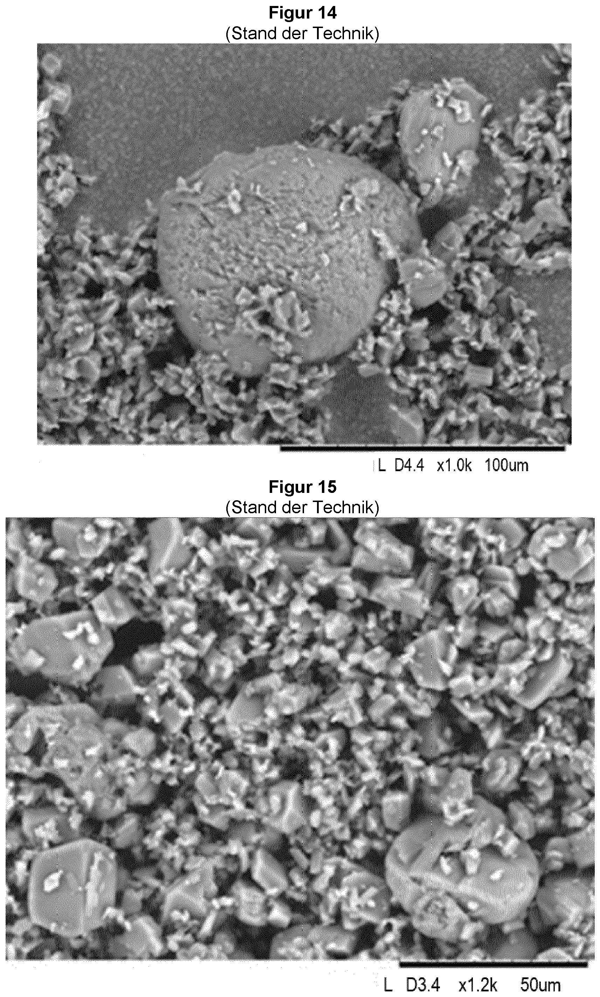

In Figur 14 ist eine nicht erfindungsgemäße Beschichtung mit Rapamycinkristallen gemäß WO 2015/039969 A1 bei 1000-facher Vergrößerung gezeigt. Es ist ein zu großer fast runder Kristall umgeben von vielen recht kleinen Kristallen unregelmäßiger Form und breiter Partikelgrößenverteilung zu sehen. Figur 15 zeigt die nicht erfindungsgemäße Beschichtung mit Rapamycinkristalle gemäß WO 2015/039969 A1 bei 1200-facher Vergrößerung. Es sind zahlreiche recht große Kristalle umgeben von vielen recht kleinen Kristallen unregelmäßiger Form und breiter Partikelgrößenverteilung zu sehen.

### Kristallbeschichtungen von Rapamycin-Mikrokristallen und "Solvent Bonding"

Es wurde geprüft, inwieweit Limus-Kristalle als Trockensubstanz (Pulver) auf Ballonkatheter aufgebracht werden können. Bewertet wurde u.a. die Haftung der Kristalle. Für die Versuche wurden von der Hemoteq hergestellte RapamycinKristalle verwendet. Für die Applikation der Beschichtung wurden PTA-Katheter mit einer Ballon-Größe von 4,0x 60mm verwendet.

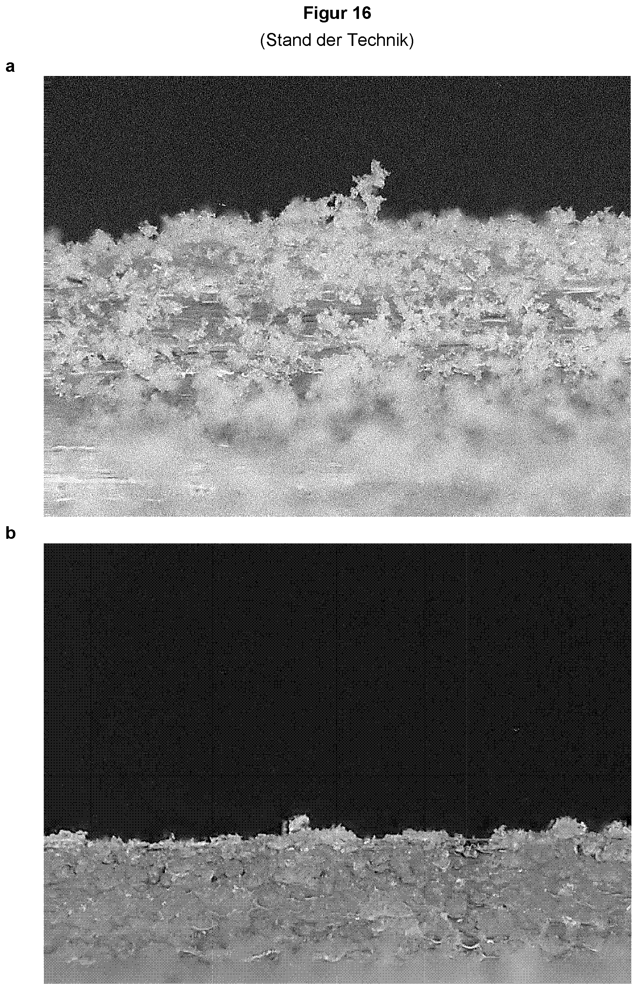

Zunächst erfolgte der Auftrag des reinen Kristallpulvers. Hierzu wird das Pulver in eine maßgefertigte Schale gefüllt und in Kontakt mit dem Ballon gebracht. Dabei nimmt der rotierende Ballon Kristalle auf, die an der Oberfläche haften bleiben. Anschließend wurde vorsichtig ein Lösungsmittel aufgesprüht, um die Kristalle leicht anzulösen, damit diese nach dem anschließenden Trocknen besser an der Ballonoberfläche haften. In Figur 16a) ist die nicht erfindungsgemäße Beschichtung mit Rapamycinkristallen bei 200-facher Vergrößerung gezeigt. In Figur 16b) ist deutlich zu sehen, dass die Mikrokristalle von Rapamycin nicht intakt bleiben und angelöst werden. Die "Bröseltests" zeigten deutlich, dass der Gesamtpartikelverlust und die Partikelfreisetzung sehr hoch sind. Die Beschichtung haftet schlecht an der Ballonoberfläche.

### Kristallbeschichtungen von Rapamycin-Mikrokristallen mit Basisbeschichtung von handelsüblichem Kleber bzw. Trioctanoylglycerollösung und optional Topcoat mit Trioctanoylglycerollösung

Es wurde geprüft, inwieweit Limus-Kristalle als Trockensubstanz (Pulver) auf Ballonkatheter aufgebracht werden können. Bewertet wurde u.a. die Haftung der Kristalle. Für die Versuche wurden von der Hemoteq hergestellte Sirolimus-Kristalle verwendet. Für die Applikation der Beschichtung wurden PTA-Katheter mit einer Ballon-Größe von 4,0x 60mm verwendet.

Zunächst erfolgte der Auftrag einer Basis-Beschichtung (base coat), die für die Haftung der Kristalle auf dem Ballon sorgen soll. Hier wurde zur Testung des Versuchsaufbau auf Eignung zunächst handelsüblicher Medizin-Klebstoff (Fa. Henkel) verwendet.

Basisbeschichtung aus handelsüblichem Klebstoff (Uhu): Der Klebstoff wird direkt aus der Tube dünn aufgetragen und gleichmäßig auf dem rotierenden Ballon verteilt. Danach erfolgt der Auftrag der Kristalle.

Weiterhin wurde Trioctanoylglycerol als Basisbeschichtung verwendet. Die Applikation der Basisbeschichtung erfolgt durch Pipettieren auf dem rotierenden Katheter. Nach ca. 10min Trocknung der Basisbeschichtung erfolgt der Auftrag des reinen Kristallpulvers. Hierzu wird das Pulver in eine maßgefertigte Schale gefüllt und in Kontakt mit dem Ballon gebracht. Dabei nimmt der rotierende Ballon Kristalle auf, die an der Oberfläche haften bleiben.

### Zusammensetzung der Lösung:

84,4% n-Heptan (Vol-%)
15,6% Ethylacetat (Vol.-%)
0,05% Butylhydroxytoluol (Masse/Volumen)
200 µl Trioctanoylglycerol werden in 2ml der Lösung gelöst. Es werden 2 x 50 µl der Basisbeschichtungs-Lösung aufgebracht.

Bei der Verwendung der Trioctanoylglycerol hatte sich gezeigt, dass die Kristalle zwar an der Ballonoberfläche haften, die Haftung der Kristalle untereinander jedoch nicht ausreichend war.

In einem dritten Beschichtungsschritt wurde daher ein Topcoat mit Trioctanoylglycerol zur Erhöhung der Haftung mit einer Pipette aufgebracht. Die Bewertung der Haftung erfolgte über einen "Biegetest". Der Biegetest ist eine Methode, bei der der beschichtete Ballon 2x um ein Glasrohr mit ca. 14mm Durchmesser gebogen wird. Lösen sich bei diesem Vorgang viele und/oder größere Fragmente aus der Beschichtung, wird die Haftung als unzureichend bewertet.

### Zusammensetzung der Topcoat-Lösung:

84,4% n-Heptan (Vol.-%)
15,6% Ethylacetat (Vol.-%)
0,25% Trioctanoylglycerol (Masse/Volumen)
0,05% Butylhydroxytoluol (Masse/Volumen)

Es wurden jeweils 2 x 30 µl Topcoat-Lösung aufpipettiert.

Bei der Verwendung der Topcoat-Lösung hatte sich gezeigt, dass die Kristalle besser an der Ballonoberfläche haften, die Haftung der Kristalle untereinander war ebenfalls verbessert, so dass beim Kantenschlagtest weniger Partikelfreisetzung auftrat. Jedoch zeigte sich beim Biegeversuch und bei der Inflation des Ballons, dass die Haftung der Kristalle untereinander nicht ausreichend ist.

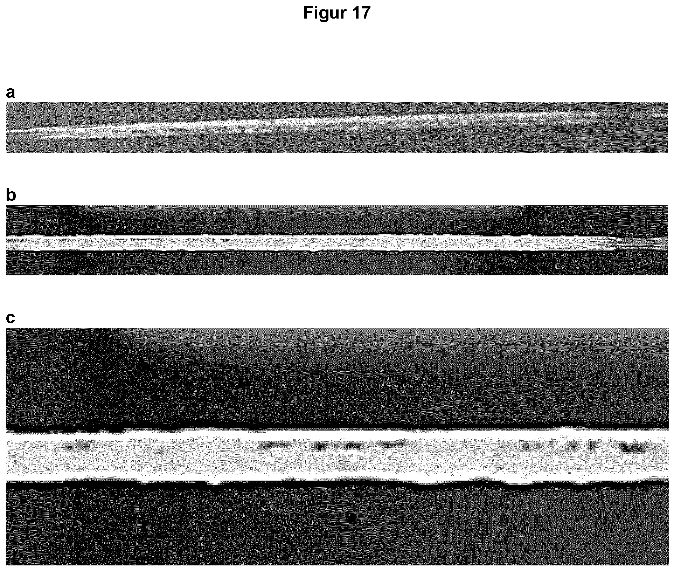

In Fig. 17 a-c sind Aufnahmen der Ballonbeschichtung mit Topcoat gezeigt. Die Figur 17c ist eine Vergrößerung der Figur 17b. Es ist mit bloßem Auge deutlich zu erkennen, dass die Beschichtungen nicht gleichmäßig sind und größere Bereiche aufweisen, in denen keine Mikrokristalle vorliegen. Zudem ist die Reproduzierbarkeit bei diesen Beschichtungen sehr schlecht. Somit konnten besonders gleichmäßige, flexible und sehr gut haftende Beschichtung mit Mikrokristallen von Rapamycin auf diese Weise nicht hergestellt werden.

### Beispiel 11

### In vivo-Studie mit Sirolimus (SIR, kristallin) und Everolimus (EVR, kristallin) und Trioctanoylglycerol (GTC, 20Gew.% bzgl. Wirkstoff) auf PTA-Kathetern

Die Studie diente zur Ermittlung der lokalen Pharmakokinetik von Sirolimus und Everolimus in Anwesenheit von Trioctanoylglycerol. Zum Vergleich wurden drei kommerziell erhältliche Sirolimus und Everolimus-eluierende Stents und Ballonkatheter herangezogen. Zum Vergleich wurde für den Sirolimus eluierenden Ballonkatheter ein kommerziell erhältlicher Sirolimus beschichteter Ballonkatheter (Magic Touch von Concept Medical) und ein Sirolimus eluting Stent (Orsiro von Biotronik) in die Studie einbezogen. Da es keinen kommerziellen Everolimus beschichteten Ballonkatheter gibt, konnte nur ein Everolimus eluierender Stent (Promus von Boston Scientific) in die Studie als Vergleich eingebracht werden.

Hierzu wurden Ballonkathetern verschiedener Größen mit EVR-Kristallsuspension/GTC (3 µg/mm² EVR, 20wt% bzgl. EVR) und SIR-Kristallsuspension/GTC (3 µg/mm² SIR, 20wt% bzgl. SIR) beschichtet. Als Versuchstiere standen 30 gesunde Hausschweine (männlich, kastriert) zur Verfügung.

Nach der Implantation werden auf den Oberflächen beider Ausführungsformen die verbleibenden Wirkstoffreste ermittelt, wobei deutlich wird, dass die Übertragung auf die Gefäßwand sehr gut funktioniert hat und nur noch kleine Reste auf dem Ballon zurückgeblieben sind und somit von einer äußerst effektiven Übertragung auf die Gefäßwand ausgegangen werden kann (siehe Tab. 17).

**Tabelle 17: Durchschnittlicher verbleibender Wirkstoffgehalt auf den PTA-Kathetern nach Implantation**

| **Proben** | **Durchschnittlicher Wirkstoffgehalt*** | **SD** |
|---|---|---|
| **SCB-2** | | |
| 3.5 - 4.0mm | 7,4% | 2,8% |
| 5.0 - 6.0mm | 3,9% | 2,4% |

| **ECB-4** | | |
|---|---|---|
| 3.5 - 4.0mm | 3,0% | 1,0% |
| 5.0 - 6.0mm | 17,1% | 5,6% |

| | | |
|---|---|---|
| *Ermittelte Wirkstoffreste auf Ballon nach Implantation in % bezogen auf Nominalbeladung | | |

Auch die komplementären Wirkstoffkonzentrationen in den Gefäßwänden nach Implantation und nach 7 und 28 Tagen zeigen eine erfolgreiche Wirkstoffabgabe - auch im Vergleich zu den Vergleichsprobe. So gibt der Magic Touch als direkter DCB-Vergleich wesentlich weniger Sirolimus an die Gefäßwand ab als der erfindungsgemäße SCB-2. Nach 7 Tagen ist die Konzentration von Sirolimus beim SBC-2 eher dem Stent vergleichbar als dem Magic Touch und dieser Unterschied setzt sich auch nach 28 Tagen fort. Damit ist der erfindungsgemäße SCB-2 definitiv dem Magic Touch als DCB und dem DES Orsiro überlegen.

**Tabelle 18: Für die Sirolimus/Trioctanoylglycerol-Gruppe (SCB) ergeben sich folgende Follow-up-Werte für die Wirkstoffkonzentration in der Arterie nach 1-2h, 7d und 28d**

| | SCB-2 | | Magic Touch DCB (Referenz) | | Sir-eluting stent (Referenz) | |
|---|---|---|---|---|---|---|
| Follow-up nach | MW | SD | MW | SD | MW | SD |
| | [µg/g] | | [µg/g] | | [µg/g] | |
| 1-2h | 22,4 | 20,5 | 4,4 | 3,2 | - | - |
| 7d | 0,91 | 1,10 | 0,28 | 0,09 | 1,07 | 0,45 |
| 28d | 3,66 | 4,98 | 0,45 | 0,64 | 1,35 | 1,52 |

Für den Everolimus/ Trioctanoylglycerol -Ballonkatheter (ECB) sind die Abgabewerte noch wesentlich erhöht und besser. Die Abgabe von Wirkstoff in die Gefäßwand ist optimal erhöht. Der Vergleich mit dem Everolimus eluting stent zeigt die Überlegenheit des erfindungsgemäßen Ballonkatheters auch gegenüber dem Stent, der sogar im Körper verbleibt bis zur Explantation.

**Tabelle 19: Für die Everolimus/Trioctanoylglycerol -Gruppe (ECB) ergeben sich folgende Follow-up-Werte für die Wirkstoffkonzentration in den Arterien nach 1-2 h, 7 d und 28 d**

| | ECB | | Promus EES* (Referenz) | |
|---|---|---|---|---|
| Follow-up nach | MW | SD | MW | SD |
| | [µg/g] | | [µg/g] | |
| 1-2h | 207,3 | 93,1 | n/a | n/a |
| 7d | 13,1 | 21,4 | 1,96 | 2,15 |
| 28d | 1,80 | 2,56 | 2,18 | 1,85 |
| *EES : Everolimus eluting stent | | | | |

Zudem zeigt sich, dass die Wiederfindungsrate des Wirkstoffes deutlich macht, dass die Wirkstoffe tatsächlich am Bestimmungsort und in der Gefäßwand angekommen sind. (HPLC-Messungen). Der kleine fehlende Rest blieb auf dem Ballonkatheter (siehe Tab. 20). Diese Daten sind ein weiterer Beweis für die Stabilität und Flexibilität als auch für die sehr gute Verfügbarkeit der Wirkstoffe bei Inflation am Zielort.

**Tabelle 20: Wiederfindungsrate der Wirkstoffe Sirolimus (SCB) und Everolimus (ECB) nach 28d (HPLC)**

| Proben | Nominalbeladung | Wiederfindungsrate Wirkstoff (n=5) | SD | Reinheit (n=5) | SD |
|---|---|---|---|---|---|
| | [µg] | [%] | [%] | [%] | [%] |
| **SCB** | | | | | |
| 4,0 x 20mm | 754,0 | 89,6 | 99,0 | 99,0 | 0,0 |
| 5,0 x 40mm | 1885,0 | 79,0 | 97,6 | 97,6 | 0,2 |
| 6,0 x 40mm | 2261,9 | 96,9 | 97,6 | 97,9 | 0,2 |

| **ECB** | | | | | |
|---|---|---|---|---|---|
| 3,5 x 20mm | 659,7 | 114,6 | 99,5 | 99,5 | 0,1 |
| 4,0 x 20mm | 754,0 | 106,3 | 99,5 | 99,5 | 0,0 |
| 5,0 x 40mm | 1885,0 | 96,3 | 98,8 | 98,8 | 0,0 |
| 6,0 x 40mm | 2261,9 | 91,5 | 98,9 | 98,9 | 0,2 |

## Patentansprüche

1. Eine Suspension zur Beschichtung eines Medizinprodukts ausgewählt aus einem Katheterballon, einem Ballonkatheter, einem Stent oder einer Kanüle, die Suspension enthaltend:
a) mindestens ein Tri-O-Acylglycerol ausgewählt aus der Gruppe bestehend aus Trioctanoylglycerol, Trinonanoylglycerol, Tridecanoylglycerol und Triundecanoylglycerol,
b) mindestens einen Limus-Wirkstoff in Form von Mikrokristallen, und
c) ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen.

2. Die Suspension gemäß Anspruch 1, wobei der mindestens eine Limus-Wirkstoff ausgewählt wird aus der Gruppe umfassend oder bestehend aus Rapamycin, Everolimus, Zotarolimus, Umirolimus, Deforolimus, Myolimus, Novolimus, Pimecrolimus Ridaforolimus, Tacrolimus und Temsirolimus.

3. Die Suspension gemäß Anspruch 1 oder 2, wobei das mindestens eine Tri-O-Acylglycerol und der Limus-Wirkstoff mit einem Massenanteil von 10% - 30% Tri-O-Acylglycerol zu 90% - 70 % Limus-Wirkstoff vorliegen.

4. Die Suspension gemäß einem der Ansprüche 1 bis 3, wobei der mindestens eine Limus-Wirkstoff in Form von Mikrokristallen mit einer Kristallgröße im Bereich von 1 µm bis 300 µm vorliegt, und/oder
wobei mindestens 70% des mindestens einen Limus-Wirkstoffs in Form von Mikrokristallen mit einer Kristallgröße im Bereich von 10 µm bis 50 µm vorliegen, und/oder
wobei der mindestens eine Limus-Wirkstoff eine Kristallinität von mindestens 90 Gew.-% aufweist.

5. Die Suspension gemäß einem der Ansprüche 1 bis 4, wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält, und/oder
wobei das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0 ist.

6. Ein Verfahren zur Herstellung der Suspension gemäß einem der Ansprüche 1 bis 5 umfassend die folgenden Schritte:
a) Lösen mindestens eines Tri-O-Acylglycerols ausgewählt aus der Gruppe bestehend aus Trioctanoylglycerol, Trinonanoylglycerol, Tridecanoylglycerol und Triundecanoylglycerol in einem Lösungsmittel oder einem Lösungsmittelgemisch;
b) Herstellen einer Suspension aus mindestens einem Limus-Wirkstoff in Form von Mikrokristallen und der Lösung aus Schritt a),
wobei sich die Mikrokristalle des mindestens einen Limus-Wirkstoffs in der Lösung aus Schritt a) nicht lösen.

7. Das Verfahren gemäß Anspruch 6, wobei der mindestens eine Limus-Wirkstoff ausgewählt wird aus der Gruppe umfassend oder bestehend aus Rapamycin, Everolimus, Zotarolimus, Umirolimus, Deforolimus, Myolimus, Novolimus, Pimecrolimus Ridaforolimus, Tacrolimus und Temsirolimus, und/oder
wobei der mindestens eine Limus-Wirkstoff in Form von Mikrokristallen mit einer Kristallgröße im Bereich von 1 µm bis 300 µm vorliegt, und/oder
wobei mindestens 70% des mindestens einen Limus-Wirkstoffs in Form von Mikrokristallen mit einer Kristallgröße im Bereich von 10 µm bis 50 µm vorliegen, und/oder
wobei der mindestens eine Limus-Wirkstoff eine Kristallinität von mindestens 90 Gew.-% aufweist.

8. Das Verfahren gemäß Anspruch 6 oder 7, wobei das Lösungsmittel ein Nichtlöser mit einer Dielektrizitätskonstante εᵣ bei 20°C von ≤ 2,0 ist oder das Lösungsmittelgemisch mindestens zu 50 Vol.-% einen Nichtlöser mit einer Dielektrizitätskonstante εᵣ bei 20°C von ≤ 2,0 enthält, und/oder
wobei das Lösungsmittelgemisch ein Gemisch von mindestens einem polaren organischen Lösungsmittel mit einem n-Octanol-Wasser-Verteilungskoeffizienten logK_{OW} von -0,5 bis +1,5 und einer Dielektrizitätskonstante εᵣ bei 20°C von 5,0 bis 30 und mindestens einem unpolaren organischen Lösungsmittel mit einer Dielektrizitätskonstante εᵣ bei 20°C von ≤ 3,0 und einem n-Octanol-Wasser-Verteilungskoeffizienten logK_{OW} von ≥ 3,0 ist.

9. Ein Verfahren zur Beschichtung eines Medizinprodukts ausgewählt aus einem Katheterballon, einem Ballonkatheter, einem Stent oder einer Kanüle umfassend die folgenden Schritte:
a) Bereitstellen des Medizinprodukts mit einer Medizinprodukteoberfläche,
b) Bereitstellen einer Suspension gemäß einem der Ansprüche 1 bis 5 enthaltend mindestens ein Tri-O-Acylglycerol ausgewählt aus der Gruppe bestehend aus Trioctanoylglycerol, Trinonanoylglycerol, Tridecanoylglycerol und Triundecanoylglycerol, mindestens einen Limus-Wirkstoff in Form von Mikrokristallen und ein Lösungsmittel oder ein Lösungsmittelgemisch, worin sich das mindestens eine Tri-O-Acylglycerol löst und sich die Mikrokristalle des mindestens einen Limus-Wirkstoffs nicht lösen, und
c) Auftragen der Beschichtungssuspension auf die Medizinprodukteoberfläche mittels Spritzenverfahren, Pipettierverfahren, Kapillarverfahren, Faltensprühverfahren, Tauchverfahren, Sprühverfahren, Schleppverfahren, Fadenschleppverfahren, Tropfenschleppverfahren oder Rollverfahren.

10. Das Verfahren gemäß Anspruch 9, weiter umfassend den folgenden Schritt d) Trocknen der Beschichtung.

11. Ein Medizinprodukt ausgewählt aus einem Katheterballon, einem Ballonkatheter, einem Stent oder einer Kanüle erhältlich nach dem Verfahren gemäß Anspruch 9 oder 10; oder
ein Medizinprodukt ausgewählt aus einem Katheterballon, einem Ballonkatheter, einem Stent oder einer Kanüle beschichtet mit der Suspension gemäß einem der Ansprüche 1 bis 5 und nachfolgender Trocknung der Beschichtung.

## Claims

1. A suspension for coating of a medical device selected from a catheter balloon, a balloon catheter, a stent, or a cannula, the suspension containing:
a) at least one tri-*O*-acylglycerol selected from the group consisting of trioctanoylglycerol, trinonanoylglycerol, tridecanoylglycerol, and triundecanoylglycerol,
b) at least one limus active agent in the form of microcrystals, and
c) a solvent or a solvent mixture in which the at least one tri-*O*-acylglycerol dissolves and in which the microcrystals of the at least one limus active agent do not dissolve.

2. The suspension according to claim 1, wherein the at least one limus active agent is selected from the group comprising or consisting of rapamycin, everolimus, zotarolimus, umirolimus, deforolimus, myolimus, novolimus, pimecrolimus, ridaforolimus, tacrolimus, and temsirolimus.

3. The suspension according to claim 1 or 2, wherein the at least one tri-*O-*acylglycerol and the limus active agent are present in a mass ratio of 10% - 30% tri-*O*-acylglycerol to 90% - 70% limus active agent.

4. The suspension according to any one of claims 1 to 3, wherein the at least one limus active agent is in the form of microcrystals having a crystal size in the range of 1 µm to 300 µm, and/or
wherein at least 70% of the at least one limus active agent is in the form of microcrystals having a crystal size ranging from 10 µm to 50 µm, and/or
wherein the at least one limus active agent has a crystallinity of at least 90% by weight.

5. The suspension according to any one of claims 1 to 4, wherein the solvent is a non-solvent having a dielectric constant εᵣ at 20°C of ≤ 2.0 or the solvent mixture contains at least 50% by volume of a non-solvent having a dielectric constant εᵣ at 20°C of ≤ 2.0, and/or
wherein the solvent mixture is a mixture of at least one polar organic solvent having an n-octanol-water partition coefficient logK_{OW} of -0.5 to +1,5 and a dielectric constant εᵣ at 20°C of 5.0 to 30, and at least one nonpolar organic solvent having a dielectric constant εᵣ at 20°C of ≤ 3.0 and an n-octanol-water partition coefficient logK_{OW} of ≥ 3.0.

6. A method of preparing the suspension according to any one of claims 1 to 5 comprising the following steps:
a) dissolving at least one tri-*O*-acylglycerol selected from the group consisting of trioctanoylglycerol, trinonanoylglycerol, tridecanoylglycerol, and triundecanoylglycerol in a solvent or a solvent mixture;
b) preparing a suspension of at least one limus active agent in the form of microcrystals and the solution from step a),
wherein the microcrystals of the at least one limus active agent do not dissolve in the solution of step a).

7. The method according to claim 9, wherein the at least one limus active agent is selected from the group comprising or consisting of rapamycin, everolimus, zotarolimus, umirolimus, deforolimus, myolimus, novolimus, pimecrolimus, ridaforolimus, tacrolimus, and temsirolimus, and/or
wherein the at least one limus active agent is in the form of microcrystals having a crystal size in the range of 1 µm to 300 µm, and/or
wherein at least 70% of the at least one limus active agent is in the form of microcrystals having a crystal size in the range of 10 µm to 50 µm,
wherein the at least one limus active agent has a crystallinity of at least 90% by weight.

8. The method according to claim 6 or 7, wherein the solvent is a non-solvent having a dielectric constant εᵣ at 20°C of ≤ 2.0 or the solvent mixture contains at least 50% by volume of a non-solvent having a dielectric constant εᵣ at 20°C of ≤ 2.0, and/or
wherein the solvent mixture is a mixture of at least one polar organic solvent having an n-octanol-water partition coefficient logK_{OW} of -0.5 to +1,5 and a dielectric constant εᵣ at 20°C of 5.0 to 30, and at least one nonpolar organic solvent having a dielectric constant εᵣ at 20°C of ≤ 3.0 and an n-octanol-water partition coefficient logK_{OW} of ≥ 3.0.

9. A method for coating of a medical device selected from a catheter balloon, a balloon catheter, a stent, or a cannula, comprising the following steps:
a) providing the medical device with a medical device surface,
b) providing a suspension according to any one of claims 1 to 5 containing at least one tri-*O*-acylglycerol selected from the group consisting of trioctanoylglycerol, trinonanoylglycerol, tridecanoylglycerol, and triundecanoylglycerol, at least one limus active agent in the form of microcrystals, and a solvent or a solvent mixture, in which the at least one tri-O-acylglycerol dissolves and in which the microcrystals of the at least one limus active agent do not dissolve, and
c) applying the coating suspension to the surface of the medical device by means of a syringe method, pipetting method, capillary method, fold spraying method, dipping method, spraying method, dragging method, thread dragging method, drop dragging method, or rolling method.

10. The method according to claim 9, further comprising the following step d) drying the coating.

11. A medical device selected from a catheter balloon, a balloon catheter, a stent, or a cannula, obtainable by the method according to claim 9 or 10;or a medical device selected from a catheter balloon, a balloon catheter, a stent, or a cannula, coated with the suspension according to any one of claims 1 to 5 and subsequent drying of the coating.

## Revendications

1. Une suspension destinée au revêtement d'un dispositif médical choisi parmi un ballonnet de cathéter, un cathéter à ballonnet, une endoprothèse ou une canule, ladite suspension contenant :
a) au moins un tri-*O*-acylglycérol choisi dans le groupe constitué de trioctanoylglycérol, de trinonanoylglycérol, de tridécanoylglycérol et de triundécanoylglycérol ;
b) au moins un principe actif de type « limus » sous forme de microcristaux ; et
c) un solvant ou un mélange de solvants dans lesquels le ou les tri-*O-*acylglycérols se dissolvent et dans lesquels les microcristaux du ou des principes actifs de type « limus » ne se dissolvent pas.

2. La suspension selon la revendication 1, où au moins un principe actif de type « limus » est choisi dans le groupe comprenant ou constitué de rapamycine, d'évérolimus, de zotarolimus, d'umirolimus, de déforolimus, de myolimus, de novolimus, de pimécrolimus, de ridaforolimus, de tacrolimus et de temsirolimus.

3. La suspension selon la revendication 1 ou 2, où au moins un tri-*O*-acylglycérol et au moins un principe actif de type « limus » sont présents dans un rapport massique compris entre 10 % et 30 % de tri-*O*-acylglycérol pour 90 % à 70 % de principe actif de type « limus ».

4. La suspension selon l'une des revendications 1 à 3, où au moins un principe actif de type « limus » se présente sous la forme de microcristaux d'une taille comprise entre 1 µm et 300 µm, et/ou
où au moins 70 % du ou des principes actifs de type « limus » se présentent sous forme de microcristaux d'une taille comprise entre 10 µm et 50 µm, et/ou où le ou les principes actifs de type « limus » ont une cristallinité d'au moins 90 % en poids.

5. La suspension selon l'une des revendications 1 à 4, où le solvant est un non-solvant présentant une permittivité relative εᵣ à 20 °C inférieure ou égale à 2,0, ou bien où le mélange de solvants contient au moins 50 % en volume d'un non-solvant présentant une permittivité relative εᵣ à 20 °C inférieure ou égale à 2,0, et/ou
où le mélange de solvants est un mélange d'au moins un solvant organique polaire présentant un coefficient de partage n-octanol/eau logK_{OW} compris entre -0,5 et +1,5 et une permittivité relative εᵣ à 20 °C comprise entre 5,0 et 30, et d'au moins un solvant organique non polaire présentant une permittivité relative εᵣ à 20 °C inférieure ou égale à 3,0 et un coefficient de partage n-octanol/eau logK_{OW} supérieur ou égal à 3,0.

6. Un procédé de préparation de la suspension selon l'une des revendications 1 à 5, comprenant les étapes suivantes :
a) dissoudre au moins un tri-*O*-acylglycérol choisi dans le groupe constitué de trioctanoylglycérol, de trinonanoylglycérol, de tridécanoylglycérol et de triundécanoylglycérol dans un solvant ou un mélange de solvants ;
b) préparer une suspension d'au moins un principe actif de type « limus » sous forme de microcristaux et de la solution de l'étape a),
où les microcristaux du ou des principes actifs de type « limus » ne se dissolvent pas dans la solution de l'étape a).

7. Le procédé selon la revendication 9, où au moins un principe actif de type « limus » est choisi dans le groupe comprenant ou constitué de rapamycine, d'évérolimus, de zotarolimus, d'umirolimus, de déforolimus, de myolimus, de novolimus, de pimécrolimus, de ridaforolimus, de tacrolimus et de temsirolimus, et/ou
où au moins un principe actif de type « limus » se présente sous forme de microcristaux d'une taille comprise entre 1 µm et 300 µm, et/ou
où au moins 70 % du ou des principes actifs de type « limus » se présentent sous forme de microcristaux d'une taille comprise entre 10 µm et 50 µm, et/ou où le ou les principes actifs de type « limus » ont une cristallinité d'au moins 90 % en poids.

8. Le procédé selon la revendication 6 à 7, où le solvant est un non-solvant présentant une permittivité relative εᵣ à 20 °C inférieure ou égale à 2,0, ou bien où le mélange de solvants contient au moins 50 % en volume d'un non-solvant présentant une permittivité relative εᵣ à 20 °C inférieure ou égale à 2,0, et/ou où le mélange de solvants est un mélange d'au moins un solvant organique polaire présentant un coefficient de partage n-octanol/eau logK_{OW} compris entre -0,5 et +1,5 et une permittivité relative εᵣ à 20 °C comprise entre 5,0 et 30, et d'au moins un solvant organique non polaire présentant une permittivité relative εᵣ à 20 °C inférieure ou égale à 3,0 et un coefficient de partage n-octanol/eau logK_{OW} supérieur ou égal à 3,0.

9. Un procédé de revêtement d'un dispositif médical choisi parmi un ballonnet de cathéter, un cathéter à ballonnet, une endoprothèse ou une canule, comprenant les étapes suivantes :
a) définir une surface à revêtir sur le dispositif médical ;
b) préparer une suspension selon l'une des revendications 1 à 5, contenant au moins un tri-*O*-acylglycérol choisi dans le groupe constitué de trioctanoylglycérol, de trinonanoylglycérol, de tridécanoylglycérol et de triundécanoylglycérol, au moins un principe actif de type « limus » sous forme de microcristaux et un solvant ou un mélange de solvants dans lesquels le ou les tri-*O*-acylglycérols se dissolvent et les microcristaux du ou des principes actifs de type « limus » ne se dissolvent pas ; et
c) appliquer la suspension de revêtement sur la surface du dispositif médical au moyen de l'une des méthodes suivantes : à la seringue, par pipetage, par capillarité, par trempage, par pulvérisation, par étalement, par étalement au fil, par étalement de gouttes ou par roulage.

10. Le procédé selon la revendication 9, comprenant en outre l'étape d) consistant à sécher le revêtement.

11. Un dispositif médical choisi parmi un ballonnet de cathéter, un cathéter à ballonnet, une endoprothèse ou une canule, pouvant être obtenu par le procédé selon la revendication 9 ou 10, ou
un dispositif médical choisi parmi un ballonnet de cathéter, un cathéter à ballonnet, une endoprothèse ou une canule, revêtu au moyen de la suspension selon l'une des revendications 1 à 5, avec séchage subséquent du revêtement.
